(19) Europäisches Patentamt
European Patent Office
Office européen des brevets

(11) Veröffentlichungsnummer: **0 046 953**
**B1**

(12) # EUROPÄISCHE PATENTSCHRIFT

(45) Veröffentlichungstag der Patentschrift: **06.12.89**

(21) Anmeldenummer: **81106535.8**

(22) Anmeldetag: **22.08.81**

(51) Int. Cl.[4]: **C 07 D 217/26**, C 07 D 209/42, C 07 D 401/06, C 07 D 401/12, C 07 D 403/12, C 07 D 405/06, C 07 D 405/12, C 07 D 409/12, C 07 D 413/12, C 07 D 417/12, A 61 K 31/40

(54) **Aminosäurederivate, Verfahren zu ihrer Herstellung, diese enthaltende Mittel und deren Verwendung.**

(30) Priorität: **30.08.80 DE 3032709**
**06.05.81 DE 3118191**

(43) Veröffentlichungstag der Anmeldung: **10.03.82 Patentblatt 82/10**

(45) Bekanntmachung des Hinweises auf die Patenterteilung: **06.12.89 Patentblatt 89/49**

(84) Benannte Vertragsstaaten: **AT BE CH DE FR GB IT LI LU NL SE**

(56) Entgegenhaltungen:
**EP-A-0 012 845**
**EP-A-0 018 549**
**EP-A-0 024 852**
**EP-A-0 037 231**
**EP-A-0 049 658**
**EP-A-0 050 800**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Geiger, Rolf, Prof. Dr., Heinrich- Bleicher-Strasse 33, D-6000 Frankfurt am Main 50 (DE)**
Erfinder: **Teetz, Volker, Dr., An der Tann 20, D-6238 Hofheim am Taunus (DE)**
Erfinder: **Urbach, Hansjörg, Dr., Le Lavandou Strasse 41, D-6242 Kronberg/Taunus (DE)**
Erfinder: **Schölkens, Bernward, Dr., Am Fliedergarten 1, D-6233 Kelkheim (Taunus) (DE)**
Erfinder: **Henning, Rainer, Dr., Dietzstrasse 7, D-6300 Giessen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Aus der EP-A-0 012 845 sind Tetrahydroisochinolin-3-carbonsäure-Derivate, die in der Position 2 einen Mercaptoalkanoyl-Rest tragen, und deren Verwendung als blutdrucksenkende Mittel bekannt.

Es wurden neue Tetrahydroisochinolin-3-carbonsäure-Derivate gefunden, die eine langdauernde, intensive blutdrucksenkende Wirkung besitzen. Die Erfindung betrifft daher Verbindungen der Formel I'

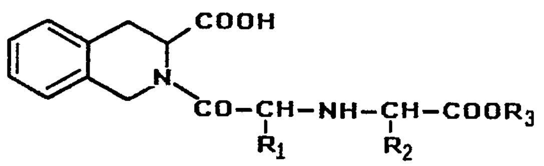

(I')

in welcher $R_1$ und $R_2$ gleich oder verschieden sind und

a) Alkyl oder Alkenyl mit bis zu 6 C-Atomen bedeuten, welche jeweils gegebenenfalls substituiert sind durch einen oder mehrere gleiche oder verschiedene Substituenten aus der Reihe

1. $(C_5$-$C_7)$-Cycloalkyl,
2. $(C_5$-$C_7)$-Cycloalkenyl,
3. Fluor,
4. Mercapto,
5. Hydroxy,
6. $(C_1$-$C_2)$-Alkoxy, das im Alkylteil gegebenenfalls substituiert ist durch
   - 6.1 Methoxy,
   - 6.2 Ethoxy,
   - 6.3 Carboxy,
   - 6.4 Carbamoyl,
   - 6.5 Amino oder
   - 6.6 $(C_1$-$C_6)$-Alkylamino,
7. Aryloxy, das gegebenenfalls substituiert ist durch die unter a) 6.1 - 6.6 genannten Reste oder durch
   - 7.1 Halogen und/oder
   - 7.2 Nitro,
8. Aralkyloxy mit 1 oder 2 C-Atomen im Alkylteil, das im Alkylteil gegebenenfalls wie unter a) 6.1 - 6.6 beschrieben substituiert ist und das im Arylteil gegebenenfalls wie vorstehend im Alkylteil, wie unter a) 7.1 oder 7.2 beschrieben substituiert ist,
9. Amino,
10. Monoalkylamino mit bis zu 7 C-Atomen, das im Alkylrest gegebenenfalls substituiert ist durch
    - 10.1 Hydroxy,
    - 10.2 Carboxy,
    - 10.3 Carbamoyl,
    - 10.4 Ethoxycarbonyl,
    - 10.5 Amino,
    - 10.6 $(C_1$-$C_6)$-Alkylamino,
    - 10.7 Di-$(C_1$-$C_6)$-alkylamino,
    - 10.8 Piperidino oder
    - 10.9 Morpholino,
11. Dialkylamino mit bis zu 7 C-Atomen, das im Alkylrest gegebenenfalls wie unter a) 10.1 - 10.9 beschrieben substituiert ist,
12. Cycloalkylamino mit bis zu 7 C-Atomen, das im Alkylrest gegebenenfalls wie unter a) 10.1 - 10.9 beschrieben substituiert ist
13. Dicycloalkylamino mit bis zu 7 C-Atomen, das im Alkylrest gegebenenfalls wie unter a) 10.1 - 10.9 beschrieben substituiert ist,
14. $(C_1$-$C_6)$-Alkoxycarbonylamino,
15. Aryloxycarbonylamino, das im Arylteil gegebenenfalls mono- oder disubstituiert ist durch
    - 15.1 $(C_1$-$C_2)$-Alkyl,
    - 15.2 $(C_1$-$C_2$ )-Alkoxy,
    - 15.3 Methylendioxy,
    - 15.4 Amino,
    - 15.5 Hydroxy,
    - 15.6 Acetoxy,
    - 15.7 Carboxy,
    - 15.8 Carbamoyl,
    - 15.9 Ethoxycarbonyl,

15.10 Halogen oder
15.11 Nitro,

16. Aralkyloxycarbonyl, das im Arylteil gegebenenfalls wie unter a) 15.1 - 15.11 beschrieben substituiert ist,
17. ($C_1$-$C_6$)-Alkylureido,
18. Arylureido, das im Arylteil gegebenenfalls wie unter a) 15.1 - 15.11 beschrieben durch gleiche oder verschiedene Reste mono- oder disubstituiert ist,
19. Aralkylureido, das im Arylteil gegebenenfalls wie unter a) 15.1 - 15.11 beschrieben mono- oder disubstituiert ist,
20. Formyl,
21. Alkanoylamino mit bis zu 10 C-Atomen,
22. Aroylamino mit bis zu 10 C-Atomen, das im Arylteil gegebenenfalls wie unter a) 15.1 - 15.11 beschrieben mono- oder disubstituiert ist,
23. Aralkanoylamino mit bis zu 10 C-Atomen, das im Arylteil gegebenenfalls wie unter a) 15.1 - 15.11 beschrieben mono- oder disubstituiert ist,
24. Arylamino, das im Arylteil gegebenenfalls wie unter a) 15.1 - 15.11 beschrieben durch gleiche oder verschiedene Reste mono- oder disubstituiert ist,
25. Aralkylamino, das im Arylteil gegebenenfalls wie unter a) 15.1 - 15.11 beschrieben substituiert ist,
26. Alkylmercapto, Alkylsulfinyl oder Alkylsulfonyl mit bis zu 7 C-Atomen, das jeweils im Alkylteil gegebenenfalls substituiert ist durch
    26.1 Methoxy,
    26.2 Ethoxy,
    26.3 Hydroxy,
    26.4 Carboxy,
    26.5 Carbamoyl
    26.6 Ethoxycarbonyl
    26.7 Amino oder
    26.8 ($C_1$-$C_6$)-Alkylamino,
27. Phenylmercapto, Phenylsulfinyl oder Phenylsulfonyl, das im Arylteil gegebenenfalls wie unter a) 26.1 - 26.8 beschrieben oder durch
    27.1 Halogen,
    27.2 Nitro oder
    27.3 Sulfamoyl substituiert ist,
28. Benzylmercapto, Benzylsulfinyl oder Benzylsulfonyl, das im Alkylteil gegebenenfalls wie unter a) 26.1 - 26.8 und das im Arylteil gegebenenfalls wie vorstehender Alkylteil oder wie unter a) 27.1 - 27.3 beschrieben substituiert ist,
29. Carboxy,
30. Ethoxycarbonyl,
31. Carbamoyl,
32. Alkylaminocarbonyl mit bis zu 6 C-Atomen,
33. Cycloalkylcarbamoyl mit bis zu 6 C-Atomen,
34. Cycloalkylaminocarbonyl mit bis zu 6 C-Atomen,
35. Dialkylcarbamoyl mit bis zu 6 C-Atomen,
36. Arylcarbamoyl, das im Arylteil gegebenenfalls substituiert ist durch
    36.1 Halogen,
    36.2 ($C_1$-$C_6$)-Alkyl,
    36.3 ($C_1$-$C_6$)-Alkoxy,
37. Aralkylcarbamoyl,
38. Guanidino,
39. Phenyl, Naphthyl, Dihydronaphthyl oder Tetrahydronaphthyl, die jeweils gegebenenfalls mono- oder disubstituiert sind durch
    39.1 Halogen,
    39.2 Hydroxy,
    39.3 Acetoxy,
    39.4 Carboxy,
    39.5 Carbamoyl,
    39.6 Sulfamoyl,
    39.7 Nitro,
    39.8 Methyl,
    39.9 Ethyl,
    39.10 Methoxy und/oder
    39.12 Ethoxy,
40. einen 5- bis 7-gliedrigen monocyclischen oder 9- bis 10-gliedrigen bicyclischen Heterocyclen-Rest, gegebenenfalls 1 bis 2 S- oder O-Atome und/oder bis zu 4 N-Atome pro Ring enthaltend, der gegebenenfalls substituiert durch
    40.1 Halogen,

40.2 Sauerstoff,
40.3 Hydroxy,
40.4 Carboxy,
40.5 Carbamoyl,
40.6 Sulfamoyl,
40.7 Nitro,
40.7 Alkyl mit bis zu 9 C-Atomen,
40.9 Aralkyl mit bis zu 9 C-Atomen,
40.10 Methoxy und/oder
40.11 Ethoxy;

b) Cycloalkyl oder Cycloalkenyl mit je 5 - 7 C-Atomen bedeuten;
c) Aryl oder teilhydriertes Aryl bedeuten; oder
d) einen mono- oder bicyclischen Heterocyclen-Rest mit 5- 7 bzw. 8 - 10 Gliedern, davon 1 - 2 -S- oder -O- und/oder bis zu 4 N-Atomen bedeuten, das gegebenenfalls wie unter a) 40.1 - 40.11 beschrieben substituiert ist

und

$R_3$ Wasserstoff, Alkyl mit 1 bis 6 C-Atomen, Alkenyl mit 2 bis 6 C-Atomen, Aralkyl mit 7 bis 14 C-Atomen inklusive p-Nitrobenzyl bedeutet,

wobei, falls im einzelnen nicht anders definiert, Aryl 6 bis 10 C-Atome aufweist und gegebenenfalls durch Halogen, ($C_1$-$C_6$)-Alkyl oder ($C_1$-$C_6$)-Alkoxy substituiert ist, und

Aralkyl Benzyl oder Phenethyl bedeutet und gegebenenfalls durch Halogen, ($C_1$-$C_6$)-Alkyl oder ($C_1$-$C_6$)-Alkoxy im Phenylkern substituiert ist, und deren physiologisch verträglichen Salze.

Als bevorzugte Beispiele für $R_1$ und $R_2$ seien insbesondere genannt: Methyl, Ethyl, n-Propyl, Isopropyl, n-Butyl, Isobutyl, tert.-Butyl, sowie die geradkettigen und verzweigten Pentyle und Hexyle, ferner Cyclopentyl, Cyclohexyl, Cycloheptyl, Cyclopentenyl, Cyclohexenyl oder Cycloheptenyl, Phenyl, Naphthyl, Di- und Tetrahydronaphthyl, Benzyl, Phenethyl, p-Fluorphenethyl, o-Methylphenethyl, p-Methoxyphenethyl, 2,4-Dichlorphenethyl, Cyclohexyläthyl.

Die 5- bis 7-gliedrigen mono- oder 9, 10-gliedrigen bicyclischen Heterocyclen können unsubstituiert sein, aber auch einen oder mehrere gleiche oder verschiedene Substituenten tragen wie Halogen, Sauerstoff (auch Sulfoxid oder Sulfon), Hydroxy, Carboxy, Carbonamido, Sulfonamido, Nitro, Alkyl und Aralkyl mit bis zu 9 C-Atomen, Methoxy oder Ethoxy. Im Falle einer Substitution ist Mono- oder Disubstitution bevorzugt.

Als bevorzugte Bedeutungen für $R_3$ seien genannt: Wasserstoff, Ethyl, Butyl, t-Butyl, Benzyl, p-Nitrobenzyl.

Die als $R_1$ oder $R_2$ in Frage kommenden Alkyle oder Alkenyle können geradkettig oder verzweigt sein.

Unter Alkyl ist, falls im Einzelfall nichts anderes gesagt, vorzugsweise ein geradkettiges oder verzweigtes Alkyl mit 1 bis 6 C-Atomen zu verstehen. Gleiches gilt für Alkanoxyl, Alkylamino, Alkylmercapto.

Aryl bedeutet bevorzugt Phenyl, durch Halogen, Alkyl oder Alkoxy substituiertes Phenyl oder Naphthyl. Gleiches gilt für Aroyl, Arylamino, Arylmercapto.

Aralkyl umfaßt bevorzugt Benzyl, Phenethyl und die entsprechenden, im Phenylkern mit Halogen, Nitro, Alkyl oder Alkoxy substituierten Verbindungen. Gleiches gilt für Aralkanoyl, Aralkylamino, Aralkylmercapto.

Verbindungen der allgemeinen Formel I, in der $R_1$ die Seitenkette einer natürlich vorkommenden Aminosäure darstellt, z. B. Methyl, Isobutyl, Methylthioethyl, Carboxymethyl, Carboxyethyl, Amino-n-butyl, Guanido-n-propyl, Imidazol-4-ethyl, Benzyl, 4-Hydroxybenzyl oder 3-Indolmethyl, sowie deren funktionelle Derivate wie Ether, Ester oder Amide sind besonders bevorzugt. Der Rest $R_1$ kann aber auch Teil von Mercapto-, Hydroxy- oder Aminosäuren sein, die nicht in der Natur vorkommen.

Die Erfindung betrifft ferner ein Verfahren zur Herstellung von Verbindungen der Formel I', das dadurch gekennzeichnet ist, daß man

a) eine Verbindung der Formel II' mit einer Verbindung der Formel III umsetzt

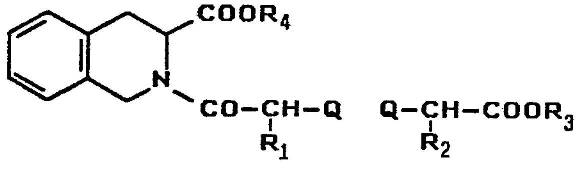

(II') (III)

in denen $R_1$, $R_2$ und $R_3$ wie oben definiert sind, Q eine nucleofuge Gruppe und das andere Q $-NH_2$ bedeutet und $R_4$ für H, Methyl, Ethyl, Benzyl oder tert.-Butyl steht, und gegebenenfalls einen erhaltenen Ester in die Carbonsäure ($R_2$ und/oder $R_4$ = Wasserstoff) überführt oder

b) eine Verbindung der Formel IV

POOC – CH – NH – CH – COOP (IV)
with $R_1$ on the first CH and $R_2$ on the second CH

worin $R_1$ und $R_2$ wie oben definiert sind, P H bedeutet, eines der beiden P jedoch auch die anderen Bedeutungen von $R_3$ inklusive tert.-Butyl haben kann, mit einer Verbindung der Formel V'

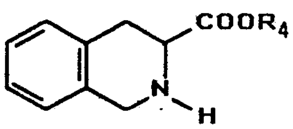

(V')

in der $R_4$ wie oben definiert ist, in Gegenwart eines Kondensationsmittels umsetzt und gegebenenfalls eine oder beide Estergruppen in die Carbonsäure überführt oder

c) eine Verbindung der Formel VI' mit einer Verbindung der Formel VII umsetzt

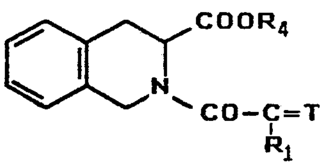

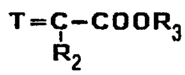

(VI') (VII)

in denen $R_1$, $R_2$, $R_3$ und $R_4$ wie oben definiert sind, ein T für ein Wasserstoffatom und eine $NH_2$ -Gruppe und das andere T für ein Sauerstoffatom steht, und die erhaltene Schiff'sche Base reduziert,

in der nach a) bis c) erhaltene Verbindung Schwefelfunktionen gegebenenfalls zum Sulfoxid oder zum Sulfon oxidiert und die erhaltene Verbindung der Formel I' gegebenenfalls in ihr Salz überführt.

Zur Herstellung der Verbindungen I' kann man beispielsweise von 2-Halogencarbonsäurederivaten II' oder III ausgehen, die mit Nucleophilen reagieren. Diese Reaktion wird bevorzugt in mit Wasser mischbaren organischen Lösungsmitteln, gegebenenfalls in Anwesenheit von Wasser, durchgeführt, wobei eine anorganische oder tertiäre oder quartäre organische Base zugegeben wird. Wenn die Reaktionspartner in aprotischen organischen Lösungsmitteln löslich sind, ist ein solches Lösungsmittel vorzuziehen, wobei als Base vorteilhaft ein Trialkylamin, Tetraalkylammoniumhydroxyd oder Tetramethylguanidin oder eine Suspension eines Alkali- oder Erdalkalicarbonats zugesetzt werden.

Das Ausgangsprodukt

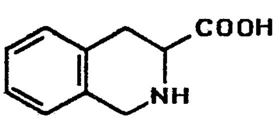

IX

ist literaturbekannt, ihre spezifische pharmakologische Wirkung als Bestandteil der erfindungsgemäßen Verbindungen der Formel I' wurde jedoch bisher nicht beschrieben. Die Verbindung IX kann nach J. Amer. Chem. Soc. 70 (1948), Seite 182 hergestellt werden.

Aus ihr erhält man die Ester mit $R_4$ in bekannter Weise und kondensiert sie mit Halogencarbonsäuren zu den Verbindungen II (Q = Halogen) entweder über die Säurechloride, gemischte Anhydride, Aktivester oder andere Methoden, wie sie in Houben-Weyl, Methoden der Organischen Chemie, Band 15, ausführlich beschrieben sind. Bei $R_1 = CH_3$ kann man z. B. von der aus L-Milchsäure leicht zugänglichen D-2-Chlorpropionsäure ausgehen.

Die Zwischenprodukte der Formel II' ($Q = NH_2$) werden aus den Carbonsäureestern der allgemeinen Formel V' durch Kondensation mit einer N-geschützten 2-Aminocarbonsäure erhalten. Die Schutzgruppe wird nach beendeter Kondensation wieder abgespalten. Als Schutzgruppe kommt z. B. Benzyloxycarbonyl in Frage.

Die Kondensation kann nicht nur mit DCC/HOBt (Dicyclohexylcarbodiimid/1-Hydroxybenzotriazol), sondern auch mit einem anderen geeigneten Kondensationsmittel, z. B. den in Houben-Weyl, Band 15 beschriebenen, durchgeführt werden.

Die Verfahrensweise (b) geht von Verbindungen der Formel IV aus.

Sind $R_1$ und $R_2$ identisch, so kann P Wasserstoff sein. Man kondensiert dann z. B. in Gegenwart von

Dicyclohexylcarbodiimid, mit einem Äquivalent einer Verbindung der Formel V' und spaltet $R_4$ in bekannter Weise ab. Die Reaktion verläuft möglicherweise über das isolierbare Anydrid XII, das anschließend mit einer Verbindung der Formel V' geöffnet wird.

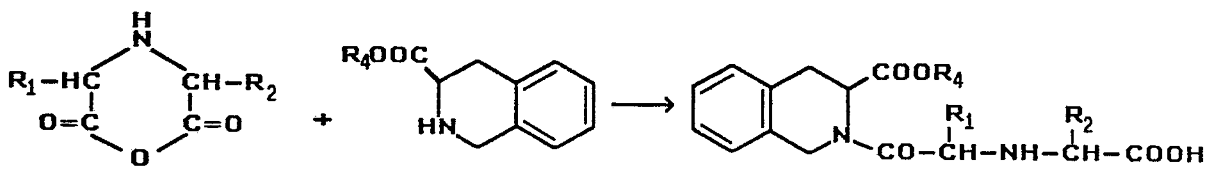

XII V'

Sind die Reste $R_1$ und $R_2$ in der Verbindung IV ungleich, so ist vorzugsweise nur einer der Reste P Wasserstoff, der andere sollte vorzugsweise die Bedeutung von $R_3$ haben oder tert.-Butyl sein. Man kondensiert dann in beschriebener Weise mit den Verbindungen der Formel V' und überführt gegebenenfalls eine oder beide Estergruppen in die Carbonsäure.

Die für T = $NH_2$ in den Formeln VI' bzw. VII bedeutsame Kondensation mit 2-Ketocarbonsäuren bzw. deren Estern gemäß (c) führt in an sich bekannter Weise über Schiff'sche Basen z. B. nach Reduktion mit Natriumborhydrid oder Natriumcyanoborhydrid, durch katalytische Hydrierung oder elektrolytische (Reduktion zu den entsprechenden Verbindungen der Formel I' in guter Reinheit.

Die neuen Verbindungen besitzen unter anderem 3 chirale Zentren an α-C-Atomen. Die Anordnung der Liganden entspricht bevorzugt der L-Konfiguration. Im allgemeinen können durch Kristallisation sterisch weitgehend einheitliche Verbindungen der Formel I' erhalten werden. Bevorzugt sind Gegenstromverteilung oder präparative HPLC zur Anreicherung sterisch einheitlicher Formen.

Die neuen Verbindungen der Formel I' besitzen eine langdauernde, intensive blutdrucksenkende Wirkung.

Sie können zur Bekämpfung des Bluthochdrucks verschiedener Genese eingesetzt und für sich in Kombination in anderen blutdrucksenkenden, gefäßerweiternden oder diuretisch wirksamen Verbindungen angewandt werden. Typische Vertreter dieser Wirkklassen sind z. B. in Erhart-Ruschig, Arzneimittel, 2. Auflage, Weinheim 1972, beschrieben. Die Anwendung kann intravenös, subcutan oder peroral erfolgen.

Die Dosierung bei peroraler Gabe liegt bei 20 - 200 mg je Einzeldosis. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser oder subcutaner Verabreichung sollte die Einzeldosis zwischen 0,01 und 10 mg liegen.

Die Verbindungen der Formel I' liegen als innere Salze vor. Falls beide Carboxylgruppen frei sind, können zusätzlich Alkali- und Erdalkalisalze und solche mit physiologisch unbedenklichen Aminen gebildet werden. Ferner kann eine vorhandene freie Aminogruppe mit einer Mineralsäure oder organischen Säure zu einem Salz umgesetzt werden. Auch die anderen Verbindungen der Formel I' können in freier Form als solche Salze angewandt werden.

Die nachfolgenden Beispiele sollen die erfindungsgemäßen Verfahrensweise erläutern, ohne die Erfindung auf die hier stellvertretend genannten Substanzen zu beschränken.

**Beispiele**

Folgende Abkürzungen werden gebraucht:

| | |
|---|---|
| 1,2,3,4-Tetrahydroisochinolin-3-carbonsäure | Tic |
| Dekahydroisochinolin-3-carbonsäure | Dic |
| Indolin-2-carbonsäure | Idc |
| Oktahydroindol-2-carbonsäure | Oic |
| Benzyloxycarbonyl | Z |
| tert.-Butyloxycarbonyl | Boc |
| tert.-Butyl | tBu, $Bu^t$, tert-$C_4H_9$ |
| 4-Nitrobenzyl | Nb |
| Dicyclohexylcarbodiimid | DCC |
| Dimethylformamid | DMF |
| Dimethylacetamid | DMA |
| N-Ethylmorpholin | NEM |
| Cyclohexylamin | CA |
| Dicyclohexylamin | DCA |

Soweit kein anderes Verfahren angegeben ist, werden die in den folgenden Beispielen beschriebenen Verbindungen zur Analyse und biologischen Bestimmung einer HPLC-Reinigung unterworfen.

**Beispiel 1**

N-(1-Carboxy-3-phenylpropyl)-L-alanyl-1,2,3,4-tetrahydroisochinolin-3-carbonsäure

2-Carbobenzoxy-3-carboxy-1,2, 3,4- tetrahydroisochinol in (Z-Tic)

188 g (1,05 Mol) 3-Carboxyl-1-2-3-4-tetrahydroisochinolin gibt man bei 0°C zu 1050 ml 1 N NaOH und tropft dann bei dieser Temperatur unter Rühren gleichzeitig 160 ml Chlorkohlensäurebenzylester und weitere 1050 ml 1N NaOH ein. Anschließend rührt man 2 Stunden bei Raumtemperatur. Man extrahiert dreimal mit Ether und säuert die alkalisch-wäßrige Phase mit konz. HCl auf pH 1 an. Das ausgefallene Öl wird in Essigester extrahiert. Man wäscht die Essigesterlösung mit Wasser, bis die Wasserphase ein pH von 3,0 zeigt. Nach Trocknung über Natriumsulfat kristallisiert das Produkt aus der Essigesterlösung nach Einengen und Anreiben. Man setzt 1,5 Liter Diisopropylether zu der Kristallsuspension und rührt eine Stunde bei Raumtemperatur, saugt ab und trocknet das Produkt im Hochvakuum über Phoshporpentoxid.

Ausbeute: 256,7 g Schmp. 138 - 39°C

2-Carbobenzoxy- 3- carboxy- 1,2,3 ,4-tetrahydroisochinolintert.-butylester

Zur Lösung von 248,8 g (0,8 Mol) 2-Carbobenzoxy-3-carboxy-1,2,3,4-tetrahydroisochinolin in 1600 ml Methylenchlorid gibt man 312 ml tert.-Butanol und 8 g 4-Dimethylaminopyridin. Man kühlt auf -5°C und setzt dann portionsweise die Lösung von 176 g Dicyclohexylcarbodiimid in 350 ml Methylenchlorid zu. Man rührt 5 Stunden bei 0°C und läßt dann 16 Stunden bei Raumtemperatur stehen. Nach Absaugen von Dicyclohexylharnstoff extrahiert man die Reaktionslösung dreimal mit gesättigter Natriumbicarbonatlösung. Man trocknet über Magnesiumsulfat, engt i. V. bei Raumtemperatur bis zur öligen Konsistenz ein. Es verbleiben 286 g Produkt als gelbliches Öl (97 % d. Th.).

NMR: 7,30 s (5H); 7,20 s (4H); 5,1 - 4,3 m (3H); 5,0 s (2H); 1,46 s (9H)

c) 3-Carboxy-1,2,3,4-tetrahydroisochinolin-tert-butylester-hydrochlorid

284 g 2-Carbobenzoxy-3-carboxy-1,2,3,4-tetrahydroisochinolin-tert.-butylester (0,775 Mol) löst man in 3 Litern Methanol, gibt 15 g 10 % Pd-Bariumsulfatkatalysator zu und hydriert mit Wasserstoff bei Normaldruck. Der pH-Wert der Lösung wird durch Zutropfen von 1 n methanolischer HCl auf pH 4,0 (Glaselektrode) gehalten. Man saugt vom Katalysator ab und dampft die Lösung im Vakuum bei Raumtemperatur zur Trockne ein. Das kristallisierte Produkt wird mit wasserfreiem Ether verrieben, abgesaugt und i. V. über Phosphorpentoxid getrocknet.

Ausbeute: 156 g Schmp. 180°C (Zers.)

Das *Tosylat* kann entweder durch Zugabe von methanolischer Toluolsulfonsäure anstelle von methanolischer Salzsäure während der Hydrierung erhalten werden oder man löst das Hydrochlorid in Wasser, versetzt mit der berechneten Menge Natriumtosylat und kühlt unter Animpfen und Rühren auf ca. 4°C ab. Dabei fällt das Tosylat in kristalliner Form aus. Die Kristalle werden abfiltriert, mit Wasser gewaschen und getrocknet.

Schmp.: 139 - 140°C

d) Carbobenzoxy-L-alanyl-3-carbonyl-1,2,3,4-tetrahydroisochinolin-tert.-butylester

Man suspendiert 27 g (0,15 Mol) 3-Carboxy-1,2,3,4-tetrahydroisochinolin-tert.-butylesterhydrochlorid in 200 ml Dimethylformamid, kühlt unter Rühren und Feuchtigkeitsausschluß auf -5°C, und gibt 19 ml (0,15 Mol) N-Äthylmorpholin sowie die Lösung von 33,4 g Carbobenzoxyalanin, 20 g N-Hydroxybenzotriazol und 33 g Dicyclohexylcarbodiimid in 100 ml Dimethylformamid zu. Man rührt eine Stunde bei 0°C, läßt über Nach bei +4°C stehen und arbeitet dann nach einstündigem Rühren bei Raumtemperatur auf. Man saugt vom ausgeschiedenen Dicyclohexylharnstoff ab und bringt die Reaktionslösung im Hochvakuum bei Raumtemperatur zur Trockne. Das verbliebene Öl nimmt man in 1 l Essigester auf und wäscht dreimal mit je 150 ml gesättigter Natriumcarbonatlösung, 5 % Kaliumbisulfatlösung und einmal mit Wasser. Nach Trocknung über wasserfreiem Natriumsulfat wird das Lösungsmittel i. V. bei Raumtemperatur abdestilliert.

Ausbeute: 59 g Öl

e) L-Alanyl-3-carboxy-1,2,3,4-tetrahydroisochinolin-tert.-butylesterhydrochlorid (Ala-Tic-O-But)

Man hydriert 15 g Carbobenzoxy-alanyl-3-carboxy-1,2,3,4-tetrahydroisochinolin-tert.-butylester in 400 ml Methanol mit 8 g 10 % Pd/Bariumsulfatkatalysator. Der scheinbare pH der Lösung (Glaselektrode) wird durch Zugabe von 1 N methanolischer Salzsäure bei 4,0 gehalten. Nach 8 Stunden saugt man vom Katalysator ab und dampft die Lösung i. V. bei Raumtemperatur bis zur Trockne. Den festen Rückstand digeriert man mit Diisopropyläther und trocknet i. V..
Schmp. 110°C (Zers.)

Analog Ala-Tic-OBut werden dargestellt:

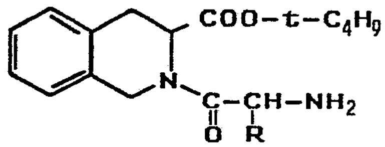

nachstehenden Verbindungen sind folgende Signale gemeinsam:

7,2 s (4H) ; 5,1 - 4,3 m (3H) ; 3,9 - 3,0 m (4H) ; 1,4 s (9H)

| für R: | |
|---|---|
| H | 7,2 s (4H) ; 3,9 - 3,0 m (5H) |
| 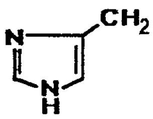 | 13,1 s (1H) ; 7,5 s (1H) ; 6,8 s (1H) : 2,8 m (2H) |
| 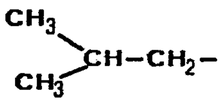 | 1,5 m (3H) ; 0,9 d (6H) |
| $CH_2F-$ | 5,1 - 4,3 m (5H) |
| 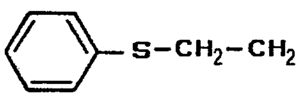 | 7,2 - 7,0 m (9H) ; 2,7 - 2,0 m (4H) |
| 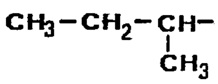 | 1,5 - 1,0 m (9H) |
| 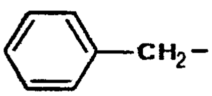 | 7,1 s (5H) ; 2,7 d (2H) |
| 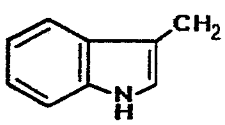 | 7,8 - 6,4 m (9H) |
| $HO-CH_2-$ | 3,7 d (2H) |
| 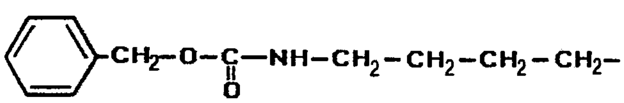 | 7,1 s (5H) ; 5,0 s (2H) ; 2,4 m (2H) ; 1,8 - 1,3 m (6H) |
| 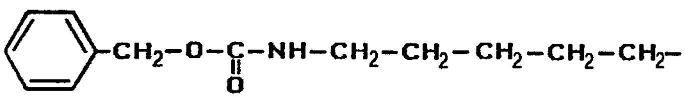 | 7,1 s (5H) ; 5,0 s (2H) ; 2,4 m (2H) ; 1,8 - 1,3 m (8H) |
| 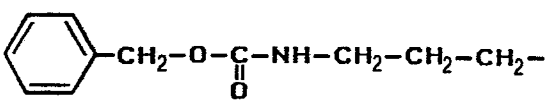 | (7,1 s (5H) ; 5,0 s (2H) ; 2,4 m (2H) ; 1,8 - 1,3 m (4H) |

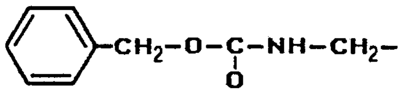

7,1 s (5H) ; 5,0 s (2H) ; 2,3 m (2H)

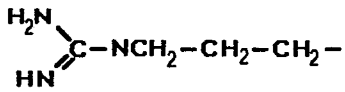

8,3 - 7,6 m (2H) ; 2,9 - 1,6 (6H)

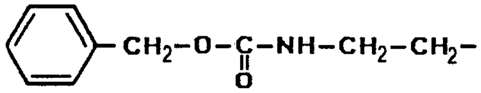

7,1 s (5H) ; 5,0 s (2H) ; 2,4 m (2H) ; 1,7 - 1,4 m (2H)

f) N-(1-Carboxy-3-phenylpropyl)-L-alanyl-1,2,3,4-tetrahydroisochinolin-3-carbonsäure-tert.-butylester

350 mg Alanyl-1,2,3,4-tetrahydroisochinolin-3-carbonsäurebutylester (Ala-Tic-OBut) und 445 mg 4-Phenyl-2-oxo-buttersäure werden in 4 ml Methanol gelöst und mit wäßriger 1 N NaOH auf pH 7,5 gebracht. Man fügt 200 mg Natriumcyanoborhydrid hinzu und läßt 36 Stunden reagieren. Man engt zur Trockne ein und gewinnt die Substanz durch Säulenchromatographie an Kieselgel (Laufmittelsystem: Chloroform/Methanol/Wasser/Eisessig 20 + 15 + 2 + 1).

Ausbeute: 382 mg; Schmp. ab 120°C Zers.

g) N-(1-Carboxy-3-phenylpropyl)-L-alanyl-1,2,3,4-tetrahydroisochinolin-3-carbonsäure

350 mg N-(1-Carboxy-3-phenylpropyl)-Ala-Tic-OBut werden in 3 ml wasserfreier Trifluoressigsäure gelöst und 30 Min. bei Raumtemperatur belassen. Man engt i. Vak. ein und verreibt das hinterbleibende Öl mit kaltem Diisopropyläther und Petroläther.

Ausbeute:276 amorphe Substanz

NMR: 7,20 u. 7,10 (s); 4,3 (s, breit); 3,0 - 3,0 (m, breit); 1,23 u. 1,15 (d)

**Beispiel 2**

N-(1-Carbethoxy-3-phenyl-propyl)-L-alanyl-1,2,3,4-tetrahydroisochinolin-3-carbonsäure

Man löst 4,8 g H-Ala-Tic-O $Bu^t$ · Tos-OH in 40 ml absol. Ethanol, stellt mit KOH in Ethanol auf pH 7 und gibt 6,2 g 2-Oxo-4-phenylbuttersäure-ethylester zu und rührt in Anwesenheit von 9 g pulverisiertem Molekularsieb 4 Å unter langsamer Zugabe von einer Lösung von 2 g Natriumcyanoborhydrid in 15 ml absol. Ethanol. Nach etwa 20 h wird filtriert und das Lösungsmittel im Vakuum abdestilliert. Der Rückstand wird zwischen Essigester und Wasser verteilt, die Essigesterphase abgetrennt und im Vakuum zur Trockne gebracht. Der Rückstand wird an Silicagel im Laufmittel Essigester/Cyclohexan (1 : 2 bis 1 : 4) chromatographiert.

Das Produkt wird in 20 ml Trifluoressigsäure gelöst und 30 Min. bei Raumtemperatur gerührt. Man destilliert die Trifluoressigsäure im Vakuum ab, destilliert mit Toluol nach und chromatographiert über Kieselgel in Methylenchlorid/Methanol (10 : 1).

Ausbeute 2,2 g

**Beispiel 3**

N-(1-Carboxy-5-aminopentyl)-L-alanyl-1,2,3,4-tetrahydroisochinolin-3-carbonsäure

1,2 g N-(1-Carboxy-5-Boc-aminopentyl)-L-alanyl-1,2,3,4-tetrahydroisochinolin-3-carbonsäure werden mit 10 ml eiskalter Trifluoressigsäure übergossen und eine Stunde bei Raumtemperatur gerührt. Anschließend wird im Vakuum eingedampft. Der Rückstand wird in Wasser gelöst und einer Gefriertrocknung unterworfen.

Ausbeute: 0,95 g

**Beispiel 4**

N-(1-Carboxy-3-phenyl-propyl)-L-alanyl-1,2,3,4-tetrahydroisochinolin-3-carbonsäure

880 mg N-(1-Carbethoxy-3-phenyl-propyl)-L-alanyl-1,2,3,4-tetrahydroisochinolin-3-carbonsäure werden mit 20 ml Dioxan/Wasser (9 : 1) gelöst und bei Raumtemperatur mit 4,5 ml 1 n Natriumhydroxid-Lösung verseift. Nach einer Stunde wird die Lösung mit der äquivalenten Menge 1 n Salzsäure angesäuert und das Dioxan weitgehend abgedampft. Der Rückstand wird mehrfach mit Essigester extrahiert und die organische Phase nach Abziehen des Lösungsmittels über einen Kationenaustauscher (®DOWEX 50) chromatographiert.

Ausbeute: 630 mg

**Beispiel 5**

N-(1-Carbethoxy)-3-amino-propyl)-L-alanyl-1,2,3,4-tetrahydro-isochinolin-3-carbonsäure

1,1 g N-(1-Carbethoxy- 3-benzoxycarbonylamino-propyl)-L-alanyl-1,2,3,4-tetrahydroisochinolin-3-carbonsäure werden in 150 ml Methanol gelöst und in Gegenwart von 100 mg Palladium-Aktivkohle (10 %-ig) bei 40°C unter Normaldruck hydriert. Nach Beendigung der Reaktion wird vom Katalysator abfiltriert und das Lösungsmittel im Vakuum abgezogen.
Ausbeute: 0,79 g

**Beispiel 6**

N-(1-Carboxy-3-phenyl-3-thiapropyl)-alanyl-1,2,3,4-tetrahydroisochinolin-3-carbonsäure

a) 2-(2-Oxo-propionyl)-1,2,3,4-tetrahydroisochinolin-3-carbonsäure-tert.-butylester
In eine auf -50°C gekühlte Lösung von 700 mg Tic-OBut und 630 mg Dicyclohexylcarbodiimid in 15 ml wasserfreiem Chloroform wird eine gekühlte und frisch destillierte Lösung von 270 mg Brenztraubensäure in 5 ml Chloroform rasch eingerührt. Man beläßt 16 Stunden im Tiefkühlschrank (-20°C) und filtriert den ausgefallenen DC-Harnstoff ab. Die Lösung wird mit $KHSO_4$- und anschließend mit $KHCO_3$-Lösung gewaschen, über $Na_2SO_4$ getrocknet und i. Vak. eingeengt. Die Verbindung (Öl) enthält etwas Dicyclohexylharnstoff; sie wurde zur Analyse über Kieselgel im System $CHCl_3/CH_3OH$ 15 : 1 chromatographiert.
Analog der Kondensation Brenztraubensäure mit Tic-OBut werden folgende Kondensationsprodukte aus Tic und der entsprechenden α-Ketocarbonsäure dargestellt.

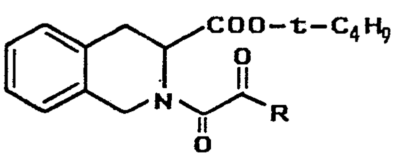

bei nachstehenden Verbindungen werden einheitlich Folgende Signale für das Grundgerüst beobachtet 7,2 s (4H); 5,1 - 4,3 m (3H); 3,9 - 0 m (2H): 1,4 s (9H)

für R:

| | |
|---|---|
| 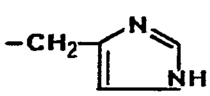 | 13,1 s (1H) ; 7,5 s (1H) ; 6,8 s (1H) ; 3,9 - 3,0 m (4H) |
| $-CH_2-CH(CH_3)_2$ | 2,4 - 1,0 m + d (9H) |
| 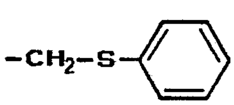 | 7,2 - 7,0 m (9H) ; 2,5 m (2H) |
| $-CH_2F$ | 4,4 s (2H) |
| 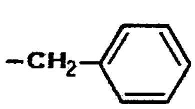 | 7,15 s (5H) ; 3,0 s (2H) |
| 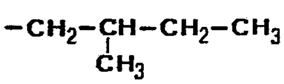 | 2,4 - 1,9 m (3H) ; 1,5 - 1,0 m (8H) |

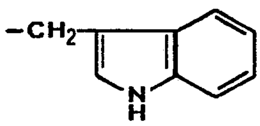

7,8 - 6,4 m (9H) ; 3,9 - 2,0 m (4H)

b) N-(1-Carboxy-3-phenyl-3-thiapropyl)-alanyl-1,2,3,4-tetrahydroisochinolin-3-carbonsäure-tert.-butylester

1,52 g Pyruvyl-Tic-OBut und 394 mg S-Phenyl-cystein werden in 5 ml Methanol gelöst und mit 1 N NaOH (wäßrig) auf pH 7,0 gebracht. Man fügt 400 mg Natriumcyanoborhydrid zu und beläßt 24 Stunden bei Raumtemperatur. Man engt i. Vak. ein, nimmt in Chloroform auf, trocknet über Natriumsulfat und engt ein. Das Produkt wird durch Kieselgelchromatographie (System $CHCl_3/CH_3OH/HAcO/H_2O$ 50/20/5/1) rein erhalten.

NMR: 7,3 - 7,0 m (9H); 5,1 - 4,3 m (3H); 3,9 - 3,0 m (4H); 2,5 m (2H)

c) N-(1-Carboxy-3-phenyl-3-thiapropyl)-alanyl-1,2,3,4-tetrahydroisochinolin-3-carbonsäure

1 g Butylester aus Beispiel 6b wird in 5 ml Trifluoressigsäure gelöst. Man engt nach 30 Min. i. Vak. ein, digeriert den Rückstand mit Diisopropyläther und trocknet über Kaliumhydroxyd.

Ausbeute: 0,95 g Trifluoracetat (hygroskopisch)

NMR: 7,4 - 7,1 m (9H); 5,2 - 4,4 m (3H); 3,9 - 3,0 m (4H); 2,5 m (2H); 1,25 m (3H)

**Beispiel 7**

N-(1-Carbethoxy-2-benzylsulfinylethyl)-L-alanyl-L-1,2,3,4-tetrahydroisochinolin-3-carbonsäure

Zu einer unter Eiskühlung gerührten Mischung von 0,64 g Natriumperjodat und 20 ml Wasser werden 1,3 g N-(1-Carbethoxy-2-benzyl-thio-ethyl)-L-alanyl-L-1,2,3,4-tetrahydroisochinolin-3-carbonsäure gelöst in 20 ml Methanol gegeben. Man läßt die Reaktionsmischung für 24 Stunden bei 0°C rühren und filtriert anschließend vom Natriumjodat ab. Das Filtrat wird mehrfach mit Methylenchlorid extrahiert. Nach Entfernen des Lösungsmittels erhält man 1,1 g des Sulfoxids. Im NMR-Spektrum werden folgende Signale beobachtet: 7,3 - 7,0 m (9H); 5,1 - 4,3 m (3H); 4,1 s (2H); 3,9 - 3,0 m (4H); 2,6 m (2H); 1,2 d (3H).

**Beispiel 8**

N-(1-Carbethoxy-3-phenylsulfonyl-propyl)-L-alanyl-1,2,3,4-tetrahydroisochinolin-3-carbonsäure

260 mg N-(1-Carbethoxy-3-phenylthio-propyl)-L-alanyl-1,2,3,4-tetrahydroisochinolin-3-carbonsäure und 20 mg Natriumwolframat Dihydrat werden in 50 ml Wasser gegeben und tropfenweise mit 1 ml ®Perhydrol (30 %-ig) versetzt. Die Mischung wird für 30 min auf 80°C erwärmt und anschließend eine Stunde bei Raumtemperatur gerührt. Das überschüssige Peroxid wird sodann mit Palladium auf Bariumsulfat zerstört und nach Beendigung der Sauerstoffentwicklung wird die Lösung vom Palladiumkatalysator abfiltriert und eingeengt. Das Rohprodukt wird durch Ionenaustauschchromatographie über ®DOWEX 50 $H^+$-Form gereinigt.

Ausbeute: 220 mg

NMR: 7,7 m (9H); 5,1 - 4,3 m (3H); 3,9 - 3,0 m (4H); 2,8 m (2H); 1,5 m (2H); 1,2 d (3H)

**Beispiel 9**

N-(2-Amino-1-carboxypropyl)-L-alanyl-1,2,3,4-tetrahydroisochinolin-3-carbonsäure

2,2 g $N^{\beta}$-Boc-α,β-Diaminobuttersäure, hergestellt nach Coll. Czech. Chem. Commun. 31, 2955 (1966) analog der $N^{\gamma}$-Boc-Verbindung, werden mit 2,4 g Pyruvyl-Tic-OBut, hergestellt nach Beispiel 6 a), in der in Beispiel 6 b) beschriebenen Weise umgesetzt. Die Boc-Schutzgruppe wird anschließend auf die Beispiel 3 beschriebene Weise abgespalten.

Ausbeute: 2,6 g

**Referenzbeispiel 1**

N-(1-Carboxy-3-phenylpropyl)-L-alanyl-L-dekahydroisochinolin-3-carbonsäure

350 mg N-(1-Carboxy-3-phenylpropyl)-Ala-Dic-OBut werden in 3 ml wasserfreier Trifluoressigsäure gelöst und 30 Minuten bei Raumtemperatur belassen. Man engt im Vakuum ein und verreibt das hinterbleibende Öl mit kaltem Diisopropyläther und Petroläther.

Ausbeute: 226 mg amorphe Substanz

NMR: 7,10 (s) 3,0 - 3,9 (m, breit); 1,23 und 1,15 (d); 2,0 - 1,0 (m, breit)

**Referenzbeispiel 2**

N-(1-Carbethoxy-3-phenyl-propyl)-L-alanyl-L-dekahydroisochinolin-3-carbonsäure

Man löst 4,83 g H-Ala-Dic-$OBu^t$ · TosOH in 40 ml absol. Ethanol, stellt mit KOH in Ethanol auf pH 7 und gibt 6,2 g 2-Oxo-4-phenylbuttersäure-ethylester zu und rührt in Anwesenheit von 9 g pulverisiertem Molekularsieb 4 Å unter langsamer Zugabe von einer Lösung von 2 g Natriumcyanoborhydrid in 15 ml absol. Ethanol. Nach etwa 20 h wird filtriert und das Lösungsmittel im Vakuum abdestilliert. Der Rückstand wird zwischen Essigester und Wasser verteilt, die Essigesterphase abgetrennt und im Vakuum zur Trockene gebracht. Der Rückstand wird an Silicagel im Laufmittel Essigester/Cyclohexan (1 : 2 bis 1 : 4) chromatographiert.

Das Produkt wird 30 min. in 20 ml Trifluoressigsäure gelöst. Man destilliert die Trifluoressigsäure im Vakuum ab, destilliert mit Toluol nach und chromatographiert über Silicagel in Methylenchlorid/Methanol (10 : 1).

Ausbeute: 1,8 g.

**Referenzbeispiel 3**

N-(1-Carboxy- 3-phenyl-propyl)-L-alanyl-L-dekahydroisochinolin-3-carbonsäure

880 mg N-(1-Carbethoxy-3-phenyl-propyl)-L-alanyl-L-dekahydroisochinolin-3-carbonsäure werden in 20 ml Dioxan/Wasser (9 : 1) gelöst und bei Raumtemperatur mit 4,5 ml 1n Natriumhydroxid-Lösung verseift. Nach einer Stunde wird die Lösung mit der äquivalenten Menge n Salzsäure angesäuert und das Dioxan weitgehend abgedampft. Der Rückstand wird mehrfach mit Essigester extrahiert und die organische Phase nach Abziehen des Lösungsmittels über einen Kationenaustauscher (®DOWEX 50) chromatographiert.

Ausbeute: 630 mg.

**Referenzbeispiel 4**

L-N-(1-Carbethoxy-5-aminopentyl)-L-alanyl-L-dekahydroisochinolin-3-carbonsäure

a) 35 g $N^{\varepsilon}$-Benzyloxycarbonyl-L-lysin-ethylester-hydrochlorid und 30,6 g α-Brompropionsäure werden in einer Mischung aus 350 ml Dioxan, 50 ml Ethanol und 75 ml 4N NaOH gelöst. Man rührt über lacht bei Raumtemperatur unter Konstanthalten des pH bei 8.8 - 9 am Autotitrator, bringt dann mit wenig HCl aus pH 7 - 8 und destilliert das Lösungsmittel im Vakuum ab. Der Rückstand wird in möglichst wenig Wasser gelöst und die Lösung mit Salzsäure auf pH 5 - 6 gebracht. Man kühlt mit Eis und filtriert den Niederschlag nach kurzem Stehen ab, wäscht mit wenig eiskaltem Wasser und Ether und trocknet im Vakuum. Man kann die wässrige Lösung auch über schwach basischem Ionenaustauscher in das Zwitterion überführen und reinigen. Das Filtrat wird dann einfach lyophilisiert.

Ausbeute: 12,9 g (52 %).

b) Man löst die Verbindung in 100 ml Dimethylformamid und versetzt nacheinander bei Raumtemperatur mit 23 g H-Dic-OBzl; TosOH (in üblicher Weise aus der Aminosäure durch Kochen in Benzylalkohol-Toluol-Toluolsulfonsäure unter Rückfluß und Wasserabscheidung hergestellt), 6,5 ml N-Ethylmorpholin, 6,8 g 1-Hydroxybenzotriazol und 11 g Dicyclohexylcarbodiimid. Nach 4 h Rühren wird vom Harnstoff abfiltriert, das Lösungsmittel im Vakuum abdestilliert und der Rückstand in Cyclohexan-Essigester (4 : 1) an Silicagel chromatographiert. Dabei tritt Auftrennung der Diastereoisomeren ein. Die einheitlichen Fraktionen, welche die Titelverbindung enthalten, werden gesammtelt, das Lösungsmittel wird im Vakuum abdestilliert.

c) Der Rückstand wird in Methanol aufgenommen, an Pd/Kohle katalytisch hydriert und ggfs. nach Abfiltrieren des Katalysators durch Einstellen des pH auf 3 in Hydrochlorid überführt. Abdestillieren des Lösungsmittels und Trocknen des Rückstandes im Vakuum.

**Referenzbeispiel 5**

L-N-[1-Carbethoxy-4-(4,6-dimethyl-pyrimidyl-2-amino)butyl]-L-alanyl-L-dekahydroisochirolin-3-carbonsäure

a) 15,3 g α-Brompropionsäure und 41,1 g H-Dic-OBut · TosOH werden in 300 ml Tetrahydrofuran in Anwesenheit von 12,8 ml N-Ethylmorpholin und 1,35 g 1-Hydroxybenzotriazol mittels 22 g Dicyclohexylcarbodiimid kondensiert. Nach 4 wird der Harnstoff abfiltriert und das Lösungsmittel im Vakuum abdestilliert. Man nimmt den Rückstand in Essigester auf, wäscht die Lösung mit Natriumhydrogencarbonat- und Citronensäurelösung und mit Wasser, trocknet die Essigesterlösung über Natriumsulfat und destilliert das Lösungsmittel im Vakuum ab.

b) 3,75 g α-Brompropionyl-Dic-OBut werden in 20 ml Tetrahydrofuran mit 2,5 g $N^{\delta}$-(4,6-Dimethyl-pyrimidin-2-yl-L-ornithin-ethylester unter Zusatz von 1 ml Diisopropylethylamin bei Raumtemperatur umgesetzt. Nach 24 h wird das Lösungsmittel im Vakuum abdestilliert, der Rückstand in Essigester aufgenommen, die Lösung mit Wasser gewaschen, über Natriumsulfat getrocknet und im Vakuum eingeengt. Der Rückstand wird an Silicagel im System Cyclohexan-Essigester (4 : 1) unter Diastereomerentrennung chromatographiert. Der t-Butylester wird durch Lösen der Verbindung in Dioxan/HCl gespalten. Nach 10 - 15 min destilliert man das Lösungsmittel ab und trocknet den Rückstand scharf im Vakuum über KOH. Die Verbindung liegt als Hypochlorid vor.

**Referenzbeispiel 6**

a) N-(1-S-Carbethoxy-3-phenyl-propyl)-S-alanyl-cisoctahydroindol-2-R,S-carbonsäurebenzylester

5 g N-(1-S-Carbethoxy-3-phenyl-propyl)-S-alanin werden in 20 ml DMF gelöst. Man fügt 2,45 g 1-Hydroxybenzotriazol, 5,1 g cis-Octahydroindol-R,S-2-carbonsäurebenzylester-hydrochlorid, 2,5 ml N-Ethylmorpholin sowie 4 g Dicyclohexylcarbodiimid zu und rührt 3 Stunden bei Raumtemperatur. Man verdünnt mit 30 ml Essigester und saugt vom Harnstoff ab. Nach Einengen im Vakuum wird in 100 ml Äther aufgenommen und mit wässr. Bicarbonatlösung 2 x extrahiert. Die über Natriumsulfat getrocknete und zur Trockene eingeengte organische Phase wird an Kieselgel chromatographiert. Man eluiert mit Essigester/Petroläther 4 : 3 und erhält 2 Fraktionen, aus denen durch Einengen im Vakuum farblose Öle erhalten werden.

| Elementaranalyse: | C | H | N | |
|---|---|---|---|---|
| ber. für $C_{31}H_{40}N_2O_5$ | 71.5 | 7.7 | 5.4 | Ausb. |
| Frakt. 1 | 71.3 | 7.8 | 5.3 | 3.3 g |
| Frakt. 2 | 71.6 | 7.6 | 5.6 | 3.9 g |

b) N-(1-S-Carbethoxy-3-phenyl-propyl)-S-alanyl-cisoctahydroindol-2-S-carbonsäure

3 g Ges Benzylesters (Fraktion 2) werden in Ethanol gelöst und mit 200 mg 10 % Pd/Kohle bei Normaldruck hydrogenolytisch entbenzyliert. Nach beendeter Wasserstoffaufnahme (etwa 1 Stunde) wird vom Katalysator abfiltriert und die Lösung im Vakuum eingeengt. Man versetzt mit etwas Pentan, erwärmt auf etwa 45°C und legt ein kräftiges Vakuum an. Es bildet sich ein amorpher, fester Schaum. Ausb.: 2,4 g.

NMR($CDCl_3$) : 1,20 (d, 3H) ; 1,23 (t, 3H) ; über Multiplett 1,0 - 2,9 (11H) ; 3,0 - 4,5 (m, 3H) ; 4,12 (q, 2H) ; 4,65 (b, 2H) ; 7,13 (s, 5H).

**Referenzbeispiel 7**

N-(1-Carbethoxy-3-Phenyl-propyl)-alanyl-octahydroindol-2-carbonsäurebenzylester

a) N-tert.Butyloxycarbonyl-alanyl-octahydroindol-2-carbonsäure-benzylester (Boc-Ala-Oic-OBzl)

19 g Boc-Ala-OH werden in 100 ml DMF gelöst und mit 13 ml N-Ethylmorpholin 13,5 g HOBt sowie 29,6 g Octahydroindol-2-carbonsäurebenzylester-hydrochlorid versetzt. Man kühlt im Eisbad, fügt 21 g Dicyclohexylcarbodiimid zu und läßt 15 Stunden bei Raumtenperatur rühren. Der ausgefallene Harnstoff wird abgesaugt, das Filtrat im Vakuum eingeengt und in Essigester aufgenommen. Man extrahiert je 3 x mit wässriger $KHSO_4$-, $KHCO_3$- und gesättigter NaCl-Lösung und engt die organische Phase im Vakuum ein. Ausbeute: 38.5 g.

NMR: 1,26 (d, 3H) ; 1,40 (s, 9H) ; 1,1 - 2,4 (m, 12H) ; 3,2 - 3,9 (m, 2H) ; 5,28 (s, 2H) ; 7,31 (s, 5H).

b) Alanyl-octahydroindol-2-carbonsäurebenzylester-trifluoracetat

21,5 g Boc-Ala-Oic-OBzl werden in 50 ml Trifluoressigsäure gelöst. Man engt im Vakuum ein, digeriert den Rückstand mehrfach mit Diiscpropyläther und trocknet im Vakuum. Ausbeute: 21 g. Im NMR fehlt das Protonensignal für die tert. Butylgruppe vollständig.

c) N-(1-Carbethoxy-3-phenyl-propyl)-alanyl-octahydroindol-2-carbonsäurebenzylester

11,1 g Ala-Oic-OBzl.TFA, 7 g α-Brom-phenylbuttersäureethylester und 3,3 ml N-Ethylmorpholin werden in 20 ml Dimethylacetamid 4 Tage bei Raumtemperatur gerührt. Man engt im Vakuum weitgehend ein, löst den Rückstand in Methanol, stellt mit wässr. 2n HCl auf pH 2 und extrahiert lipophile Verbindungen mit Petroläther. Die methanolische Phase wird eingeengt, mit 5 % Sodalösung versetzt und das Reaktionsprodukt in Essigester extrahiert. Chromatographie über Kieselgel (System Essigester/Petroläther 5 : 3) liefert die Titelverbindung als farbloses Öl.

Die nachstehend aufgeführten Beispiele sind nach einer der folgenden Methoden A - H hergestellt, wobei die bezeichneten Methoden wie folgt charakterisiert werden:

**Methode A**

Reduktive Aminierung eines Kondensationsproduktes aus Tic-$OtC_4H_9$-Ester oder -Benzylester und einer entsprechenden α-Ketocarbonsäure, dargestellt nach Beispiel (6a), mit einem entsprechenden Aminosäurederivat nach Beispiel (6b), Abspaltung der Schutzgruppen nach Beispiel (6c) und gegebenenfalls Verseifung nach Referenzbeispiel (3).

**Methode B**

Reduktive Aminierung eines Dipeptid-t-Butylesters, dargestellt nach Beispiel (1e), mit einem entsprechenden α-Ketocarbonsäurederivat nach Beispiel (1f) und ggfs. Abspaltung der Schutzgruppen nach Referenzbeispiel (1 bzw. 2) und/oder ggfs. nach Referenzbeispiel (3).

**Methode C**

Reduktive Aminierung nach Methode A und mit einer ω-geschützten bifunktionellen Aminosäure, Abspaltung einer Schutzgruppe aus dem eingesetzten Aminosäurederivat nach Beispiel (3).

**Methode D**

Oxidation der zugrundeliegenden Schwefel-Verbindung zum Sulfoxid nach Beispiel (7).

**Methode E**

Oxidation der zugrundeliegenden Schwefel-Verbindung zum Sulfon nach Beispiel (8).

**Methode F**

Kondensation einer entsprechenden N-alkylierten α-Aminosäure (dargestellt analog Referenzbeispiel 4a) mit einem Aminosäureester (z. B. Dic-$OBu^t$ oder Dic-OBzl bzw. Oic-$OBu^t$ oder Oic-OBzl) nach Referenzbeispiel (4b) oder (6a) und Abspaltung der Schutzgruppe nach Referenzbeispiel (4c) oder (6b).

**Methode G**

Umsetzung eines N-acylierten Aminosäureesters, der im Acylteil in α-Position zur Amidfunktion eine nucleofuge Gruppe trägt (wie z. B. Halogen, Arylsulfonyl oder Alkylsulfonyl), dargestellt nach Referenzbeispiel (5a), mit einer entsprechenden Aminosäure oder einem entsprechenden Aminosäureester nach Referenzbeispiel (5b) und Abspaltung der Schutzgruppen.

**Methode H**

Umsetzung eines Dipeptid-Benzylesters, dargestellt nach Referenzbeispiel (7a und 7b), mit einer entsprechend substituierten α-Arylsulfonyl- oder α-Alkylsulfonyl- oder α-Halogen-carbonsäure bzw. Carbonsäureester, dargestellt nach Referenzbeispiel (7c) und Spaltung des Benzylesters zu Verbindungen der Formel I' nach der in Referenzbeispiel (6b) angegebenen Weise.

Falls nicht anders angegeben, gelten für die nachstehenden Verbindungen folgende NMR-Daten (ϑ-Werte in ppm; TMS als Standard): 7,3 s (4H), 5,1 - 4,3 m (3H), 3,9 - 3,0 m (4H)

Im falle der Ethylester treten noch folgende Signale auf:

| | | | | |
|---|---|---|---|---|
| 4.2 | q | 7 | Hz | (2H) |
| 1.2 | t | 7 | Hz | (3H) |

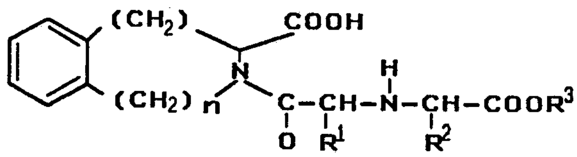

| | n | $R^1$ | $R^2$ | $R^3$ | Methode | NMR |
|---|---|---|---|---|---|---|
| 1 | 1 | $CH_3$ | $CH_2-\overset{H}{N}-CHO$ | $C_2H_5$ | A | 8,3 2s (1H); 2,9m (2H); 1,2d (3H) |
| 2 | 1 | $CH_3$ | $CH_2-\overset{H}{N}-COCH_3$ | $C_2H_5$ | A | 2,9m (2H); 2,1s (3H), 1,2d (3H) |
| 3 | 1 | $CH_3$ | 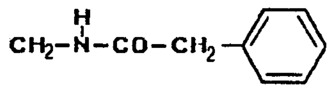 | $C_2H_5$ | A | 3,9 - 3,0m (6H) 2,9m (2H); 1,2d (3H) |
| 4 | 1 | H | 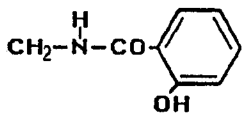 | $C_2H_5$ | A | 7,2 - 6,8m (8H); 3,9 - 3,0m (5H); 2,9m (2H) |
| 5 | 1 | H | 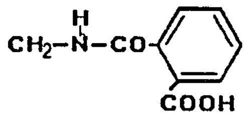 | $C_2H_5$ | A | 7,9 - 7,0m (8H);3,9 - 3,0m (5H); 2,9m (2H) |
| 6 | 1 | $CH_3$ | 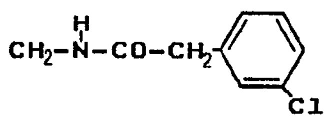 | $C_2H_5$ | A | 7,65 - 7,1m (8H); 3,9 - 3,0m (6H) 2,9m (2H); 1,2d (3H) |
| 7 | 1 | $CH_3$ | 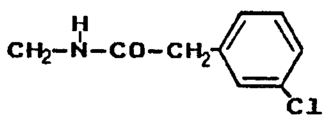 | H | A | 7,65 - 7,1m (8H); 3,9 - 3,0m (6H) 2,9m (2H); 1,2d (3H) |
| 8 | 1 | $CH_3$ | 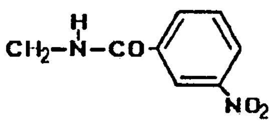 | $C_2H_5$ | A | 8,2 - 7,1m (8H); 2,9m (2H); 1,2d (3H) |
| 9 | 1 | $CH_3$ | 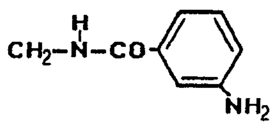 | $C_2H_5$ | A | 7,2 - 6,8m (8H); 2,9s (2H); 1,2d (3H) |
| 10 | 1 | 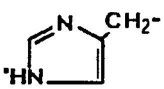 | 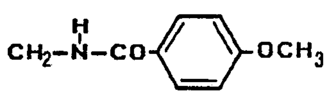 | $C_2H_5$ | A | 13,0s (1H) 7,5 - 6,8m (10H); 3,9s (3H) 2,9 - 2,6m (4H) |
| 11 | 1 | $CH_3$ | 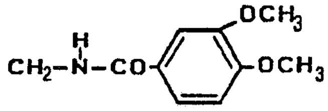 | $C_2H_5$ | A | 7,5 - 6,9m (7H); 3,9s (6H); 2,9m (2H); 1,2d (3H) |
| 12 | 1 | $CH_3$ | 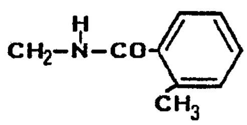 | $C_2H_5$ | A | 7,4 - 7,0m (8H); 2,9m (2H); 2,4s (3H); 1,2d (3H) |
| 13 | 1 | $CH_3$ | 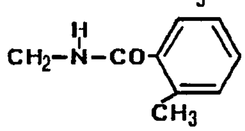 | H | A | 7,4 - 7,0m (8H); 2,9m (2H); 2,4s (3H) 1,2d (3H) |

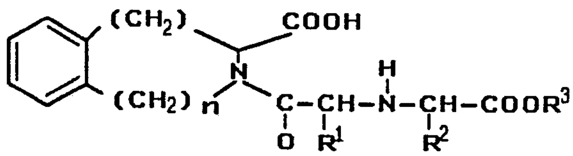

| | n | $R^1$ | $R^2$ | $R^3$ | Methode | NMR |
|---|---|---|---|---|---|---|
| 14 | 1 | $CH_3$ | $CH_2-CH_2-N(CH_3)_2$ | $C_2H_5$ | A | 2,4m (2H); 2,2s (6H); 1,7m (2H); 1,2d (3H) |
| 15 | 1 | $CH_3$ | 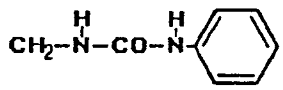 | $C_2H_5$ | A | 7,3 - 7,0m (9H); 2,9m (2H) 1,2d (3H) |
| 16 | 1 | $CH_3$ | 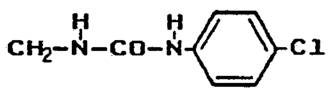 | $C_2H_5$ | A | 8,0 - 7,0m (8H); 2,9m (2H) 1,2d (3H) |
| 17 | 1 | $CH_3$ | 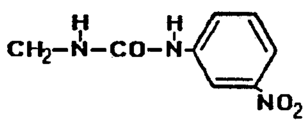 | $C_2H_5$ | A | 8,3 - 7,1m (8H); 2,9m (2H) 1,2d (3H) |
| 18 | 1 | $(CH_3)_2CH-CH_2$ | 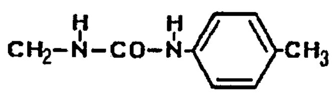 | $C_2H_5$ | A | 7,4 - 7,0m (8H); 2,9m (2H); 2,3s (3H) 1,0d (6H) |
| 19 | 1 | $CH_3$ | 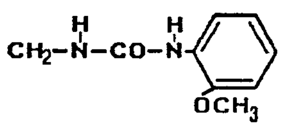 | $C_2H_5$ | A | 7,3 - 6,7m (8H); 3,8s (3H); 2,9m (2H); 1,2d (3H) |
| 20 | 1 | $CH_3$ | 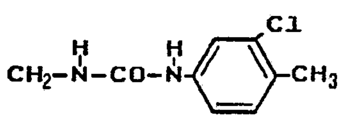 | $C_2H_5$ | A | 7,6 - 6,9m (7H); 2,9m (2H); 2,4s (3H); 1,2d (3H) |
| 21 | 1 | 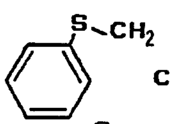 | $CH_2-NH-CO-NH-CH_3$ | $C_2H_5$ | A | 7,3 - 7,0m (9H); 3,0 - 2,6 m+s (7H) |
| 22 | 1 | 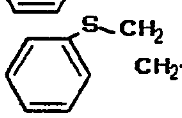 | $CH_2-NH-CO-NH-CH_3$ | H | A | 7,3 - 7,0m (9H); 3,0 - 2,6 m+s (7H) |
| 23 | 1 | $CH_2F$ | $CH_2-NH-CO-NH-C_4H_9$ | $C_2H_5$ | A | 5,1 - 4,3m (5H); 2,9m (2H); 1,0t (3H) |
| 24 | 1 | $CH_3$ | 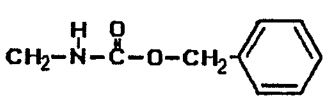 | $C_2H_5$ | A | 7,1s (5H); 5,0s (2H); 2,9m (2H); 1,2d (3H) |
| 25 | 1 | $CH_3$ | $CH_2-NH-C(=O)-O-C_2H_5$ | $C_2H_5$ | A | 4,2q (4H); 2,9m (2H);1,2 t+d (9H) |
| 26 | 1 | $CH_3$ | $CH_2-CH_2-NH-CHO$ | $C_2H_5$ | A | 8,2 2s (1H); 2,9m (2H); 1,5m (2H); 1,2d (3H) |
| 27 | 1 | H | $CH_2-CH_2-NH-COCH_3$ | $C_2H_5$ | A | 3,9 - 3,0m (5H); 2,9m (2H) 2,1s (3H); 1,5m (2H) |
| 28 | 1 | $CH_3$ | 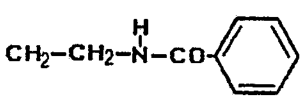 | $C_2H_5$ | A | 7,6 - 7,0m (9H); 2,9m (2H) 1,5m (2H); 1,2d (3H) |

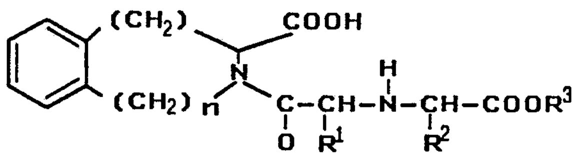

| | n | $R^1$ | $R^2$ | $R^3$ | Methode | NMR |
|---|---|---|---|---|---|---|
| 29 | 1 | $CH_3$ | 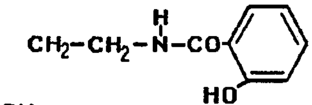 | $C_2H_5$ | A | 7,2 - 6,8m (8H); 2,9m (2H) 1,5m (2H); 1,2d (3H) |
| 30 | 1 | 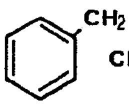 | 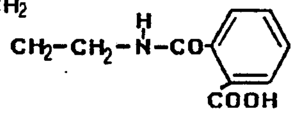 | $C_2H_5$ | A | 7,9 - 7,0m (13H); 2,9 - 2,7m (4H); 1,5m (2H) |
| 31 | 1 | $CH_3$ | 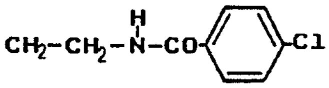 | $C_2H_5$ | A | 8,0 - 7,1m (8H); 2,9m (2H); 1,5m (2H); 1,2d (3H) |
| 32 | 1 | $CH_3$ | 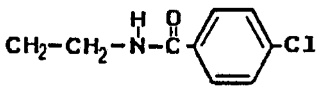 | H | A | 8,0 - 7,1m (8H); 2,9m (2H); 1,5m (2H); 1,2d (3H) |
| 33 | 1 | $CH_3$ | 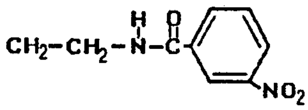 | $C_2H_5$ | A | 8,3 - 7,0m (8H); 2,9m (2H); 1,5m (2H); 1,2d (3H) |
| 34 | 1 | $CH_3$ | 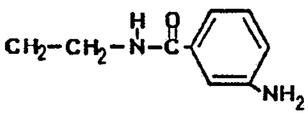 | $C_2H_5$ | A | 7,2 - 6,8m (8H); 2,9m (2H); 1,5m (2H) 1,2d (3H) |
| 35 | 1 | $CH_3$ | 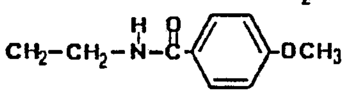 | $C_2H_5$ | A | 7,5 - 6,8m (8H); 3,8s (3H); 2,9m (2H); 1,5m (2H); 1,2d (3H) |
| 36 | 1 | $CH_3$ | 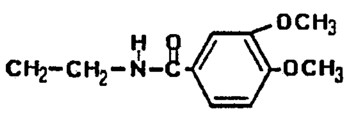 | $C_2H_5$ | A | 7,5 - 6,9m (7H); 3,9s (6H); 2,9m (2H); 1,5m (2H); 1,2d (3H) |
| 37 | 1 | $CH_3$ | 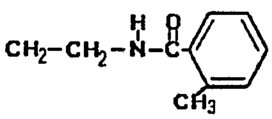 | $C_2H_5$ | A | 7,4 - 7,0m (8H); 2,9m (2H); 2,3s (3H); 1,5m (2H); 1,2d (3H) |
| 38 | 1 | $CH_3$ | 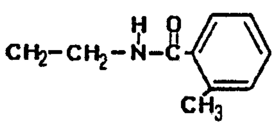 | H | A | 7,4 - 7,0m (8H); 2,9m (2H); 2,3s (3H); 1,5m (2H); 1,2d (3H) |
| 39 | 1 | $CH_3$ | $CH_2-CH_2-CH_2-N(C_2H_5)_2$ | $C_2H_5$ | A | 2,6 - 2,4m (6H); 1,5m (4H); 1,2d (3H) 0,9m (6H) |
| 40 | 1 | $CH_3$ | 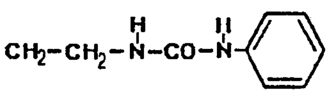 | $C_2H_5$ | A | 7,5 - 7,0m (9H); 2,9m (2H); 1,5m (2H); 1,2d (3H) |
| 41 | 1 | $CH_3$ | 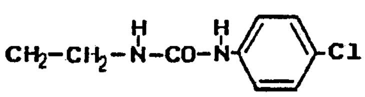 | $C_2H_5$ | A | 7,5 - 7,0m (8H); 2,9m (2H); 1,5m (2H) 1,2d (3H) |
| 42 | 1 | $CH_3$ | 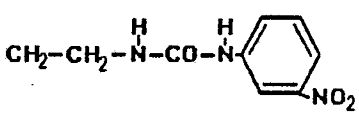 | $C_2H_5$ | A | 8,3 - 7,1m (8H); 2,9s (2H); 1,5m (2H) 1,2d (3H) |

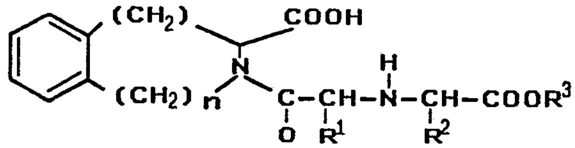

| | n | $R^1$ | $R^2$ | $R^3$ | Methode | NMR |
|---|---|---|---|---|---|---|
| 43 | 1 | $CH_3$ | 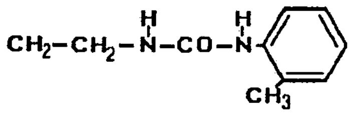 | $C_2H_5$ | A | 7,4 - 7,0m (8H); 2,9m (2H); 2,3s (3H); 1,5m (2H); 1,2d (3H) |
| 44 | 1 | $CH_3$ | 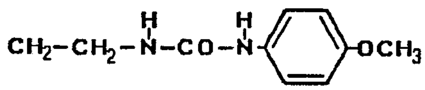 | $C_2H_5$ | A | 7,2 - 6,5m (8H); 3,9s (3H); 2,9m (2H); 1,5m (2H); 1,2d (3H) |
| 45 | 1 | $CH_3$ | 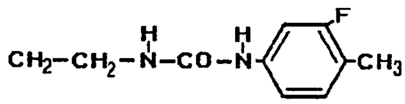 | $C_2H_5$ | A | 7,3 - 6,9m (7H); 2,9m (2H); 2,3s (3H); 1,5m (2H); 1,2d (3H) |
| 46 | 1 | $(CH_3)_2CH-CH_2$ | $CH_2-CH_2-\overset{H}{N}-CO-\overset{H}{N}-CH_3$ | $C_2H_5$ | A | 3,0 - 2,6m (5H); 1,7 - 1,3m (5H); 1,0d (6H) |
| 47 | 1 | $(CH_3)_2CH-CH_2$ | $CH_2-CH_2-\overset{H}{N}-CO-\overset{H}{N}-C_4H_9$ | $C_2H_5$ | A | 3,0 - 2,6m (4H); 1,7 - 1,2m (9H); 0,9d+t (9H) |
| 48 | 1 | $(CH_3)_2CH-CH_2$ | $CH_2-CH_2-\overset{H}{N}-CO-\overset{H}{N}-C_4H_9$ | H | A | 3,0 - 2,6m (4H); 1,7 - 1,2m (9H); 0,9d+t (9H) |
| 49 | 1 | $CH_3$ | 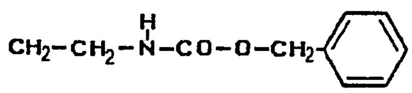 | $C_2H_5$ | A | 7,1s (5H); 5,0s (2H); 2,9m (2H); 1,5m (2H); 1,2d (3H) |
| 50 | 1 | $CH_3$ | $CH_2-CH_2-\overset{H}{N}-CO-O-C_2H_5$ | $C_2H_5$ | A | 4,2q (4H); 2,9m (2H); 1,5m (2H); 1,2d+t (9H) |
| 51 | 1 | $CH_3$ | $CH_2-CH_2-\overset{H}{N}-CH_3$ | $C_2H_5$ | A | 2,4m (2H); 2,3s (3H); 1,5m (2H); 1,2d (3H) |
| 52 | 1 | $CH_3$ | 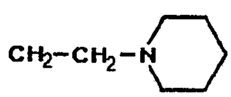 | $C_2H_5$ | A | 2,4m (6H); 1,8 - 1,2m (8H); 1,2d (3H); |
| 53 | 1 | $CH_3$ | 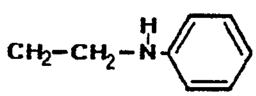 | $C_2H_5$ | A | 7,2 - 6,5m (9H); 2,5m (2H); 1,5m (2H) 1,2d (3H) |
| 54 | 1 | $(CH_3)_2CH-CH_2$ | 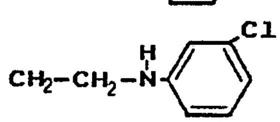 | $C_2H_5$ | A | 7,2 - 6,6m (8H); 2,4m (2H); 1,5m (5H) 1,0d (6H) |
| 55 | 1 | 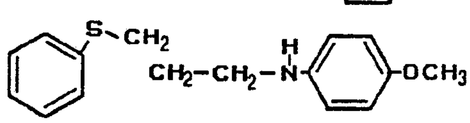 | | $C_2H_5$ | A | 7,3 - 6,4m (3H); 3,8s (3H); 2,5 - 2,2m (4H); 1,5m (2H) |
| 56 | 1 | $CH_2F$ | 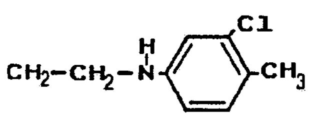 | $C_2H_5$ | A | 7,2 - 6,6m (7H); 5,1 - 4,3m (5H); 2,4m (2H); 2,3s (3H); 1,5m (2H); |
| 57 | 1 | $CH_3$ | 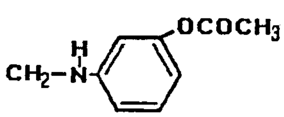 | $C_2H_5$ | A | 7,3 - 6,5m (8H); 2,5m (2H); 2,1s (3H); 1,2d (3H) |

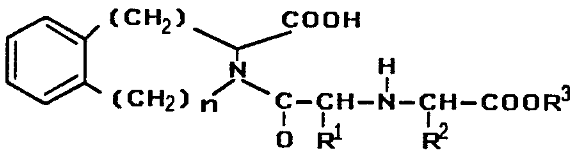

| | n | $R^1$ | $R^2$ | $R^3$ | Methode | NMR |
|---|---|---|---|---|---|---|
| 58 | 1 | $CH_3$ | 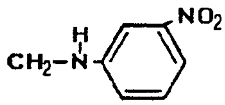 | $C_2H_5$ | A | 7,7 - 7,0m (8H); 2,5m (2H); 1,2d (3H); 1,5m (2H); 1,2d (3H) |
| 59 | 1 | $CH_3$ | 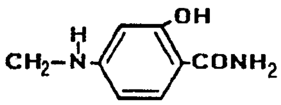 | $C_2H_5$ | A | 7,6 - 6,7m (7H); 2,4m (2H); 11,2d (3H) |
| 60 | 1 | $CH_3$ | 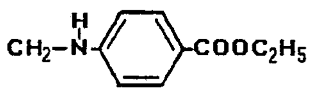 | $C_2H_5$ | A | 7,6 - 6,8m (8H); 4,2q (4H); 2,5m (2H); 1,2d+t (9H) |
| 61 | 1 | $CH_3$ | 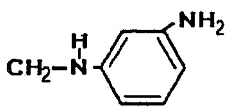 | $C_2H_5$ | A | 7,2 - 6,7m (8H); 2,5m (2H); 1,2d (3H); |
| 62 | 1 | $CH_3$ | $-CH_2-NH-CH_2-CH_2-COOC_2H_5$ | $C_2H_5$ | A | 4,2q (4H); 2,6 - 2,3m (6H); 1,2 d+t (9H) 0,9d+t (9H) |
| 63 | 1 | $CH_3$ | $CH_2-CH_2-CH_2-NH_2$ | H | C | 2,3m (2H); 1,5m (4H); 1,2d (3H); |
| 64 | 1 | $CH_3$ | $CH_2-NH-CH_2-CH_2-N(C_2H_5)_2$ | $C_2H_5$ | A | 2,6 - 2,4m (10H); 1,2d+t (9H); |
| 65 | 1 | $CH_3$ | 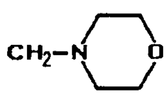 | $C_2H_5$ | A | 3,9 - 3,0m (12H); 2,3m (2H); 1,2d (3H) |
| 66 | 1 | $CH_3$ | 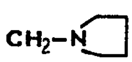 | $C_2H_5$ | A | 2,6 - 2,3m (6H); 1,6 - 1,4m (4H); 1,2d (3H) |
| 67 | 1 | $CH_3$ | $CH_2-NH-CH_2-CH_2-CONH_2$ | $C_2H_5$ | A | 2,6 - 2,2m (6H); 1,2d (3H); |
| 68 | 1 | $CH_3$ | $CH_2-CH_2-CH_2-N(CH_3)_2$ | $C_2H_5$ | A | 2,4m (2H); 2,2s (6H); 1,5m (4H); 1,2d (3H) |
| 69 | 1 | $CH_3$ | $CH_2-NH-CH_2-CONH_2$ | $C_2H_5$ | A | 3,9 - 3,0m (6H); 2,4m (2H); 1,2d (3H); |
| 70 | 1 | $CH_3$ | $(CH_2)_5-NH_2$ | H | C | 2,3m (2H); 1,6 - 1,2m (8H); 1,2d (3H) |
| 71 | 1 | $H_2N-(CH_2)_4$ | $(CH_2)_4-NH_2$ | H | C | 2,4m (4H); 1,6 - 1,2m (12H); |
| 72 | 1 | $CH_3$ | $CH_2-CH_2-NH-CH_3$ | $C_2H_5$ | A | 2,4m (2H); 2,2s (3H); 1,5m (2H); 1,2d (3H) |
| 73 | 1 | $CH_3$ | $CH_2-NH-CH_3$ | $C_2H_5$ | A | 2,4m (2H); 2,1s (3H); 1,2d (3H) |
| 74 | 1 | $CH_3$ | 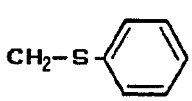 | $C_2H_5$ | A | 7,4 - 7,0m (9H); 2,7m (2H); 1,2d (3H); |

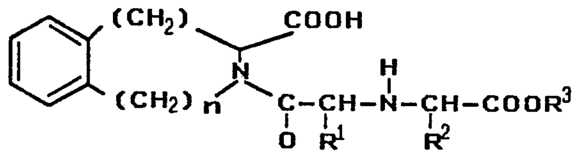

| | n | $R^1$ | $R^2$ | $R^3$ | Methode | NMR |
|---|---|---|---|---|---|---|
| 75 | 1 | $CH_3$ | 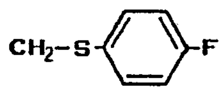 | $C_2H_5$ | A | 7,3 - 6,8m (8H); 2,7m (2H); 1,2d (3H); |
| 76 | 1 | $CH_3$ | $CH_2-SH$ | $C_2H_5$ | A | 2,4m (2H); 1,2d (3H) |
| 77 | 1 | $CH_3$ | 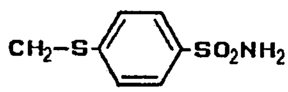 | $C_2H_5$ | A | 8,1 - 7,1m (8H); 6,5bs (2H); 2,7m (2H); 1,3d (3H); |
| 78 | 1 | $(CH_3)_2CH-CH_2$ | 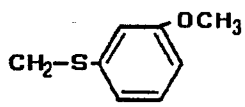 | $C_2H_5$ | A | 7,2 - 6,7m (8H); 3,8s (3H); 2,7m (2H); 1,6 - 1,2m (3H); 1,0d (6H) |
| 79 | 1 | 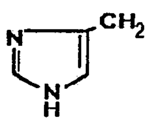 | 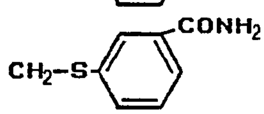 | $C_2H_5$ | A | 13,0s (1H); 7,8 - 6,8m (3H); 6,0bs (2H); 2,9 - 2,6m (4H); |
| 80 | 1 | 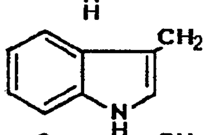 | 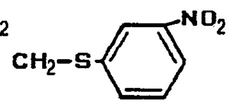 | $C_2H_5$ | A | 8,2 - 6,4m (14H); 2,9 - 2,6m (4H); |
| 81 | 1 | 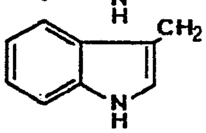 | 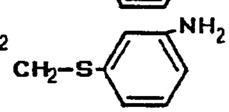 | $C_2H_5$ | A | 7,8 - 6,4m (14H); 2,9 - 2,6m (4H); |
| 82 | 1 | $CH_3$ | 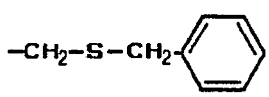 | $C_2H_5$ | A | 7,3 - 7,0m (9H); 3,9 - 3,0m+s (6H); 2,4m (2H); 1,2d (3H) |
| 83 | 1 | 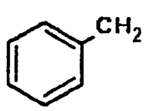 | $-CH_2-S-CH(CH_3)_2$ | $C_2H_5$ | A | 7,3 - 6,9m (9H); 2,6 - 2,3m (5H); 0,9d (6H) |
| 84 | 1 | $CH_2F$ | $-CH_2-S-CH_2-CH_2-CONH_2$ | $C_2H_5$ | A | 5,1 - 4,3m (5H); 2,5 - 2,2m (6H); |
| 85 | 1 | $CH_3$ | $-CH_2-S-CH_2-CH_2-COOC_2H_5$ | $C_2H_5$ | A | 4,2q (4H); 2,5 - 2,2m (6H); 1,2 d+t (9H); |
| 86 | 1 | $CH_3$ | $-CH_2-S-CH_2-CH_2-OC_2H_5$ | $C_2H_5$ | A | 3,9 - 3,0q+m (8H); 2,4 - 2,2m (4H); 1,2d+t (6H) |
| 87 | 1 | $CH_3$ | 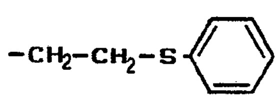 | $C_2H_5$ | A | 7,3 - 7,0m (9H); 2,7m (2H); 1,5m (2H); 1,2d (3H) |
| 88 | 1 | $CH_3$ | 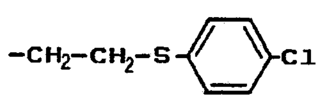 | $C_2H_5$ | A | 7,5 - 7,0m (8H); 2,7m (2H); 1,5m (2H); 1,2d (3H); |
| 89 | 1 | $CH_3$ | 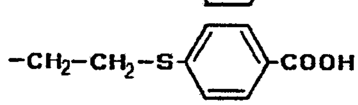 | $C_2H_5$ | A | 7,9 - 6,9m (8H); 2,7m (2H); 1,5m (2H); 1,2d (3H); |
| 90 | 1 | $CH_3$ | 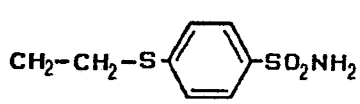 | $C_2H_5$ | A | 8,0 - 6,9m (8H); 6,5bs (2H); 2,6m (2H); 1,5m (2H); 1,2d (3H) |

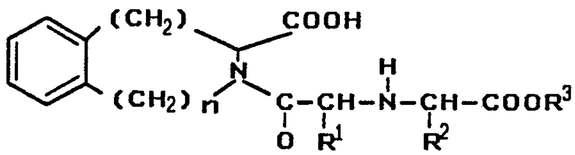

| | n | $R^1$ | $R^2$ | $R^3$ | Methode | NMR |
|---|---|---|---|---|---|---|
| 91 | 1 | $CH_3$ | 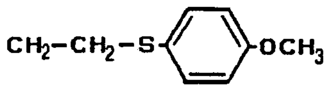 | $C_2H_5$ | A | 7,3 - 6,4m (8H); 3,8s (3H); 2,7m (2H); 1,5m (2H); 1,2d (3H) |
| 92 | 1 | $CH_3$ | 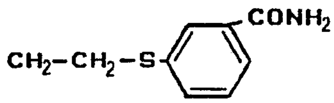 | $C_2H_5$ | A | 7,8 - 6,9m (8H); 6,0bs (2H); 2,6m (2H); 1,5m (2H); 1,2d (3H) |
| 93 | 1 | $CH_3$ | 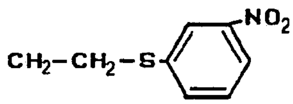 | $C_2H_5$ | A | 8,1 - 6,9 (8H); 2,7m (2H); 1,5m (2H); 1,2d (3H) |
| 94 | 1 | $CH_3$ | 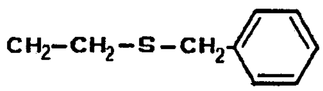 | $C_2H_5$ | A | 7,4 - 7,0m (9H); 3,9 - 3,0m+s (6H); 2,4m (2H); 1,5m (2H); 1,2d (3H) |
| 95 | 1 | $CH_3$ | $CH_2-CH_2-S-CH-(CH_3)_2$ | $C_2H_5$ | A | 2,6 - 2,3m (3H); 1,5m (2H); 1,2d (3H); 1,0d (6H) |
| 96 | 1 | $CH_3$ | $CH_2-CH_2-S-CH_2-CH_2-CONH_2$ | $C_2H_5$ | A | 6,0bs (2H); 2,5 - 2,2m (6H); 1,5m (2H); 1,2d (3H); |
| 97 | 1 | $CH_3$ | 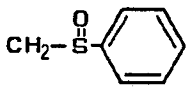 | $C_2H_5$ | D | 7,5 - 7,0m (9H); 2,6m (2H); 1,2d (3H) |
| 98 | 1 | $CH_3$ | 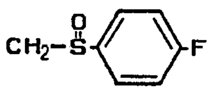 | $C_2H_5$ | D | 7,4 - 6,9m (8H); 2,6m (2H); 1,2d (3H) |
| 99 | 1 | $CH_3$ | 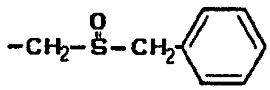 | $C_2H_5$ | D | 7,3 - 7,0m (9H); 4,1s (2H); 2,6m (2H); 1,2d (3H) |
| 100 | 1 | $CH_3$ | 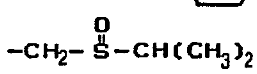 | $C_2H_5$ | D | 2,8 - 2,5m (3H); 1,2c (3H); 1,0d (6H) |
| 101 | 1 | $CH_3$ | 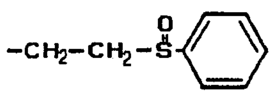 | $C_2H_5$ | D | 7,4 - 7,0m (9H); 2,6m (2H); 1,5 (2H); 1,2d (3H)) |
| 102 | 1 | $CH_3$ | 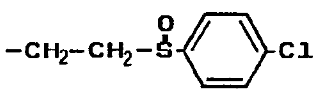 | $C_2H_5$ | D | 7,5 - 6,9m (8H); 2,6m (2H); 1,5m (2H); 1,2d (3H) |
| 103 | 1 | $CH_3$ | 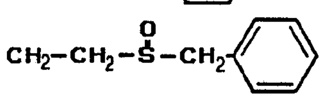 | $C_2H_5$ | D | 7,3 - 7,0m (9H); 4,1s (2H); 2,7m (2H); 1,5m (2H); 1,2d (3H) |
| 104 | 1 | $CH_3$ | $CH_2-CH_2-S-CH(CH_3)_2$ | $C_2H_5$ | D | 2,8 - 2,5m (3H); 1,5m (2H); 1,2d (3H); 0,9d (6H) |
| 105 | 1 | $CH_3$ | 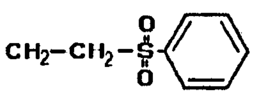 | $C_2H_5$ | E | 7,8 - 7,1m (9H); 2,8m (2H); 1,2d (3H); |
| 106 | 1 | $CH_3$ | 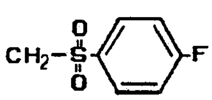 | $C_2H_5$ | E | 7,7 - 6,9m (8H); 2,8m (2H); 1,2d (3H); |
| 107 | 1 | $CH_3$ | 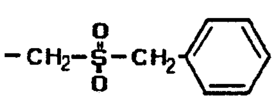 | $C_2H_5$ | E | 7,6 - 7,0m (9H); 5,0 - 4,3m (5H); 2,8m (2H); 1,2d (3H) |

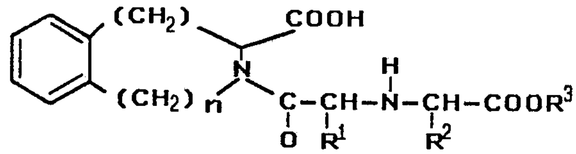

| | n | $R^1$ | $R^2$ | $R^3$ | Methode | NMR |
|---|---|---|---|---|---|---|
| 108 | 1 | $CH_3$ | 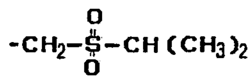 | $C_2H_5$ | E | 3,0 - 2,7m (3H); 1,2d+t (9H); |
| 109 | 1 | $CH_3$ | 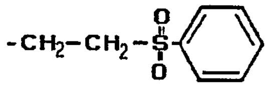 | $C_2H_5$ | E | 7,7 - 7,2m (9H); 2,8m (2H); 1,5m (2H); 1,2d (3H) |
| 110 | 1 | $CH_3$ | 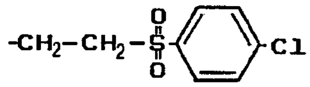 | $C_2H_5$ | E | 8,0 - 7,1m (8H); 2,8m (2H); 1,5m (2H); 1,2d (3H) |
| 111 | 1 | $CH_3$ | 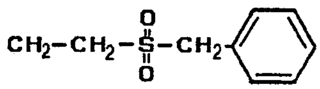 | $C_2H_5$ | E | 7,2 - 6,9m (9H); 4,2s (2H); 2,8m (2H); 1,5m (2H); 1,2d (3H) |
| 112 | 1 | $CH_3$ | 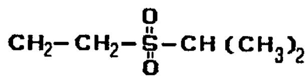 | $C_2H_5$ | E | 3,0 - 2,7m (3H); 1,5m (2H); 1,1d+t (9H) |
| 113 | 1 | $CH_3$ | $-CH_2-OH$ | $C_2H_5$ | A | 3,9 - 3,0m (6H); 1,2d (3H) |
| 114 | 1 | $CH_3$ | 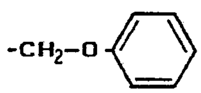 | $C_2H_5$ | A | 7,3 - 6,7m (9H); 3,9 - 3,0m (6H) 1,2d (3H) |
| 115 | 1 | $CH_3$ | 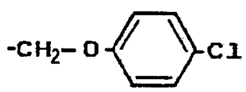 | $C_2H_5$ | A | 7,3 - 6,7m (8H); 3,9 - 3,0m (6H); 1,2d (3H) |
| 116 | 1 | $H_2N-(CH_2)_4$ | 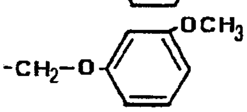 | $C_2H_5$ | B | 7,2 - 6,4m (8H); 3,9 - 3,0m+s (9H); 2,4m (2H); 1,7 - 1,3m (6H) |
| 117 | 1 | $H_2N-(CH_2)_3$ | 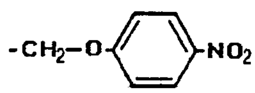 | $C_2H_5$ | B | 8,2 - 6,8m (8H); 3,9 - 3,0m (6H); 2,4m (2H); 1,7 - 1,3m (4H) |
| 118 | 1 | $(CH_3)_2-CH-CH_2-$ | 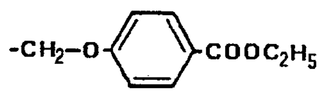 | $C_2H_5$ | A | 7,6 - 6,8m (8H); 4,2q (4H); 3,9 - 3,0m (6H); 1,9 - 1,3m (3H); 1,2t (6H) 1,0d (6H) |
| 119 | 1 | $CH_3$ | 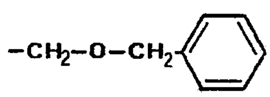 | $C_2H_5$ | A | 7,2 - 6,9m (9H); 4,0g (2H); 3,9 - 3,0m (6H); 1,3d (3H) |
| 120 | 1 | $CH_3$ | 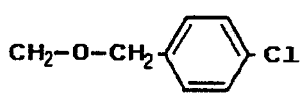 | $C_2H_5$ | A | 7,4 - 6,9m (8H); 4,0s (2H); 3,9 - 3,0m (6H); 1,2d (3H) |
| 121 | 1 | 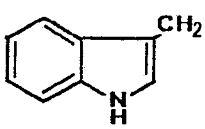 | 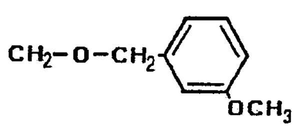 | $C_2H_5$ | A | 7,8 - 6,4m (14H); 4,0s (2H); 3,8s (3H) 3,9 - 3,0m (6H); 2,7m (2H) |
| 122 | 1 | H | 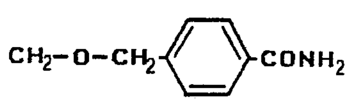 | $C_2H_5$ | A | 7,8 - 7,0m (8H); 6,0 bs (2H); 4,0s (2H); 3,9 - 3,0m (7H) |

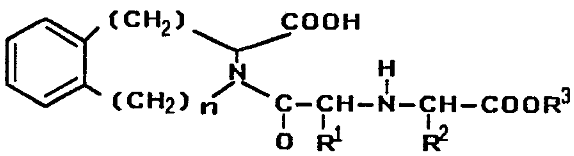

| | n | $R^1$ | $R^2$ | $R^3$ | Methode | NMR |
|---|---|---|---|---|---|---|
| 123 | 1 | $HS-CH_2$ | 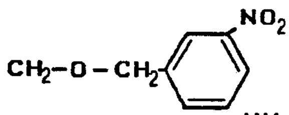 | $C_2H_5$ | B | 8,2 - 7,0m (8H); 4,0s (2H); 3,9 - 3,0m (6H); 2,3m (2H) |
| 124 | 1 | $HS-CH_2$ | 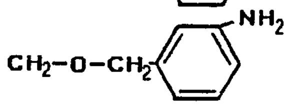 | $C_2H_5$ | B | 7,2 - 6,4m (8H); 4,0s (2H); 3,9 - 3,0m (6H); 2,3m (2H) |
| 125 | 1 | $CH_3$ | 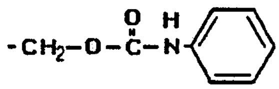 | $C_2H_5$ | A | 7,6 - 6,9m (9H); 4,0m (2H); 1,2d (3H) |
| 126 | 1 | $CH_3$ | $CH_2-COOH$ | H | A | 2,1m (2H); 1,2d (3H) |
| 127 | 1 | $CH_3$ | $CH_2-CONH_2$ | H | F | 6,5bs (2H); 2,1m (2H); 1,2d (3H) |
| 128 | 1 | $CH_3$ | $CH_2-CON(CH_3)_2$ | H | F | 3,9 - 3,0m+2s (10H); 2,1m (2H); 1,2d (3H) |
| 129 | 1 | 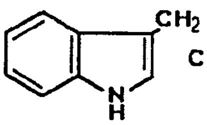 | 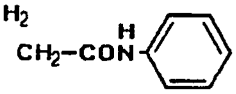 | H | F | 7,8 - 6,4m (15H); 2,8 - 2,0m (4H) |
| 130 | 1 | $CH_3$ | 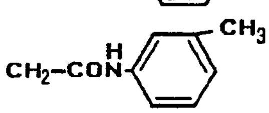 | H | F | 7,3 - 6,9m (8H); 2,3s (3H); 2,2m (2H); 1,2d (3H) |
| 131 | 1 | $CH_3$ | 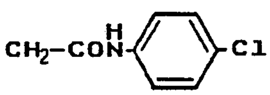 | H | F | 7,7 - 6,9m (8H), 2,3m (2H); 1,2d (3H) |
| 132 | 1 | 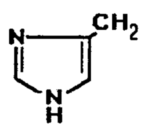 | 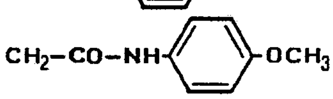 | H | F | 13,0s (1H); 7,5 - 6,3m (10H); 3,9s (3H); 2,7m (2H); 2,1m (2H) |
| 133 | 1 | $CH_3$ | 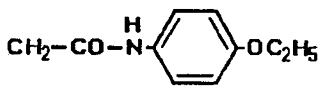 | H | F | 7,2 - 6,2m (8H); 3,5q (2H); 2,1m (2H); 1,2d+t (6H) |
| 134 | 1 | $CH_3$ | 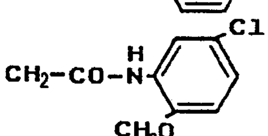 | H | F | 7,2 - 6,4m (7H); 3,9s (3H); 2,1m (2H); 1,2d (3H) |
| 135 | 1 | $CH_3$ | 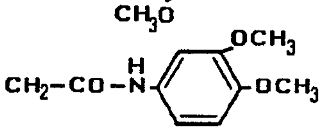 | H | F | 7,2 - 6,4m (7H); 3,8s (6H); 2,1m (2H); 1,2d (3H) |
| 136 | 1 | $CH_3$ | 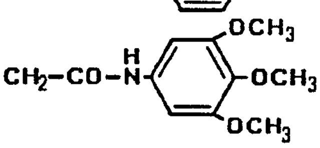 | H | F | 7,2 - 6,2m (6H); 3,9s (9H); 2,1m (2H); 1,2d (3H) |
| 137 | 1 | $CH_3$ | $CH_2-COOC_2H_5$ | H | F | 4,2q (2H); 2,0m (2H); 1,2d+t (6H) |
| 138 | 1 | $CH_3$ | 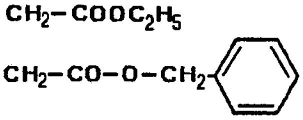 | H | F | 7,3 - 7,0m (9H); 5,3s (2H); 2,1m (2H); 1,2d (3H) |
| 139 | 1 | $CH_3$ | $CH_2-CH_2-COOH$ | $C_2H_5$ | A | 2,1m (2H); 1,5m (2H); 1,2d (3H) |

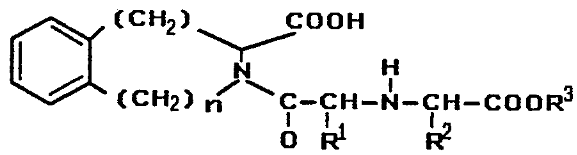

| | n | $R^1$ | $R^2$ | $R^3$ | Methode | NMR |
|---|---|---|---|---|---|---|
| 140 | 1 | $CH_3$ | $CH_2-CH_2-CONH_2$ | $C_2H_5$ | A | 2,1m (2H); 1,5m (2H); 1,2d (3H) |
| 141 | 1 | $CH_3$ | $CH_2-CH_2-CON(CH_3)_2$ | $C_2H_5$ | A | 3,02s (6H); 1,5m (2H); 1,2d (3H) |
| 142 | 1 | $CH_3$ | 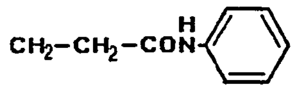 | $C_2H_5$ | A | 7,4 - 7,0m (9H); 2,1m (2H); 1,5m (2H); 1,2d (3H) |
| 143 | 1 | $CH_3$ | 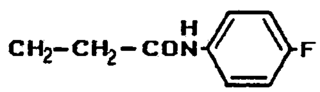 | $C_2H_5$ | A | 7,3 - 6,9m (8H); 2,1m (2H); 1,5m (2H); 1,2d (3H) |
| 144 | 1 | $H_2N-(CH_2)_4$ | 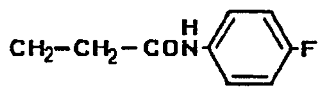 | $C_2H_5$ | B | 7,3 - 6,8m (8H); 2,4 - 2,1m (4H); 1,7 - 1,3m (8H) |
| 145 | 1 | $CH_3$ | 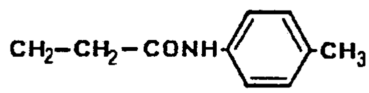 | $C_2H_5$ | A | 7,2 - 6,4m (8H); 3,9s (3H); 2,1m (2H); 1,5m (2H); 1,2d (3H) |
| 146 | 1 | $CH_3$ | 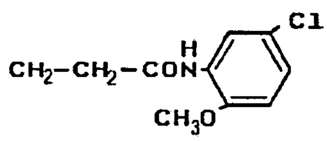 | H | A | 7,3 - 6,4m (7H); 3,9s (3H); 2,1m (2H); 1,5m (2H); 1,2d (2H) |
| 147 | 1 | $CH_3$ | 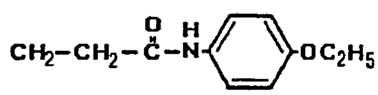 | H | A | 7,3 - 6,4m (8H); 3,9 - 3,0 m+q (6H); 2,1m (2H); 1,5m (2H); 1,2 d+t (6H) |
| 148 | 1 | $CH_3$ | 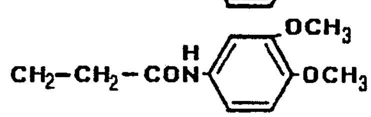 | $C_2H_5$ | A | 7,3 - 6,3m (7H); 3,8s (6H); 2,1m (2H); 1,5m (2H); 1,2d (3H) |
| 149 | 1 | $CH_3$ | 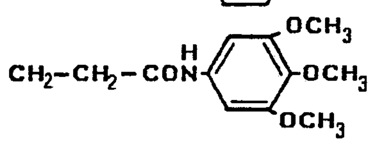 | $C_2H_5$ | A | 7,3 - 6,2m (6H); 3,9s (9H); 2,1m (2H); 1,5m (2H); 1,2H (3H) |
| 150 | 1 | $CH_3$ | $CH_2-CH_2-CONH-CH(CH_3)_2$ | H | A | 2,8 - 2,2m (3H); 1,5m (2H); 1,2d (3H); 1,0d (6H) |
| 151 | 1 | $CH_3$ | 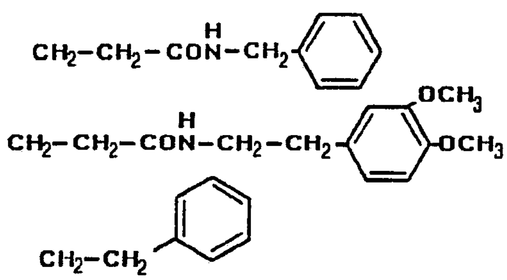 | H | A | 7,4 - 7,0m (9H); 5,1 - 4,3 m+s (5H): 2,1m (2H); 1,5m (2H); 1,2d (3H) |
| 152 | 1 | $CH_3$ | | $C_2H_5$ | A | 7,3 - 6,2m (7H); 3,9s (6H); 2,9 - 2,1m (6H); 1,5m (2H); 1,2d (3H) |
| 153 | 1 | $CH_3$ | | $C_2H_5$ | B | 7,2bs (9H); 2,6m (2H); 1,7m (2H); 1,2d (3H) |
| 154 | 1 | $CH_3$ | 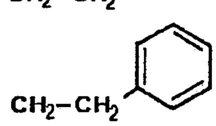 | H | B | 7,2bs (9H); 2,6m (2H); 1,7m (2H) |

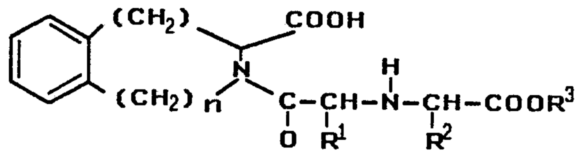

| | n | $R^1$ | $R^2$ | $R^3$ | Methode | NMR |
|---|---|---|---|---|---|---|
| 155 | 1 | $CH_3$ | 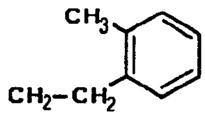 | $C_2H_5$ | B | 7,4 - 7,0m (8H); 2,6m (2H); 2,3s (3H); 1,7m (2H); 1,2d (3H) |
| 156 | 1 | $HO-CH_2$ | 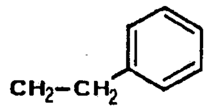 | $C_2H_5$ | B | 7,2bs (9H); 2,8 - 2,5m (4H); 1,7m (2H) |
| 157 | 1 | $H_2N-(CH_2)_4-$ | 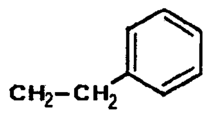 | H | B | 7,2bs (9H); 2,8 - 2,3m (4H); 1,8 - 1,3m (8H) |
| 158 | 1 | $H_2N(CH_2)_3-$ | 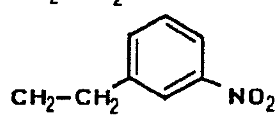 | H | B | 8,2 - 7,1m (8H); 2,8 - 2,3m (4H); 1,8 - 1,3m (6H) |
| 159 | 1 | $CH_3$ | 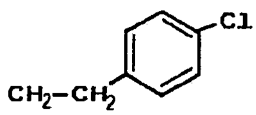 | $C_2H_5$ | B | 7,5 - 6,9m (8H); 2,7m (2H); 1,7m (2H); 1,2d (3H) |
| 160 | 1 | $CH_3$ | 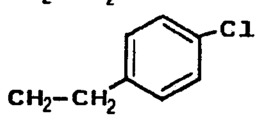 | H | B | 7,5 - 6,9m (8H); 2,7m (2H); 1,7m (2H); 1,2d (3H) |
| 161 | 1 | $CH_3$ | 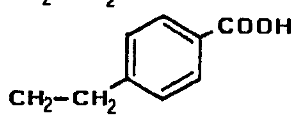 | H | B | 7,8 - 7,1m (8H); 2,7m (2H); 1,7m (2H); 1,2d (3H) |
| 162 | 1 | HN<br>C–NH–$(CH_2)_4$<br>$H_2N$ | 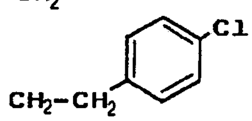 | $C_2H_5$ | B | 7,5 - 7,0m (8H); 2,8 - 2,6m (4H); 1,7 - 1,3m (6H) |
| 163 | 1 | $H_2N-(CH_2)_2$ | 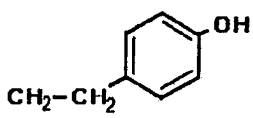 | H | B | 7,2 - 6,5m (8H); 2,8 - 2,3m (4H); 1,7 - 1,3m (4H) |
| 164 | 1 | $H_2N-(CH_2)_5$ | 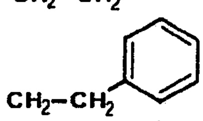 | H | B | 7,2bs (9H); 2,7 - 2,3m (4H); 1,7 - 1,2m (10H) |
| 165 | 1 | $H_2N-(CH_2)_4$ | 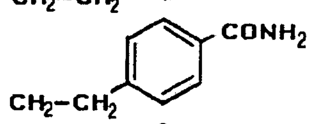 | H | B | 7,8 - 7,1m (8H); 2,7 - 2,3m (4H); 1,8 - 1,3m (8H) |
| 166 | 1 | $H_2N-CH_2$ | 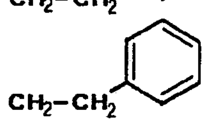 | H | B | 7,2bs (9H); 2,7 - 2,3m (4H); 1,7m (2H) |
| 167 | 1 | $CH_3$ | 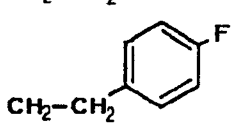 | $C_2H_5$ | B | 7,2 - 6,8m (8H); 2,7m (2H); 1,7m (2H); 1,2d (3H) |

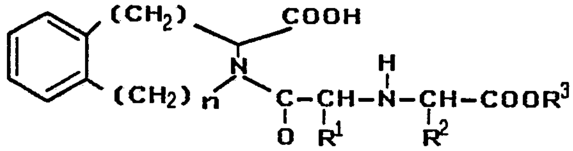

| | n | $R^1$ | $R^2$ | $R^3$ | Methode | NMR |
|---|---|---|---|---|---|---|
| 168 | 1 | $CH_3$ | 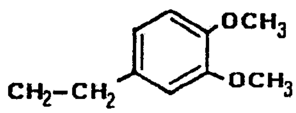 | $C_2H_5$ | B | 7,2 - 6,3m (7H); 3,9s (6H); 2,7m (2H); 1,7m (2H); 1,2d (3H) |
| 169 | 1 | $CH_3$ | 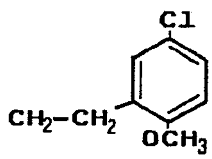 | $C_2H_5$ | B | 7,3 - 6,4m (7H); 3,8s (3H); 2,6m (2H); 1,7m (2H); 1,2d (3H) |
| 170 | 1 | $CH_3$ | 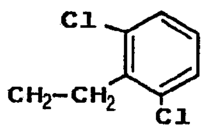 | $C_2H_5$ | B | 7,4 - 6,8m (7H); 2,7m (2H); 1,7m (2H); 1,2d (3H) |
| 171 | 1 | $CH_3$ | 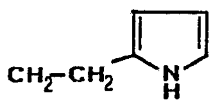 | $C_2H_5$ | B | 7,3-6,0m (7H); 2,8m (2H); 1,7m (2H); 1,2d (3H) |
| 172 | 1 | $CH_3$ | 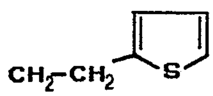 | $C_2H_5$ | B | 7,3 - 6,9m (7H); 2,9m (2H); 1,7m (2H); 1,2d (3H) |
| 173 | 1 | $H_2N-(CH_2)_4$ | 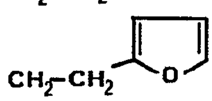 | H | B | 7,4 - 6,3m (7H); 2,8 - 2,3m (4H); 1,8 - 1,3m (8H) |
| 174 | 1 | $CH_3$ | 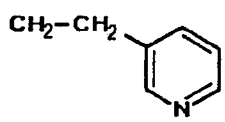 | $C_2H_5$ | B | 8,6 - 7,1m (8H); 2,9m (2H); 1,7m (2H); 1,2d (3H) |
| 175 | 1 | $CH_3$ | 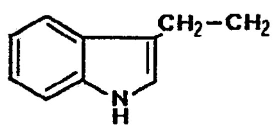 | $C_2H_5$ | B | 7,8 - 6,5m (10H); 2,9m (2H); 1,7m (2H); 1,2d (3H) |
| 176 | 1 | $CH_3$ | 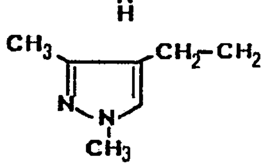 | $C_2H_5$ | B | 7,4 - 7,0m (5H); 3,9 - 3,0 m+s (7H); 2,8m (2H); 2,0s (3H); 1,7m (2H); 1,2d (3H) |
| 177 | 1 | $CH_3$ | 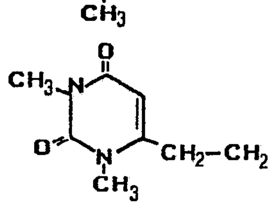 | $C_2H_5$ | B | 5,2s (1H); 3,2 2s (6H); 2,9m (2H); 1,7m (2H); 1,2d (3H) |
| 178 | 1 | $CH_3$ | 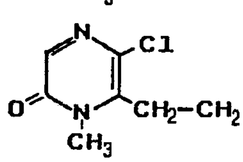 | $C_2H_5$ | B | 8,1s (1H); 3,2s (3H); 2,9m (2H); 1,7m (2H); 1,2d (3H) |

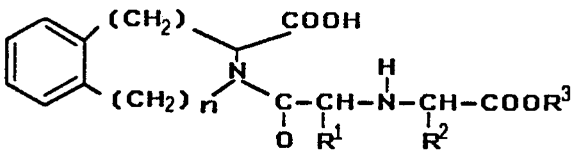

| | n | $R^1$ | $R^2$ | $R^3$ | Methode | NMR |
|---|---|---|---|---|---|---|
| 179 | 1 | $CH_3$ | 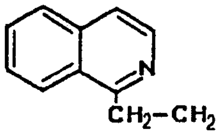 | $C_2H_5$ | B | 8,5 - 7,5m (6H); 3,1m (2H); 1,8m (2H); 1,2d (3H); |
| 180 | 1 | $CH_3$ | 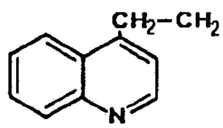 | $C_2H_5$ | B | 8,8 - 7,4m (6H); 3,0m (2H); 1,7m (2H); 1,2d (3H) |
| 181 | 1 | $CH_3$ | 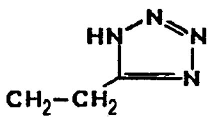 | $C_2H_5$ | B | 13,0s (1H); 2,9m (2H); 1,8m (2H); 1,2d (3H) |
| 182 | 1 | $CH_3$ | 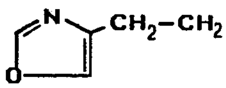 | $C_2H_5$ | B | 7,9 - 7,4 2s (2H); 2,8m (2H); 1,7m (2H); 1,2d (3H) |
| 183 | 1 | $CH_3$ | 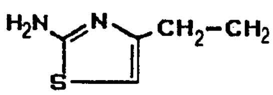 | $C_2H_5$ | B | 8,0s (1H); 2,8m (2H); 1,7m (2H); 1,2d (3H) |
| 184 | 1 | $CH_3$ | 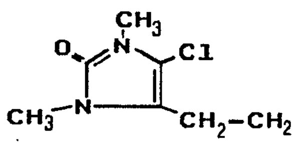 | $C_2H_5$ | B | 3,2 2s (6H); 3,1m (2H); 1,8m (2H); 1,2d (3H) |
| 185 | 1 | $CH_3$ | 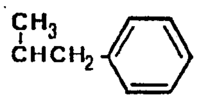 | $C_2H_5$ | B | 7,2bs (9H); 2,7m (2H); 2,2m (1H); 1,2d (3H); 1,0d (3H) |
| 186 | 1 | $H_2N-(CH_2)_4$ | 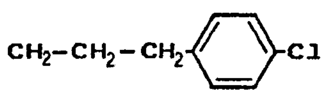 | H | B | 7,4 - 7,0m (8H); 2,7 - 2,3m (4H); 1,8 - 1,3m (10H) |
| 187 | 1 | $CH_3$ | 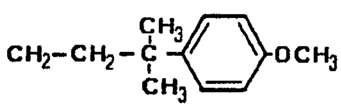 | $C_2H_5$ | B | 7,3 - 6,3m (8H); 3,9s (3H); 1,9 - 1,2m (4H); 1,2d (3H); 1,0s (6H) |
| 188 | 1 | 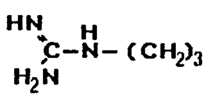 | 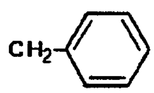 | H | B | 7,2bs (9H); 2,9 - 2,6m (4H); 1,7 - 1,3m m (4H); |
| 189 | 1 | H | 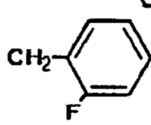 | H | B | 7,4 - 6,9m (8H); 3,9 - 3,0m (5H); 2,7m (2H) |
| 190 | 1 | H | 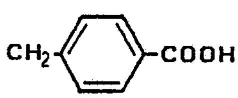 | H | B | 7,8 - 7,0m (8H); 3,9 - 3,0m (5H); 2,7m (2H) |
| 191 | 1 | H | 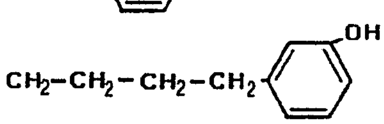 | H | B | 7,3 - 6,5m (8H); 3,9 - 3,0m (5H); 2,7m (2H); 1,8 - 1,3m (6H) |

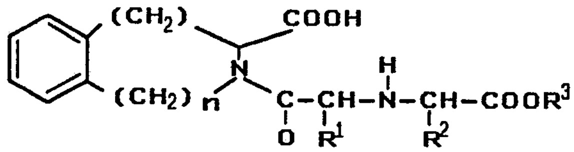

| | n | $R^1$ | $R^2$ | $R^3$ | Methode | NMR |
|---|---|---|---|---|---|---|
| 192 | 1 | $CH_3$ | 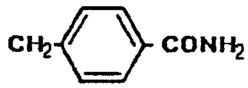 | H | B | 7,8 - 7,0m (8H); 2,8m (2H); 1,2d (3H) |
| 193 | 1 | $CH_3$ | 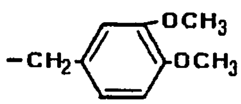 | H | B | 7,2 - 6,3m (7H); 3,9s (6H); 2,7m (2H); 1,2d (3H) |
| 194 | 1 | $(CH_3)_2CH-CH_2$ | 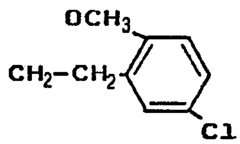 | $C_2H_5$ | B | 7,2 - 6,4m (1H); 3,8s (3H); 2,8m (2H); 2,0 - 1,5m (5H); 0,9d (6H) |
| 195 | 1 | $CH_3$ | 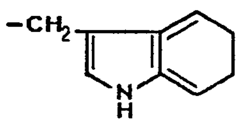 | $C_2H_5$ | B | 7,8 - 6,5m (10H); 2,9m (2H); 1,2d (3H) |
| 196 | 1 | $CH_3$ | 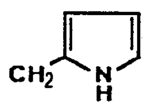 | H | B | 7,3 - 6,9m (7H); 2,8m (2H); 1,2d (3H) |
| 197 | 1 | $CH_3$ | 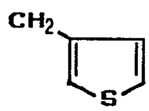 | H | B | 7,3 - 6,9m (7H); 2,9m (2H); 1,2d (3H) |
| 198 | 1 | $CH_3$ | 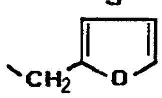 | H | B | 7,4 - 6,3m (7H); 2,8m (2H); 1,2d (3H) |
| 199 | 1 | $CH_3$ | 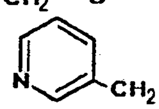 | H | B | 8,6 - 7,1m (8H); 2,9m (2H); 1,2d (3H) |
| 200 | 1 | $CH_3$ | 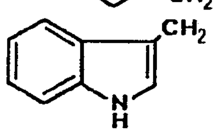 | H | B | 7,8 - 6,5m (10H); 2,9m (2H); 1,2d (3H) |
| 201 | 1 | $(CH_3)_2CH-CH_2$ | 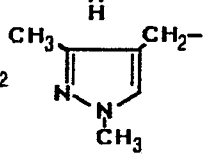 | $C_2H_5$ | B | 7,4 - 7,0m (5H); 3,9 - 3,0 m+s (7H); 2,8m (2H); 2,0s (3H); 1,8 - 1,3m (3H); 1,0d (6H) |
| 202 | 1 | H | 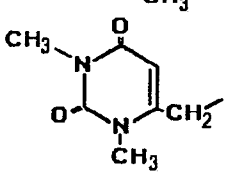 | $C_2H_5$ | B | 5,2s (1H); 3,2s (6H); 2,9m (2H) |
| 203 | 1 | $CH_3$ | 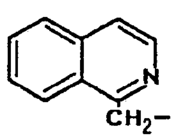 | H | B | 8,5 - 7,5m (6H); 3,1m (2H); 1,2d (3H) |

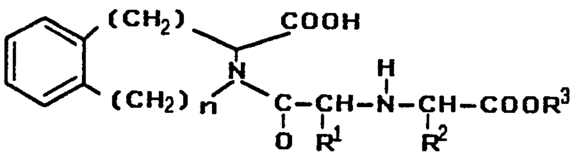

| | n | $R^1$ | $R^2$ | $R^3$ | Methode | NMR |
|---|---|---|---|---|---|---|
| 204 | 1 | $CH_3$ | 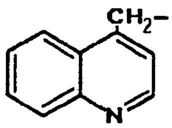 | H | B | 8,8 - 7,4m (6H); 3,0m (2H); 1,2d (3H) |
| 205 | 1 | $CH_3$ | 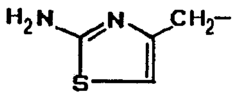 | H | B | 8,0s (1H); 2,8m (2H); 1,2d (3H) |
| 206 | 1 | $CH_3$ | 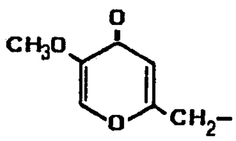 | H | B | 8,1s (1H); 6,4s (1H); 3,7s (3H); 2,9m (2H); 1,2d (3H) |
| 207 | 1 | $(CH_3)_2CH-CH_2$ | 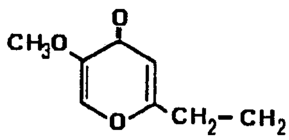 | H | B | 8,1s (1H); 6,4s (1H); 3,7s (3H); 2,9m (2H); 1,8 - 1,3m (5H); 1,0d (6H) |
| 208 | 1 | $CH_3$ | 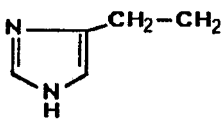 | $C_2H_5$ | B | 7,7 - 7,1m (6H); 2,9m (2H); 1,7m (2H); 1,2d (3H) |
| 209 | 1 | $CH_3$ | 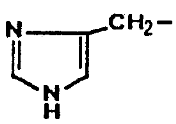 | $C_2H_5$ | B | 7,7 - 7,1m (6H); 2,9m (2H); 1,2d (3H) |
| 210 | 1 | $CH_3$ | $(CH_3)_2-CH-CH_2-CH_2-CH_2$ | $C_2H_5$ | A | 1,9 - 1,3m (7H); 1,2d (3H); 0,9d (6H) |
| 211 | 1 | $H_2N-(CH_2)_4$ | $(CH_3)_2CH-CH_2-CH_2$ | $C_2H_5$ | B | 2,4m (2H); 1,9 - 1,3m (11H); 0,9d (6H) |
| 212 | 1 | $H_2N-(CH_2)_3$ | $(CH_3)_2CH-CH_2$ | $C_2H_5$ | B | 2,4m (2H); 1,9 - 1,3m (7H); 0,9d (6H) |
| 213 | 1 | $CH_3-CH_2-\underset{CH_3}{CH}-$ | 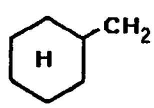 | H | A | 1,9 - 1,2m (16H); 1,0d+t (6H) |
| 214 | 1 | $CH_3$ | 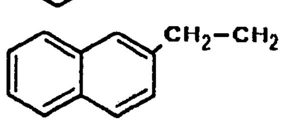 | $C_2H_5$ | A | 7,8 - 7,0m (11H); 2,7m (2H); 1,5m (2H); 1,2d (3H) |
| 215 | 1 | $CH_3$ | 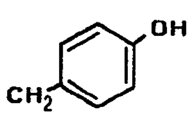 | H | B | 7,3 - 6,5m (8H); 2,8m (2H); 1,2d (3H) |
| 216 | 1 | $CH_3$ | 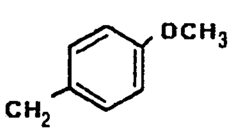 | H | B | 7,3 - 6,4m (8H); 3,9s (3H); 2,9m (2H); 1,2d (3H) |

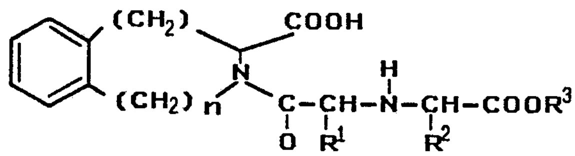

| | n | $R^1$ | $R^2$ | $R^3$ | Methode | NMR |
|---|---|---|---|---|---|---|
| 217 | 1 | $CH_3$ | 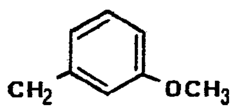 | $C_2H_5$ | B | 7,6 - 6,4m (8H); 3,8s (3H); 2,9m (2H); 1,2d (3H) |
| 218 | 1 | $CH_3$ | 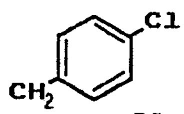 | $C_2H_5$ | B | 7,6 - 7,0m (8H); 2,9m (2H); 1,2d (3H) |
| 219 | 1 | $CH_3$ | 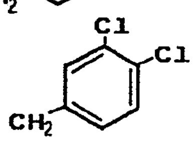 | H | B | 7,8 - 7,0m (7H); 2,9m (2H); 1,2d (3H) |
| 220 | 1 | $CH_3$ | 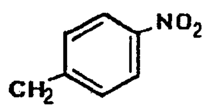 | H | B | 8,3 - 7,0m (8H); 2,9m (2H); 1,2d (3H) |
| 221 | 1 | $CH_3$ | 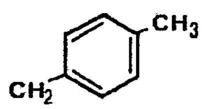 | $C_2H_5$ | B | 7,4 - 7,0m (8H); 2,9m (2H); 2,3s (3H); 1,2d (3H) |
| 222 | 1 | $CH_3$ | 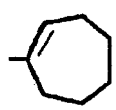 | H | A | 5,3m (1H); 2,3m (4H); 1,8 - 1,3m (6H); 1,2d (3H) |
| 223 | 1 | $CH_3$ | 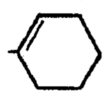 | H | A | 5,4m (1H); 2,3m (4H); 1,8 - 1,4m (4H); 1,2d (3H) |
| 224 | 1 | $CH_3$ | 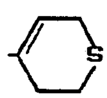 | H | A | 5,5m (1H); 3,9 - 3,0m (6H); 2,5 - 2,1m (4H); 1,2d (3H) |
| 225 | 1 | $CH_3$ | 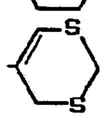 | H | A | 5,9s (1H); 3,9 - 3,0m (8H); 1,2d (3H) |
| | 1 | $CH_3$ | 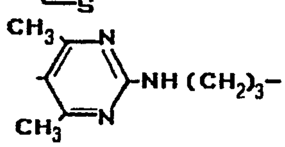 | $C_2H_5$ | A | 7,4s (1H); 2,5m (2H); 2,3s (6H); 1,8 - 1,2m (4H) |
| 226 | 1 | $CH_3$ | 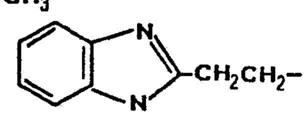 | $C_2H_5$ | A | 7,5-6,8m (8H); 2,8m (2H); 1,5m (2H) |
| 227 | 1 | $CH_3$ | 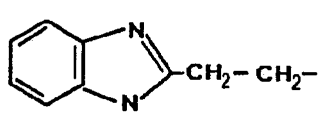 | H | A | 7,5 - 6,8m (8H); 2,8m (2H); 1,5m (2H) |

**Patentansprüche** für die Vertragsstaaten: BE, DE, GB, FR, IT, LU, LI, CH, NL, SE

1. Verbindung der Formel I'

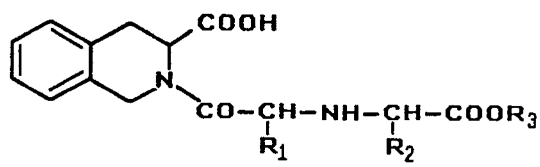

(I')

in welcher $R_1$ und $R_2$ gleich oder (verschieden sind und

a) Alkyl oder Alkenyl mit bis zu 6 C-Atomen bedeuten, welche jeweils gegebenenfalls substituiert sind durch einen oder mehrere gleiche oder verschiedene Substituenten aus der Reihe

1. ($C_5$-$C_7$)-Cycloalkyl,
2. ($C_5$-$C_7$)-Cycloalkenyl,
3. Fluor,
4. Mercapto,
5. Hydroxy,
6. ($C_1$-$C_2$)-Alkoxy, das im Alkylteil gegebenenfalls substituiert ist durch
   6.1 Methoxy,
   6.2 Ethoxy,
   6.3 Carboxy,
   6.4 Carbamoyl,
   6.5 Amino oder
   6.6 ($C_1$.$C_6$)-Alkylamino,
7. Aryloxy, das gegebenenfalls substituiert ist durch die unter a) 6.1 - 6.6 genannten Reste oder durch
   7.1 Halogen und/oder
   7.2 Nitro,
8. Aralkyloxy mit 1 oder 2 C-Atomen um Alkylteil, das im Alkylteil gegebenenfalls wie unter A) 6.1 - 6.6 beschrieben substituiert ist und das im Arylteil gegebenenfalls wie vorstehend im Alkylteil, wie unter a) 7.1 oder 7.2 beschrieben substituiert ist,
9. Amino,
10. Monoalkylamino mit bis zu 7 C-Atomen, das im Alkylrest gegebenenfalls substituiert ist durch
    10.1 Hydroxy,
    10.2 Carboxy,
    10.3 Carbamoyl,
    10.4 Ethoxycarbonyl,
    10.5 Amino,
    10.6 ($C_1$-$C_6$)-Alkylamino,
    10.7 Di-($C_1$-$C_6$)-alkylamino,
    10.8 Piperidino oder
    10.9 Morpholino,
11. Dialkylamino mit bis zu 7 C-Atomen, das im Alkylrest gegebenenfalls wie unter a) 10.1 - 10.9 beschrieben substituiert ist,
12. Cycloalkylamino mit bis zu 7 C-Atomen, das im Alkylrest gegebenenfalls wie unter a) 10.1 - 10.9 beschrieben substituiert ist,
13. Dicycloalkylamino mit bis zu 7 C-Atomen, das im Alkylrest gegebenenfalls wie unter a) 10.1 - 10.9 beschrieben substituiert ist,
14. ($C_1$-$C_6$)-Alkoxycarbonylamino,
15. Aryloxycarbonylamino, das im Arylteil gegebenenfalls mono- oder disubstituiert ist durch
    15.1 ($C_1$-$C_2$)-Alkyl,
    15.2 ($C_1$-$C_2$)Alkoxy,
    15.3 Methylendioxy,
    15.4 Amino,
    15.5 Hydroxy,
    15.6 Acetoxy,
    15.7 Carboxy,
    15.8 Carbamoyl,
    15.9 Ethoxycarbonyl,
    15.10 Halogen oder
    15.11 Nitro,
16. Aralkyloxycarbonylamino, das im Arylteil gegebenenfalls wie unter a) 15.1 - 15.11 beschrieben substituiert ist,

17. $(C_1-C_6)$-Alkylureido,
18. Arylureido, das im Arylteil gegebenenfalls wie unter a) 15.1 - 15.11 beschrieben durch gleiche oder verschiedene Reste mono - oder disubstituiert ist,
19. Aralkylureido, das im Arylteil gegebenenfalls wie unter a) 15.1 - 15.11 beschrieben mono - oder disubstituiert ist,
20. Formyl,
21. Alkanoylamino mit bis zu 10 C-Atomen,
22. Aroylamino mit bis zu 10 C-Atomen, das im Arylteil gegebenenfalls wie unter a) 15.1 - 15.11 beschrieben mono - oder disubstituiert ist,
23. Aralkanoylamino mit bis zu 10 C-Atomen, das im Arylteil gegebenenfalls wie unter a) 15.1 - 15.11 beschrieben mono - oder disubstituiert ist,
24. Arylamino, das im Arylteil gegebenenfalls wie unter a) 15.1 - 15.11 beschrieben durch gleiche oder verschiedene Reste mono - oder disubstituiert ist,
25. Aralkylamino, das im Arylteil gegebenenfalls wie unter a) 15.1 - 15.11 beschrieben substituiert ist,
26. Alkylmercapto, Alkylsulfinyl oder Alkylsulfonyl mit bis zu 7 C-Atomen, das jeweils im Alkylteil gegebenenfalls substituiert ist durch
    26.1 Methoxy,
    26.2 Ethoxy,
    26.3 Hydroxy
    26.4 Carboxy,
    26.5 Carbamoyl
    26.6 Ethoxycarbonyl
    26.7 Amino oder
    26.8 $(C_1-C_6)$Alkylamino,
27. Phenylmercapto, Phenylsulfinyl oder Phenylsulfonyl, das im Arylteil gegebenenfalls wie unter a) 26.1 - 26.8 beschrieben oder durch
    27.1 Halogen,
    27.2 Nitro oder
    27.3 Sulfamoyl substituiert ist,
28. Benzylmercapto, Benzylsulfinyl oder Benzylsulfonyl, das im Alkylteil gegebenenfalls wie unter a) 26.1 - 26.8 und das im Arylteil gegebenenfalls wie vorstehender Alkylteil oder wie unter a) 27.1 - 27.3 beschrieben substituiert ist,
29. Carboxy,
30. Ethoxycarbonyl
31. Carbamoyl,
32. Alkylaminocarbonyl mit bis zu 6 C-Atomen,
33. Cycloalkylcarbamoyl mit bis zu 6 C-Atomen,
34. Cycloalkylenaminocarbonyl mit bis zu 6 C-Atomen,
35. Dialkylcarbamoyl mit bis zu 6 C-Atomen,
36. Arylcarbamoyl, das im Arylteil gegebenenfalls substituiert ist durch
    36.1 Halogen,
    36.2 $(C_1-C_6)$-Alkyl oder
    36.3 $(C_1-C_6)$-Alkoxy,
37. Aralkylcarbamoyl,
38. Guanidino,
39. Phenyl, Naphthyl, Dihydronaphthyl oder Tetrahydronaphthyl, die jeweils gegebenenfalls mono - oder disubstituiert sind durch
    39.1 Halogen,
    39.2 Hydroxy
    39.3 Acetoxy
    39.4 Carboxy,
    39.5 Carbamoyl,
    39.6 Sulfamoyl,
    39.7 Nitro,
    39.8 Methyl,
    39.9 Ethyl,
    39.10 Methoxy und/oder
    39.12 Ethoxy,
40. einen 5- bis 7-gliedrigen monocyclischen oder 9- bis 10-gliedrigen bicyclischen Heterocyclen-Rest, gegebenenfalls 1 bis 2 S- oder O-Atome und/oder bis zu 4 N-Atome pro Ring enthaltend, der gegebenenfalls substituiert durch
    40.1 Halogen,
    40.2 Sauerstoff,
    40.3 Hydroxy,
    40.4 Carboxy,

40.5 Carbamoyl,
40.6 Sulfamoyl,
40.7 Nitro,
40.7 Alkyl mit bis zu 9 C-Atomen,
40.9 Aralkyl mit bis zu 9 C-Atomen,
40.10 Methoxy und/oder
40.11 Ethoxy;

b) Cycloalkyl oder Cycloalkenyl mit je 5 - 7 C-Atomen bedeuten;
c) Aryl oder teilhydriertes Aryl bedeuten; oder
d) einen mono- oder bicyclischen Heterocyclen-Rest mit 5 - 7 bzw. 8 - 10 Gliedern, davon 1 - 2 -S- oder -O- und/oder bis zu 4 N-Atomen bedeuten, das gegebenenfalls wie unter a) 40.1 - 40.11 beschrieben substituiert ist,
und

$R_3$ Wasserstoff, Alkyl mit 1 bis 6 C-Atomen, Alkenyl mit 2 bis 6 C-Atomen, Aralkyl mit 7 bis 14 C-Atomen inklusive p-Nitrobenzyl bedeutet,
wobei, falls im einzelnen nicht anders definiert, Aryl 6 bis 10 C-Atome aufweist und gegebenenfalls durch Halogen, ($C_1$-$C_6$)-Alkyl oder ($C_1$-$C_6$)-Alkoxy substituiert ist, und
Aralkyl Benzyl oder Phenethyl bedeutet und gegebenenfalls durch Halogen, ($C_1$-$C_6$)-Alkyl oder ($C_1$-$C_6$)-Alkoxy im Phenylkern substituiert ist, und deren physiologisch verträglichen Salze.

2. Verbindung gemäß Anspruch 1, in welcher $R_1$ Methyl und $R_2$ Phenethyl oder durch Halogen, Methyl oder Methoxy substituiertes Phenethyl ist.

3. Verbindung gemäß Anspruch 1 oder 2, in welcher $R_3$ Wasserstoff, Ethyl, Butyl, t-Butyl, Benzyl, p-Nitrobenzyl bedeutet.

4. Verbindung gemäß Anspruch 1 oder 3, in welcher $R_1$ die Seitenkette einer natürlich vorkommenden L-Aminosäure oder deren Ethers, Esters oder Amids bedeutet.

5. Verbindung gemäß Anspruch 4, in welcher $R_1$ Methyl, Isobutyl, Methylthioethyl, Carboxymethyl, Carboxyethyl, Amino-n-butyl, Guanidino-n-propyl, Imidazol-4-ethyl, Benzyl, 4-Hydroxybenzyl oder 3-Indolmethyl bedeutet.

6. Verbindung gemäß Anspruch 1 oder 3, in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

7. Verbindung gemäß Anspruch 1 oder 3, in welcher $R_2$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

8. 1-[N-(1-Carboxy-3-phenyl-propyl)-(S)-alanyl]-1,2,3,4-tetrahydroisochinolin-3-carbonsäure oder deren physiologisch verträgliches Salz.

9. 1-[N-(1-Ethoxycarbonyl-3-phenyl-propyl)-(S)-alanyl]-1,2,3,4-tetrahydroisochinolin-3-carbonsäure oder deren physiologisch verträglichem Salz.

10. Verbindung gemäß einem der Ansprüche 1 bis 9, in welcher die drei chiralen Zentren an α-Atomen in der L-Konfiguration vorliegen.

11. Verfahren zur Herstellung einer Verbindung der Formel I', gemäß einem der Ansprüche 1 bis 10, dadurch gekennzeichnet, daß man

a) eine Verbindung der Formel II' mit einer Verbindung der Formel III umsetzt

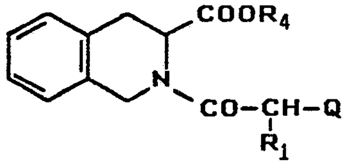
(II')

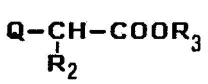
(III)

in denen $R_1$, $R_2$ und $R_3$ wie im Anspruch 1 deiniert sind, Q eine nucleofuge (Gruppe und das andere Q -$NH_2$ bedeutet und $R_4$ für H, Methyl, Ethyl, enzyl oder tert.-Butyl steht, und gegebenenfalls einen erhaltenen Ester in die Carbonsäure ($R_2$ und/oder $R_4$ = Wasserstoff) überführt oder

b) eine Verbindung der Formel IV

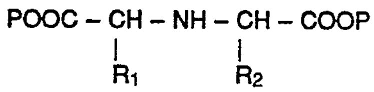

(IV)

worin $R_1$ und $R_2$ wie im Anspruch 1 definiert sind, P H bedeutet, eines der beiden P jedoch auch die anderen Bedeutungen von $R_3$ im Anspruch 1 inklusive tert.-Butyl haben kann, mit einer Verbindung der Formel V'

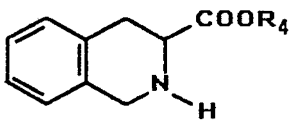

(V')

in der $R_4$ wie oben definiert ist, in Gegenwart eines Kondensationsmittels umsetzt und gegebenenfalls eine oder beide Estergruppen in die Carbonsäure überführt oder

c) eine Verbindung der Formel VI' mit einer Verbindung der Formel VII umsetzt

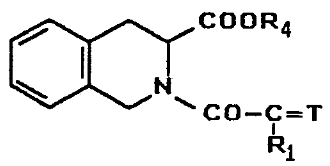

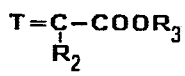

(VI') (VII)

in denen $R_1$, $R_2$ und $R_3$ wie im Anspruch 1 und $R_4$ wie oben definiert sind, ein T für ein Wasserstoffatom und eine $NH_2$-Gruppe und das andere T für ein Sauerstoffatom steht, und die erhaltene Schiff'sche Base reduziert,

in der nach a) bis c) erhaltene Verbindung Schwefelfunktionen gegebenenfalls zum Sulfoxid oder zum Sulfon oxidiert und die erhaltene Verbindung der Formel I' gegebenenfalls in ihr Salz überführt.

12. Mittel) enthaltend eine Verbindung gemäß einem der Ansprüche 1 bis 10 oder deren physiologisch verträgliches Salz.

13. Verbindung gemäß einem der Ansprüche 1 bis 10 zur Anwendung als Heilmittel.

14. Verbindung gemäß einem der Ansprüche 1 bis 10 zur Anwendung als Heilmittel bei der Behandlung des Bluthochdrucks.

**Patentansprüche** für den Vertragstaat: AT

1. Verfahren zur Herstellung einer Verbindung der Formel I'

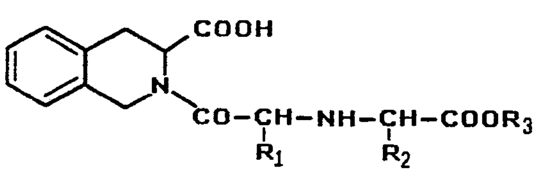

(I)

in welcher $R_1$ und $R_2$ gleich oder (verschieden sind und

a) Alkyl oder Alkenyl mit bis zu 6 C-Atomen bedeuten, welche jeweils gegebenenfalls substituiert sind durch einen oder mehrere gleiche oder verschiedene Substituenten aus der Reihe
   1. ($C_5$-$C_7$)-Cycloalkyl,

2. $(C_5-C_7)$-Cycloalkenyl,
3. Fluor,
4. Mercapto,
5. Hydroxy,
6. $(C_1-C_2)$-Alkoxy, das im Alkylteil gegebenenfalls substituiert ist durch
   6.1 Methoxy,
   6.2 Ethoxy,
   6.3 Carboxy,
   6.4 Carbamoyl,
   6.5 Amino oder
   6.6 $(C_1C_6)$-Alkylamino,
7. Aryloxy, das gegebenenfalls substituiert ist durch die unter a) 6.1 - 6.6 genannten Reste oder durch
   7.1 Halogen und/oder
   7.2 Nitro,
8. Aralkyloxy mit 1 oder 2 C-Atomen um Alkylteil, das im Alkylteil gegebenenfalls wie unter a) 6.1 - 6.6 beschrieben substituiert ist und das im Arylteil gegebenenfalls wie vorstehend im Alkylteil, wie unter a) 7.1 oder 7.2 beschrieben substituiert ist,
9. Amino,
10. Monoalkylamino mit bis zu 7 C-Atomen, das im Alkylrest gegebenenfalls substituiert ist durch
    10.1 Hydroxy,
    10.2 Carboxy,
    10.3 Carbamoyl,
    10.4 Ethoxycarbonyl,
    10.5 Amino,
    10.6 $(C_1-C_6)$-Alkylamino,
    10.7 Di-$(C_1-C_6)$-alkylamino,
    10.8 Piperidino oder
    10.9 Morpholino,
11. Dialkylamino mit bis zu 7 C-Atomen, das im Alkylrest gegebenenfalls wie unter a) 10.1 - 10.9 beschrieben substituiert ist,
12. Cycloalkylamino mit bis zu 7 C-Atomen, das im Alkylrest gegebenenfalls wie unter a) 10.1 - 10.9 beschrieben substituiert ist,
13. Dicycloalkylamino mit bis zu 7 C-Atomen, das im Alkylrest gegebenenfalls wie unter a) 10.1 - 10.9 beschrieben substituiert ist,
14. $(C_1-C_6)$-Alkoxycarbonylamino,
15. Aryloxycarbonylamino, das im Arylteil gegebenenfalls mono- oder disubstituiert ist durch
    15.1 $(C_1-C_2)$-Alkyl,
    15.2 $(C_1C_2)$Alkoxy,
    15.3 Methylendioxy,
    15.4 Amino,
    15.5 Hydroxy,
    15.6 Acetoxy,
    15.7 Carboxy,
    15.8 Carbamoyl,
    15.9 Ethoxycarbonyl,
    15.10 Halogen oder
    15.11 Nitro,
16. Aralkyloxycarbonylamino, das im Arylteil gegebenenfalls wie unter a) 15.1 - 15.11 beschrieben substituiert ist,
17. $(C_1-C_6)$-Alkylureido,
18. Arylureido, das im Arylteil gegebenenfalls wie unter a) 15.1 - 15.11 beschrieben durch gleiche oder verschiedene Reste mono - oder disubstituiert ist,
19. Aralkylureido, das im Arylteil gegebenenfalls wie unter a) 15.1 - 15.11 beschrieben mono - oder disubstituiert ist,
20. Formyl,
21. Alkanoylamino mit bis zu 10 C-Atomen,
22. Aroylamino mit bis zu 10 C-Atomen, das im Arylteil gegebenenfalls wie unter a) 15.1 - 15.11 beschrieben mono - oder disubstituiert ist,
23. Aralkanoylamino mit bis zu 10 C-Atomen, das im Arylteil gegebenenfalls wie unter a) 15.1 - 15.11 beschrieben mono - oder disubstituiert ist,
24. Arylamino, das im Arylteil gegebenenfalls wie unter a) 15.1 - 15.11 beschrieben durch gleiche oder verschiedene Reste mono - oder disubstituiert ist,
25. Aralkylamino, das im Arylteil gegebenenfalls wie unter a) 15.1 - 15.11 beschrieben substituiert ist,
26. Alkylmercapto, Alkylsulfinyl oder Alkylsulfonyl mit bis zu 7 C-Atomen, das jeweils im Alkylteil gegebenenfalls substituiert ist durch
    26.1 Methoxy,

26.2 Ethoxy
26.3 Hydroxy
26.4 Carboxy,
26.5 Carbamoyl
26.6 Ethoxycarbonyl
26.7 Amino oder
26.8 ($C_1$-$C_6$)Alkylamino,

27. Phenylmercapto, Phenylsulfinyl oder Phenylsulfonyl, das im Arylteil gegebenenfalls wie unter a) 26.1 - 26.8 beschrieben oder durch
27.1 Halogen,
27.2 Nitro oder
27.3 Sulfamoyl substituiert ist,
28. Benzylmercapto, Benzylsulfinyl oder Benzylsulfonyl, das im Alkylteil gegebenenfalls wie unter a) 26.1 - 26.8 und das im Arylteil gegebenenfalls wie vorstehender Alkylteil oder wie unter a) 27.1 - 27.3 beschrieben substituiert ist,
29. Carboxy,
30. Ethoxycarbonyl
31. Carbamoyl,
32. Alkylaminocarbonyl mit bis zu 6 C-Atomen,
33. Cycloalkylcarbamoyl mit bis zu 6 C-Atomen,
34. Cycloalkylenaminocarbonyl mit bis zu 6 C-Atomen,
35. Dialkylcarbamoyl mit bis zu 6 C-Atomen,
36. Arylcarbamoyl, das im Arylteil gegebenenfalls substituiert ist durch
36.1 Halogen,
36.2 ($C_1$-$C_6$)-Alkyl oder
36.3 ($C_1$-$C_6$)-Alkoxy,
37. Aralkylcarbamoyl,
38. Guanidino,
39. Phenyl, Naphthyl, Dihydronaphthyl oder Tetrahydronaphthyl, die jeweils gegebenenfalls mono - oder disubstituiert sind durch
39.1 Halogen,
39.2 Hydroxy
39.3 Acetocy
39.4 Carboxy,
39.5 Carbamoyl,
39.6 Sulfamoyl,
39.7 Nitro,
39.8 Methyl,
39.9 Ethyl,
39.10 Methoxy und/oder
39.12 Ethoxy,
40. einen 5- bis 7-gliedrigen monocyclischen oder 9- bis 10-gliedrigen bicyclischen Heterocyclen-Rest, gegebenenfalls 1 bis 2 S- oder O-Atome und/oder bis zu 4 N-Atome pro Ring enthaltend, der gegebenenfalls substituiert durch
40.1 Halogen,
40.2 Sauerstoff,
40.3 Hydroxy,
40.4 Carboxy,
40.5 Carbamoyl,
40.6 Sulfamoyl,
40.7 Nitro,
40.7 Alkyl mit bis zu 9 C-Atomen,
40.9 Aralkyl mit bis zu 9 C-Atomen,
40.10 Methoxy und/oder
40.11 Ethoxy;

b) Cycloalkyl oder Cycloalkenyl mit je 5 - 7 C-Atomen bedeuten;
c) Aryl oder teilhydriertes Aryl bedeuten; oder
d) einen mono- oder bicyclischen Heterocyclen-Rest mit 5 - 7 bzw. 8 - 10 Gliedern, davon 1 - 2 -S- oder -O- und/oder bis zu 4 N-Atomen bedeuten, das gegebenenfalls wie unter a) 40.1 - 40.11 beschrieben substituiert ist,
und

$R_3$ Wasserstoff, Alkyl mit 1 bis 6 C-Atomen, Alkenyl mit 2 bis 6 C-Atomen, Aralkyl mit 7 bis 14 C-Atomen inklusive p-Nitrobenzyl bedeutet,

wobei, falls im einzelnen nicht anders definiert, Aryl 6 bis 10 C-Atome aufweist und gegebenenfalls durch Halogen, ($C_1$-$C_6$)-Alkyl oder ($C_1$-$C_6$)-Alkoxy substituiert ist, und
Aralkyl Benzyl oder Phenethyl bedeutet und gegebenenfalls durch Halogen, ($C_1$-$C_6$)-Alkyl oder ($C_1$-$C_6$)Alkoxy im Phenylkern substituiert ist, oder deren physiologisch verträglichen Salze. *dadurch gekennzeichnet,* daß man

a) eine Verbindung der Formel II' mit einer Verbindung der Formel III umsetzt

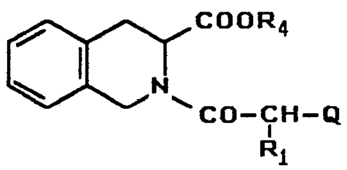

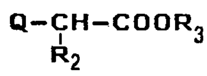

(II') (III)

in denen $R_1$, $R_2$ und $R_3$ wie oben definiert sind, ein Q eine nucleofuge Gruppe und das andere Q -$NH_2$ bedeutet und $R_4$ für H, Methyl, Ethyl, Benzyl oder tert.-Butyl steht, und gegebenenfalls einen erhaltenen Ester in die Carbonsäure ($R_2$ und/oder $R_4$ = Wasserstoff) überführt oder

b) eine Verbindung der Formel IV

POOC – CH – NH – CH – COOP (IV)
       |              |
      $R_1$           $R_2$                                    (IV)

worin $R_1$ und $R_2$ wie oben definiert sind, P H bedeutet, eines der beiden P jedoch auch die anderen oben definierten Bedeutungen von $R_3$ inklusive tert.-Butyl haben kann, mit einer Verbindung der Formel V'

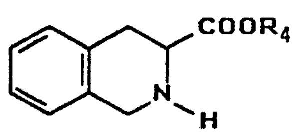

(V')

in der $R_4$ wie oben definiert ist, in Gegenwart eines Kondensationsmittels umsetzt und gegebenenfalls eine oder beide Estergruppen in die Carbonsäure überführt
oder

c) eine Verbindung der Formel VI' mit einer Verbindung der Formel VII umsetzt

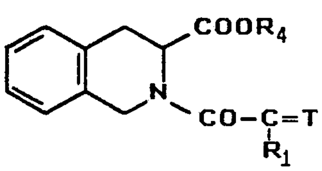

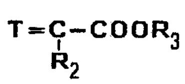

(VI') (VII)

in denen $R_1$, $R_2$, $R_3$ und $R_4$ wie oben definiert sind, ein T für ein Wasserstoffatom und eine $NH_2$-Gruppe und das andere T für ein Sauerstoffatom steht, und die erhaltene Schiff'sche Base reduziert,

in der nach a) bis c) erhaltenen Verbindung Schwefelfunktionen gegebenenfalls zum Sulfoxid oder zum Sulfon oxidiert und die erhaltene Verbindung der Formel I' gegebenenfalls in das Salz überführt.

2. Verfahren gemäß Anspruch 1, dadurch gekennzeichnet, daß eine Verbindung hergestellt wird, in der $R_1$ Methyl und $R_2$ Phenethyl oder durch Halogen, Methyl oder Methoxy substituiertes Phenethyl ist.

3. Verfahren gemäß Anspruch 1 oder 2 zur Herstellung einer Verbindung der Formel I', in welcher $R_3$ Wasserstoff, Ethyl, Butyl, t-Butyl, Benzyl, p-Nitrobenzyl bedeutet.

4. Verfahren gemäß Anspruch 1 oder 3 zur Herstellung einer Verbindung der Formel I' in welcher $R_1$ die Seitenkette

einer natürlich vorkommenden L-Aminosäure oder deren Ethers, Esters oder Amids bedeutet.

5. Verfahren gemäß Anspruch 4 zur Herstellung einer Verbindung der Formel I', in welcher $R_1$ Methyl, Isobutyl, Methylthioethyl, Carboxymethyl, Carboxyethyl, Amino-n-butyl, Guanidino-n-propyl, Imidazol-4-ethyl, Benzyl, 4-Hydroxybenzyl oder 3-Indolmethyl bedeutet.

6. Verfahren gemäß Anspruch 1 oder 3 zur Herstellung einer Verbindung der Formel I', in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

7. Verfahren gemäß Anspruch 1 oder 3 zur Herstellung einer Verbindung der Formel I', in welcher $R_2$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

8. Verfahren gemäß einem der Ansprüche 1 bis 7 zur Herstellung von

1-[N-(1-Carboxy-3-phenyl-propyl)-(S)-alanyl]-1,2,3,4-tetrahydroisochinolin-3-carbonsäue oder deren physiologisch verträglichem Salz.

9. Verfahren gemäß einem der Ansprüche 1 bis 7 zur Herstellung von
1-[N-(1-Ethoxycarbonyl-3-phenyl-propyl)-(S)-alanyl]-1,2,3,4-tetrahydroisochinolin-3-cabonsäure oder deren physiologisch verträglichem Salz.

10. Verfahren gemäß einem der Ansprüche 1 bis 9 zur Herstellung einer Verbindung der Formel I', in welcher die drei chiralen Zentren an α-C-Atomen in der L,-Konfiguration vorliegen.

11. Verfahren zur Herstellung eines Mittels, enthaltend eine Verbindung der Formel I' gemäß einem der Ansprüche 1 bis 10 oder deren physiologisch verträgliches Salz, dadurch gekennzeichnet, daß man diese in eine geeignete Darreichungsform bringt.

12. Verfahren gemäß einem der Ansprüche 1 bis 10 zur Herstellung einer Verbindung der Formel I' zur Anwendung als Heilmittel.

13. Verfahren gemäß einem der Ansprüche 1 bis 10 zur Herstellung einer Verbindung der Formel I' zur Anwendung als Heilmittel bei der Behandlung des Bluthochdrucks.

**Claims** for the Contracting States: BE, DE, GB, FR, IT, LU, LI, CH, NL, SE

1. A compound of the formula I'

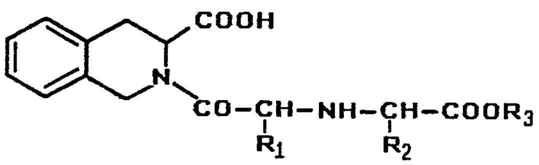

(I')

in which $R_1$ and $R_2$ are identical or different and denote

a) alkyl or alkenyl having up to 6 C atoms, each of which may be substituted by one or more indentical or different substituents from the series comprising
   1. ($C_5$-$C_7$)-cycloalkyl,
   2. ($C_5$-$C_7$)-cycloalkenyl,
   3. fluorine,
   4. mercapto,
   5. hydroxyl,
   6. ($C_1$-$C_2$) -alkoxy which may be substituted in the alkyl moiety by
      6.1 methoxy,
      6.2 ethoxy,

6.3 carboxyl,
6.4 carbamoyl,
6.5 amino or
6.6 ($C_1$-$C_6$)-alkylamino,

7. aryloxy which may be substituted by the radicals stated under a) 6.1 - 6.6 or by
   7.1 halogen and/or
   7.2 nitro,
8. aralkyloxy having 1 or 2 C atoms in the alkyl moiety, which may be substituted in the alkyl moiety as described under a) 6.1 - 6.6 and which may be substituted in the aryl moiety as described above for the alkyl moiety or as described under a) 7.1 or 7.2,
9. amino,
10. monoalkylamino having up to 7 C atoms, which may be substituted in the alkyl radical by
    10.1 hydroxyl,
    10.2 carboxyl,
    10.3 carbamoyl,
    10.4 ethoxycarbonyl,
    10.5 amino,
    10.6 ($C_1$-$C_6$)-alkylamino,
    10.7 di-($C_1$-$C_6$)-alkylamino,
    10.8 piperidino or
    10.9 morpholino,
11. dialkylamino having up to 7 C atoms, which may be substituted in the alkyl radical as described under a) 10.1-10.9,
12. cycloalkylamino having up to 7 C atoms, which may be substituted in the alkyl radical as described under a) 10.1-10.9,
13. dicycloalkylamino having up to 7 C atoms, which may be substituted in the alkyl radical as described under a) 10.1-10.9,
14. ($C_1$-$C_6$)-alkoxycarbonylamino,
15. aryloxycarbonylamino which may be monosubstituted or disubstituted in the aryl moiety by
    15.1 ($C_1$-$C_2$)-alkyl,
    15.2 ($C_1$-$C_2$)-alkoxy,
    15.3 methylenedioxy,
    15.4 amino,
    15.5 hydroxyl,
    15.6 acetoxy,
    15.7 carboxyl,
    15.8 carbamoyl,
    15.9 ethoxycarbonyl,
    15.10 halogen or
    15.11 nitro,
16. aralkyloxycarbonylamin which may be substituted in the aryl moiety as described under a) 15.1-15.11,
17. ($C_1$-$C_6$)-alkylureido,
18. arylureido which may be monosubstituted or disubstituted in the aryl moiety by identical or different radicals as described under a) 15.1 - 15.11,
19. aralkylureido which may be monosubstituted or disubstituted in the aryl moiety as described under a) 15.1 - 15.11,
20. formyl,
21. alkanoylamino having up to 10 C atoms,
22. aroylamino having up to 10 C atoms, which may be monosubstituted or disubstituted in the aryl moiety as described under a) 15.1 - 15.11,
23. aralkanoylamino having up to 10 C atoms, which may be monosubstituted or disubstituted in the aryl moiety as described under a) 15.1 - 15.11,
24. arylamino which may be monosubstituted or disubstituted in the aryl moiety by identical or different radicals as described under a) 15.1 - 15.11,
25. aralkylamino which may be substituted in the aryl moiety as described under a) 15.1 - 15.11,
26. alkylmercapto, alkylsulfinyl or alkylsulfonyl having up to 7 C atoms, each of which may be substituted in the alkyl moiety by
    26.1 methoxy,
    26.2 ethoxy,
    26.3 hydroxyl,
    26.4 carboxyl,
    26.5 carbamoyl,
    26.6 ethoxycarbonyl,
    26.7 amino or
    26.8 ($C_1$-$C_6$)-alkylamino,

27. phenylmercapto, phenylsulfinyl or phenylsulfonyl, which may be substituted in the aryl moiety as described under a) 26.1 - 26.8 or by
    27.1 halogen,
    27.2 nitro or
    27.3 sulfamoyl,
28. benzylmercapto, benzylsulfinyl or benzylsulfonyl, which may be substituted in the alkyl moiety as described under a) 26.1 - 26.8 and which may be substituted in the aryl moiety as for the above alkyl moiety or as described under a) 27.1 - 27.3,
29. carboxyl,
30. ethoxycarbonyl,
31. carbamoyl,
32. alkylaminocarbonyl having up to 6 C atoms,
33. cycloalkylcarbamoyl having up to 6 C atoms,
34. cycloalkyleneaminocarbonyl having up to 6 C atoms,
35. dialkylcarbamoyl having up to 6 C atoms,
36. arylcarbamoyl which may be substituted in the aryl moiety by
    36.1 halogen,
    36.2 ($C_1$-$C_6$)alkyl or
    36.3 ($C_1$-$C_6$)-alkoxy,
37. aralkylcarbamoyl,
38. guanidino,
39. phenyl, naphthyl, dihydronaphthyl or tetrahydronaphthyl, each of which may be monosubstituted or disubstituted by
    39.1 halogen,
    39.2 hydroxyl,
    39.3 acetoxy,
    39.4 carboxyl,
    39.5 carbamoyl,
    39.6 sulfamoyl,
    39,7 nitro,
    39.8 methyl,
    39.9 ethyl,
    39.10 methoxy and/or
    39.11 ethoxy,
40. a 5-membered to 7 membered monocyclic or 9 membered or 10-membered bicyclic heterocyclic radical which may contain 1 or 2 S or O atoms and/or up to 4 N atoms per ring and which may be substituted by
    40.1 halogen,
    40.2 oxygen,
    40.3 hydroxyl,
    40.4 carboxyl,
    40.5 carbamoyl,
    40.6 sulfamoyl,
    40.7 nitro,
    40.8 alkyl having up to 9 C atoms,
    40.9 aralkyl having up to 9 C atoms,
    40.10 methoxy and/or
    40.11 ethoxy;

b) denote cycloalkyl or cycloalkenyl, each having 5 - 7 C atoms;
c) denote aryl or partially hydrogenated aryl; or
d) denote a monocyclic or bicyclic heterocyclic radical having 5 - 7 or 8 - 10 members, including 1 or 2 -S- or -O- and/or up to 4 N atoms, which radical may be substituted as described under a) 40.1 - 40.11,
   and
   $R_3$ denotes hydrogen, alkyl having 1 to 6 C atoms, alkenyl having 2 to 6 C atoms, aralkyl having 7 to 14 C atoms, including p-nitrobenzyl, and, unless specifically defined otherwise, aryl has 6 to 10 C atoms and may be substituted by halogen, ($C_1$-$C_6$) -alkyl or ($C_1$-$C_6$)-alkoxy, and denotes aralkyl, benzyl or phenethyl and may be substituted by halogen, ($C_1$-$C_6$)-alkyl or ($C_1$-$C_6$)-alkoxy in the phenyl nucleus, or their physiologically tolerated salts.

2. A compound as claimed in claim 1, wherein $R_1$ is methyl and $R_2$ is phenethyl or phenethyl which is substituted by halogen, methyl or methoxy.

3. A compound as claimed in claim 1 or 2, wherein $R_3$ is hydrogen, ethyl, butyl, tert-butyl, benzyl or p-nitrobenzyl.

4. A compound as claimed in claim 1 or 3, wherein $R_1$ denotes the side chain of a naturally occurring L-amino acid or its ether, ester or amide.

5. A compound as claimed in claim 4, wherein $R_1$ is methyl, isobutyl, methylthioethyl, carboxymethyl, carboxyethyl, amino-n-butyl, guanidino-n-propyl, imidazol-4-ethyl, benzyl, 4-hydroxybenzyl or 3-indolmethyl.

6. A compound as claimed in claim 1 or 3, wherein $R_1$ is methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, di- or tetrahydronaphthyl, benzyl, phenethyl, p-fluorophenethyl, o-methylphenethyl, p-methoxyphenethyl, 2,4-dichlorophenethyl or cyclohexylethyl.

7. A compound as claimed in claim 1 or 3, wherein $R_2$ is methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, di- or tetrahydronaphthyl, benzyl, phenethyl, p-fluorophenethyl, o-methylphenethyl, p-methoxyphenethyl, 2,4-dichlorophenethyl or cyclohexylethyl.

8. 1-[N-(1-Carboxy-3-phenylpropyl)-(S)-alanyl]1,2,3,4-tetrahydroisoquinoline-3-carboxylic acid or its physiologically tolerated salt.

9. 1-[N-(1-ethoxycarbonyl-3-phenylpropyl)-(S)alanyl]-1,2,3,4-tetrahydroisoquinoline-3-carboxylic acid or its physiologically tolerated salt.

10. A compound as claimed in any of claims 1 to 9, wherein the three chiral centers are present at α-atoms in the L configuration.

11. A process for the preparation of a compound of the formula I' as claimed in any one of claims 1 to 10, which comprises

a) reacting a compound of the formula II' with a compound of the formula III

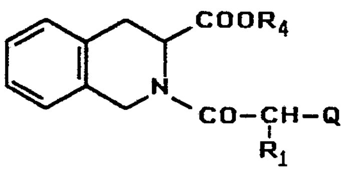 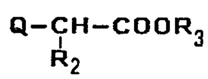

(II') (III)

in which $R_1$, $R_2$ and $R_3$ are as defined in claim 1, Q denotes a nucleofugic group and the other Q denotes $-NH_2$, and $R_4$ represents H, methyl, ethyl, benzyl or tert-butyl, and, if appropriate, converting an ester obtained into the carboxylic acid ($R_2$ and/or $R_4$ = hydrogen), or

b) reacting a compound of the formula IV

POOC – CH – NH – CH – COOP (IV)
with $R_1$ on the first CH and $R_2$ on the second CH

wherein $R_1$ and $R_2$ are as defined in claim 1, P denotes H, but one of the two Ps can also have the other meanings

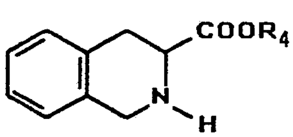

(V')

of $R_3$ in claim 1, including tertbutyl, with a compound of the formula V'

in which $R_4$ is as defined above, in the presence of a condensing agent, and, if appropriate, converting one or both ester groups into the carboxylic acid, or

c) reacting a compound of the formula IV' with a compound of the formula VII

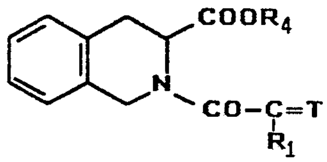 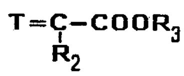

(VI') (VII)

in which $R_1$, $R_2$ and $R_3$ are as defined in claim 1 and $R_4$, is as defined above, one T represents a hydrogen atom and an group and the other T represents an oxygen atom, and reducing the Schiff's base obtained,

if required oxidizing sulfur functions in the compound obtained according to a) to c) to the sulfoxide or to the sulfone, and, if appropriate, converting the resulting compound of the formula I' into its salt.

12. An agent containing a compound as claimed in any one of claims 1 to 10 or its physiologically tolerated salt.

13. A compound as claimed in any one of claims 1 to 10 for use as a medicament.

14. A compound as claimed in any one of claims 1 to 10 for use as a medicament in the treatment of high blood pressure.

**Claim** for the Contracting State: AT

1. A process for the preparation of a compoud of the formula I'

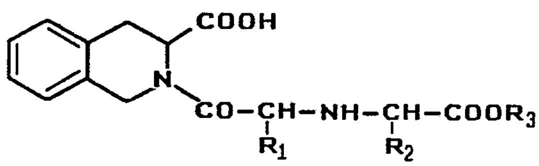

(I')

in which $R_1$ and $R_2$ are identical or different and denote

a) alkyl or alkenyl having up to 6 C atoms, each of which may be substituted by one or more indentical or different substituents from the series comprising
   1. ($C_5$-$C_7$)-cycloalkyl,
   2. ($C_5$-$C_7$)-cycloalkenyl,
   3. fluorine,
   4. mercapto,
   5. hydroxyl,
   6. ($C_1$-$C_2$) -alkoxy which may be substituted in the alkyl moiety by
      6.1 methoxy,
      6.2 ethoxy,
      6.3 carboxyl,
      6.4 carbamoyl,
      6.5 amino or
      6.6 ($C_1$-$C_6$)-alkylamino,
   7. aryloxy which may be substituted by the radicals stated under a) 6.1 - 6.6 or by
      7.1 halogen and/or
      7.2 nitro,
   8. aralkyloxy having 1 or 2 C atoms in the alkyl moiety, which may be substituted in the alkyl moiety as described under a) 6.1 - 6.6 and which may be substituted in the aryl moiety as described above for the alkyl moiety or as described under a) 7.1 or 7.2,
   9. amino,
   10. monoalkylamino having up to 7 C atoms, which may be substituted in the alkyl radical by
      10.1 hydroxyl,
      10.2 carboxyl,
      10.3 carbamoyl,
      10.4 ethoxycarbonyl,
      10.5 amino,
      10.6 ($C_1$-$C_6$)-alkylamino,

10.7 di-($C_1$-$C_6$)-alkylamino,
10.8 piperidino or
10.9 morpholino,

11. dialkylamino having up to 7 C atoms, which may be substituted in the alkyl radical as described under a) 10.1 - 10.9,
12. cycloalkylamino having up to 7 C atoms, which may be substituted in the alkyl radical as described under a) 10.1-10.9,
13. dicycloalkylamino having up to 7 C atoms, which may be substituted in the alkyl radical as described under a) 10.1-10.9,
14. ($C_1$-$C_6$)-alkoxycarbonylamino,
15. aryloxycarbonylamino which may be monosubstituted or disubstituted in the aryl moiety by
15.1 ($C_1$-$C_2$)-alkyl,
15.2 ($C_1$-$C_2$)-alkoxy,
15.3 methylenedioxy,
15.4 amino,
15.5 hydroxyl,
15.6 acetoxy,
15.7 carboxyl,
15.8 carbamoyl,
15.9 ethoxycarbonyl,
15.10 halogen or
15.11 nitro,
16. aralkyloxycarbonylamin which may be substituted in the aryl moiety as described under a) 15.1 - 15.11,
17. ($C_1$-$C_6$)-alkylureido,
18. arylureido which may be monosubstituted or disubstituted in the aryl moiety by identical or different radicals as described under a) 15.1 - 15.11,
19. aralkylureido which may be monosubstituted or disubstituted in the aryl moiety as described under a) 15.1 - 15.11,
20. formyl,
21. alkanoylamino having up to 10 C atoms,
22. aroylamino having up to 10 C atoms, which may be monosubstituted or disubstituted in the aryl moiety as described under a) 15.1 - 15.11,
23. aralkanoylamino having up to 10 C atoms, which may be monosubstituted or disubstituted in the aryl moiety as described under a) 15.1 - 15.11,
24. arylamino which may be monosubstituted or disubstituted in the aryl moiety by identical or different radicals as described under a) 15.1 - 15.11,
25. aralkylamino which may be substituted in the aryl moiety as described under a) 15.1 - 15.11,
26. alkylmercapto, alkylsulfinyl or alkylsulfonyl having up to 7 C atoms, each of which may be substituted in the alkyl moiety by
26.1 methoxy,
26.2 ethoxy,
26.3 hydroxyl,
26.4 carboxyl,
26.5 carbamoyl,
26.6 ethoxycarbonyl,
26.7 amino or
26.8 ($C_1$-$C_6$)-alkylamino,
27. phenylmercapto, phenylsulfinyl or phenylsulfonyl, which may be substituted in the aryl moiety as described under a) 26.1 - 26.8 or by
27.1 halogen,
27.2 nitro or
27.3 sulfamoyl,
28. benzylmercapto, benzylsulfinyl or benzylsulfonyl, which may be substituted in the alkyl moiety as described under a) 26.1 - 26.8 and which may be substituted in the aryl moiety as for the above alkyl moiety or as described under a) 27.1 - 27.3,
29. carboxyl,
30. ethoxycarbonyl,
31. carbamoyl,
32. alkylaminocarbonyl having up to 6 C atoms,
33. cycloalkylcarbamoyl having up to 6 C atoms,
34. cycloalkyleneaminocarbonyl having up to 6 C atoms,
35. dialkylcarbamoyl having up to 6 C atoms,
36. arylcarbamoyl which may be substituted in the aryl moiety by
36.1 halogen,
36.2 ($C_1C_6$)-alkyl or

36.3 ($C_1C_6$)-alkoxy,

37. aralkylcarbamoyl,
38. guanidino,
39. phenyl, naphthyl, dihydronaphthyl or tetrahydronaphthyl, each of which may be monosubstituted or disubstituted by
    39.1 halogen,
    39.2 hydroxyl,
    39.3 acetoxy,
    39.4 carboxyl,
    39.5 carbamoyl,
    39.6 sulfamoyl,
    39,7 nitro,
    39.8 methyl,
    39.9 ethyl,
    39.10 methoxy and/or
    39.11 ethoxy,
40. a 5-membered to 7 membered monocyclic or 9 membered or 10-membered bicyclic heterocyclic radical which may contain 1 or 2 S or O atoms and/or up to 4 N atoms per ring and which may be substituted by
    40.1 halogen,
    40.2 oxygen,
    40.3 hydroxyl,
    40.4 carboxyl,
    40.5 carbamoyl,
    40.6 sulfamoyl,
    40.7 nitro,
    40.8 alkyl having up to 9 C atoms,
    40.9 aralkyl having up to 9 C atoms,
    40.10 methoxy and/or
    40.11 ethoxy;

b) denote cycloalkyl or cycloalkenyl, each having 5 - 7 C atoms;
c) denote aryl or partially hydrogenated aryl; or
d) denote a monocyclic or bicyclic heterocyclic radical having 5 - 7 or 8 - 10 members, including 1 or 2 -S- or -O- and/or up to 4 N atoms, which radical may be substituted as described under a) 40.1 - 40.11,

and
$R_3$ denotes hydrogen, alkyl having 1 to 6 C atoms, alkenyl having 2 to 6 C atoms, aralkyl having 7 to 14 C atoms, including p-nitrobenzyl, and, unless specifically defined otherwise, aryl has 6 to 10 C atoms and may be substituted by halogen, ($C_1$-$C_6$) -alkyl or ($C_1$-$C_6$)-alkoxy, and denotes aralkyl, benzyl or phenethyl and may be substituted by halogen, ($C_1$-$C_6$)-alkyl or ($C_1$-$C_6$)-alkoxy in the phenyl nucleus, or their physiologically tolerated salts.
which comprises

a) reacting a compound of the formula II' with a compound of the formula III

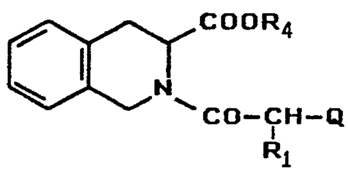

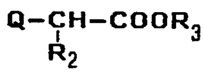

(II') (III)

in which $R_1$, $R_2$ and $R_3$ are as defined above, one Q denotes a nucleofugic group and the other Q denotes $-NH_2$ and $R_4$ represents H, methyl, ethyl, benzyl or tert-butyl, and, if appropriate, converting an ester obtained into the carboxylic acid ($R_2$ and/or $R_4$ = hydrogen), or

b) reacting a compound of the formula IV

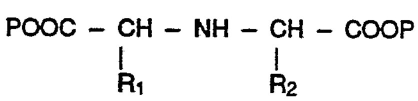

(IV)

wherein $R_1$ and $R_2$ are as defined above, p denotes H, but one of the two Ps can also have the other meanings of $R_3$ defined above, including tert-butyl, with a compound of the formula V'

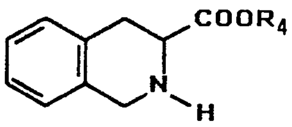

(V')

in which $R_4$ is as defined above, in the presence of a condensing agent, and, if appropriate, converting one of the two ester groups into the carboxylic acid, or

c) reacting a compound of the formula VI' with a compound of the formula VII

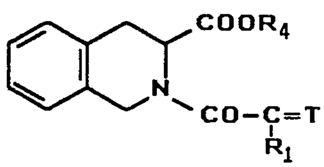

(VI')

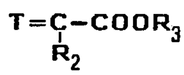

(VII)

in which $R_1$, $R_2$, $R_3$ and $R_4$ are as defined above, one T represents a hydrogen atom and an $NH_2$ group and the other T represents an oxygen atom, and reducing the resulting Schiff's base,

if required oxidizing sulfur functions in the compound obtained according to a) to c) into the sulfoxide or into the sulfone, and, if appropriate, converting the resulting compound of the formula I' into the salt.

2. A process as claimed in claim 1, which comprises preparing a compound in which $R_1$ is methyl and $R_2$ is phenethyl or phenethyl which is substituted by halogen, methyl or methoxy.

3. A process as claimed in claim 1 or 2, for the preparation of a compound of the formula I', wherein $R_3$ is hydrogen, ethyl, butyl, tertbutyl, benzyl or p-nitrobenzyl.

4. A process as claimed in claim 1 or 3, for the preparation of a compound of the formula I', wherein $R_1$ denotes the side chain of a naturally occurring L-amino acid or its ether, ester or amide.

5. A process as claimed in claim 4 for the preparation of a compound of the formula I', wherein $R_1$ denotes methyl, isobutyl, methylthioethyl, carboxymethyl, carboxyethyl, amino-n-butyl, guanidino-n-propyl, imidazol4-ethyl, benzyl, 4-hydroxybenzyl or 4-indolmethyl.

6. A compound as claimed in claim 1 or 3 for the preparation of a compound of the formula I', wherein $R_1$ denotes methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, dior tetrahydronaphthyl, benzyl, phenethyl, p-fluorophenethyl, omethylphenethyl, p-methoxyphenethyl, 2,4-dichlorophenethyl or cyclohexylethyl.

7. A compound as claimed in claim 1 or 3 for the preparation of a compound of the formula I', wherein $R_2$ denotes methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, dior tetrahydronaphthyl, benzyl, phenethyl, p-fluorophenethyl, o-methylphenethyl, p-methoxyphenethyl, 2, 4 -dichlorophenethyl or cyclohexylethyl.

8. A process as claimed in any one of claims 1 to 7 for the preparation of 1-[N-(1-carboxy-3-phenylpropyl)(S)-alanyl]-1,2,3,4-tetrahydroisoquinoline-3-carboxylic acid or its physiologically tolerated salt.

9. A process as claimed in any one of claims 1 to 7 for the preparation of 1-[N-(1-ethoxycarbonyl-3-phenylpropyl)-(S)-alanyl]-1,2,3,4-tetrahydroisoquinoline-3-carboxylic acid or its physiologically tolerated salt.

10. A compound as claimed in any one of claims 1 to 9 for the preparation of a compound of formula I', in which the three chiral centers are present at a-C atoms in the L configuration.

11. A process for the preparation of an agent containing a compound of the formula I' as claimed in any one of claims 1 to 10 or its physiologically tolerated salt, which comprises bringing the said compound or salt into a suitable administration form.

12. A process as claimed in any one of claims 1 to 10 for the preparation of a compound of the formula I' for use as a medicament.

13. A process as claimed in any one of claims 1 to 10 for the preparation of a compound of the formula I' for use as a medicament in the treatment of high blood pressure.

**Revendications** pour les Etats contractants: BE, CH, DE, FR, GB, IT, LI, LU, NL, SE

1. Composé de formule I'

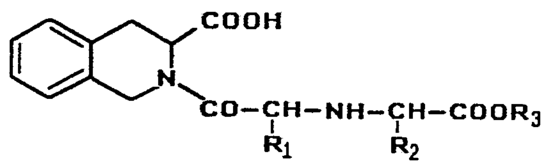

(I')

dans laquelle $R_1$ et $R_2$ sont identiques ou différents, et représentent

a) des groupes alkyle ou alcényle ayant jusqu'à 6 atomes de carbone, qui sont éventuellement substitués chacun par un ou plusieurs substituants identiques ou différents, choisis parmi
   1. un groupe cycloalkyle en $C_5$-$C_7$,
   2. un groupe cycloalcényle en $C_5$-$C_7$,
   3. un atome de fluor,
   4. le groupe mercapto,
   5. le groupe hydroxy,
   6. un groupe alcoxy en $C_1$-$C_2$ qui est éventuellement substitué sur le fragment alkyle par un radical
      6.1. méthoxy,
      6.2. éthoxy,
      6.3. carboxy,
      6.4. carbamoyle,
      6.5. amino ou
      6.6. alkyl($C_1$-$C_6$)-amino,
   7. un groupe aryloxy qui est éventuellement substitué par les radicaux 6.1. - 6.6. mentionnés en a), ou par
      7.1. un atome d'halogène et/ou
      7.2. le groupe nitro.
   8. un groupe aralkyloxy ayant 1 ou 2 atomes de carbone dans le fragment alkyle, qui est éventuellement substitué sur le fragment alkyle comme décrit en a) 6.1 - 6.6., et qui est éventuellement substitué sur le fragment aryle comme indiqué précédemment pour le fragment alkyle, ou comme décrit en a) 7.1. ou 7.2.,
   9. le groupe amino,
   10. un groupe monoalkylamino ayant jusqu'à 7 atomes de carbone, qui est éventuellement substitué sur le reste alkyle par un radical
      10.1. hydroxy,
      10.2. carboxy,
      10.3. carbamoyle,
      10.4. éthoxycarbonyle,
      10.5. amino,
      10.6. alkyl($C_1$-$C_6$)-amino,
      10.7. dialkyl($C_1$-$C_6$)-amino,
      10.8. pipéridino ou
      10.9. morpholino,
   11. un groupe dialkylamino ayant jusqu'à 7 atomes de carbone, qui est éventuellement substitué sur le reste alkyle comme décrit en a) 10.1. - 10.9.,
   12. un groupe cycloalkylamino ayant jusqu'à 7 atomes de carbone, qui est éventuellement substitué sur le reste alkyle comme décrit en a) 10.1. - 10.9.,
   13. un groupe dicycloalkylamino ayant jusqu'à 7 atomes de carbone, qui est éventuellement substitué sur le reste alkyle comme décrit en a) 10.1. - 10.9.,
   14. un groupe alcoxy ($C_1$-$C_6$)-carbonylamino,
   15. un groupe aryloxycarbonylamino qui est éventuellement mono- ou disubstitué sur le fragment aryle par un radical
      15.1. alkyle en $C_1$-$C_2$,
      15.2. alcoxy en $C_1$-$C_2$,
      15.3. méthylènedioxy,
      15.4. amino,

15.5. hydroxy,
15.6. acétoxy,
15.7. carboxy,
15.8. carbamoyle,
15.9. éthoxycarbonyle,
15.10. un atome d'halogène ou
15.11. le groupe nitro,

16. un groupe aralkyloxycarbonyle qui est éventuellement substitué sur le fragment aryle comme décrit en a) 15.1. - 15.11.,
17. un groupe alkyl($C_1$-$C_6$)-uréido,
18. un groupe aryluréido qui est éventuellement mono- ou disubstitué sur le fragment aryle par un ou des radicaux identiques ou différents, tels que décrits en a) 15.1. - 15.11.,
19. un groupe aralkyluréido qui est éventuellement monoou disubstitué sur le fragment aryle comme décrit en a) 15.1 - 15.11,
20. le groupe formyle,
21. un groupe alcanoylamino ayant jusqu'à 10 atomes de carbone,
22. un groupe aroylamino ayant jusqu'à 10 atomes de carbone, qui est éventuellement mono- ou disubstitué sur le fragment aryle comme décrit en a) 15.1 - 15.11,
23. un groupe aralkanoylamino ayant jusqu'à 10 atomes de carbone, qui est éventuellement mono- ou disubstitué sur le fragment aryle comme décrit en a) 15.1. - 15.11.,
24. un groupe arylamino qui est éventuellement mono- ou disubstitué par un-ou des radicaux identiques ou différents tels que décrits en a) 15.1. - 15.11.,
25. un groupe aralkylamino qui est éventuellement substitué sur le fragment aryle comme décrit en a) 15.1 - 15.11.,
26. un groupe alkylmercapto, alkylsulfinyle ou alkylsulfonyle ayant jusqu'à 7 atomes de carbone, qui dans chaque cas est éventuellement substitué sur le fragment alkyle par un radical
    26.1. méthoxy,
    26.2. éthoxy,
    26.3. hydroxy,
    26.4. carboxy,
    26.5. carbamoyle,
    26.6. éthoxycarbonyle,
    26.7. amino ou
    26.8. alkyl($C_1$-$C_6$)-amino,
27. un groupe phénylmercapto, phénylsulfinyle ou phénylsulfonyle qui est éventuellement substitué sur le fragment aryle comme décrit en a) 26.1 - 26.8., ou par
    27.1. un atome d'halogène ou
    27.2. le groupe nitro ou
    27.3. sulfamoyle,
28. un groupe benzylmercapto, benzylsulfinyle ou benzylsulfonyle, qui est éventuellement substitué sur le fragment alkyle comme décrit en a) 26.1 - 26.8 et qui est éventuellement substitué sur le fragment aryle comme le fragment alkyle précédent ou comme décrit en a) 27.1. - 27.3.,
29. le groupe carboxy,
30. le groupe éthoxycarbonyle,
31. le groupe carbamoyle,
32. un groupe alkylaminocarbonyle ayant jusqu'à 6 atomes de carbone,
33. un groupe cycloalkylcarbamoyle ayant jusqu'à 6 atomes de carbone,
34. un groupe cycloalkylaminocarbonyle ayant jusqu'à 6 atomes de carbone,
35. un groupe dialkylcarbamoyle ayant jusqu'à 6 atomes de carbone,
36. un groupe arylcarbamoyle qui est éventuellement substitué sur le fragment aryle par
    36.1. un atome d'halogène,
    36.2. un radical alkyle en $C_1$-$C_6$,
    36.3. un radical alcoxy en $C_1$-$C_6$,
37. un groupe aralkylcarbamoyle,
38. le groupe guanidino,
39. des radicaux phényle, naphtyle, dihydronaphtyle ou tétrahydronaphtyle, qui sont éventuellement mono- ou disubstitués chacun par
    39.1. un ou des atomes d'halogène, ou un ou des groupes
    39.2. hydroxy,
    39.3. acétoxy,
    39.4. carboxy,
    39.5. carbamoyle,
    39.6. sulfamoyle,
    39.7. nitro,
    39.8. méthyle,

39.9. éthyle,
39.10. méthoxy et/ou
39.11. éthoxy,

40. un radical hétérocyclique monocyclique à 5 - 7 chaînons ou bicyclique à 9 - 10 chaînons, contenant éventuellement un ou deux atomes de S ou O et/ou jusqu'à 4 atomes d'azote par cycle, qui est éventuellement substitué par
40.1. un atome d'halogène,
40.2. un atome d'oxygène,
40.3. un groupe hydroxy,
40.4. carboxy,
40.5. carbamoyle,
40.6. sulfamoyle,
40.7. nitro,
40.8. alkyle ayant jusqu'à 9 atomes de carbone,
40.9. aralkyle ayant jusqu'à 9 atomes de carbone,
40.10. méthoxy et/ou
40.11. éthoxy;

b) un groupe cycloalkyle ou cycloalcényle ayant de 5 à 7 atomes de carbone;
c) un groupe aryle ou aryle partiellement hydrogéné; ou
d) un radical hétérocyclique mono- ou bicyclique à 5 - 7 ou respectivement 8 - 10 chaînons, dont 1 ou 2 représente(nt) un ou des atome(s) de S ou O et/ou jusqu'à 4 des atomes d'azote, qui est éventuellement substitué comme décrit en a) 40.1. - 40.11.;
et
$R_3$ représente un atome d'hydrogène ou un groupe alkyle ayant de 1 à 6 atomes de carbone, alcényle ayant de 2 à 6 atomes de carbone, aralkyle ayant de 7 à 14 atomes de carbone, y compris le groupe p-nitrobenzyle;

à moins d'indication contraire, le radical aryle comportant de 6 à 10 atomes de carbone, et étant éventuellement substitué par un atome d'halogène ou par un groupe alkyle en $C_1$-$C_6$ ou alcoxy en $C_1$-$C_6$, et "aralkyle" signifiant le radical benzyle ou phénéthyle et étant éventuellement substitué sur le noyau phényle par un atome d'halogène ou par un groupe alkyle en $C_1$-$C_6$ ou alcoxy en $C_1$-$C_6$;
ou sels physiologiquement acceptables de celui-ci.

2. Composé selon la revendication 1, dans lequel $R_1$ est le radical méthyle et $R_2$ un radical phénéthyle ou phénéthyle substitué par un atome d'halogène ou par un groupe méthyle ou méthoxy.

3. Composé selon la revendication 1 ou 2, dans lequel $R_3$ représente un atome d'hydrogène ou un groupe éthyle, butyle, tert-butyle, benzyle, p-nitrobenzyle.

4. Composé selon la revendication 1 ou 3, dans lequel $R_1$ représente la chaîne latérale d'un L-aminoacide existant dans la nature, ou de son éther, ester ou amide.

5. Composé selon la revendication 4, dans lequel $R_1$ représente le groupe méthyle, isobutyle, méthylthioéthyle, carboxyméthyle, carboxyéthyle, amino-n-butyle, guanidino-n-propyle, imidazole-4-éthyle, benzyle, 4-hydroxybenzyle ou 3-indole-méthyle.

6. Composé selon la revendication 1 ou 3, dans lequel $R_1$ représente le groupe méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

7. Composé selon la revendication 1 ou 3, dans lequel $R_2$ représente le groupe méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

8. Acide 1-[N-(1-carboxy-3-phényl-propyl)-(S)-alanyl]-1,2,3,4-tétrahydroisoquinoléine-3-carboxylique ou sel physiologiquement acceptable de celui-ci.

9. Acide 1-[N-(1-éthoxycarbonyl-3-phényl-propyl)-(S)alanyl]-1,2,3,4-tétrahydroisoquinoléine-3-carboxylique ou sel physiologiquement acceptable de celui-ci.

10. Composé selon l'une des revendications 1 à 9, dans lequel les trois centres chiraux au niveau des atomes de carbone α se trouvent dans la configuration L.

11. Procédé pour la préparation d'un composé de formule I', selon l'une des revendications 1 à 10, caractérisé en ce que

a) on fait réagir un composé de formule II' avec un composé de formule III

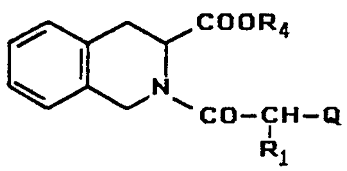
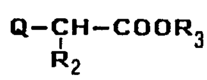

(II') (III)

dans lesquelles formules $R_1$, $R_2$ et $R_3$ sont tels que définis dans la revendication 1, Q est un groupe nucléofuge et l'autre radical Q représente $-NH_2$, et $R_4$ représente H ou le groupe méthyle, éthyle, benzyle ou tert-butyle, et éventuellement on convertit un ester obtenu en l'acide carboxylique ($R_2$ et/ou $R_4$ = hydrogène), ou

b) on fait réagir en présence d'un agent de condensation un composé de formule IV

POOC – CH – NH – CH – COOP (IV)
with $R_1$ on the first CH and $R_2$ on the second CH

dans laquelle $R_1$ et $R_2$ sont tels que définis dans la revendication 1, P représente H, l'un des deux P pouvant toutefois avoir également les autres significations de $R_3$, dans la revendication 1, y compris celle du groupe tert-butyle, avec un composé de formule V'

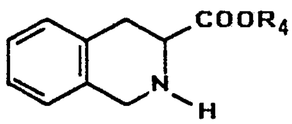

(V')

dans laquelle $R_4$ est tel que défini plus haut, et éventuellement on convertit un ou les deux groupe(s) ester en l'acide carboxylique, ou

c) on fait réagir un composé de formule VI' avec un composé de formule VII

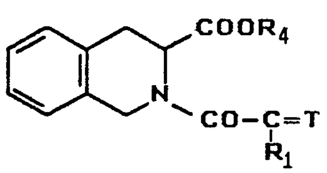
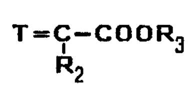

(VI') (VII)

dans lesquelles $R_1$, $R_2$, $R_3$ et $R_4$ sont tels que définis dans la revendication 1, un radical T représente un atome d'hydrogène et le groupe $NH_2$, et l'autre T représente un atome d'oxygène, puis on réduit la -base de Schiff obtenue,

éventuellement on oxyde, dans le composé obtenu selon a) à c), les fonctions soufre en sulfoxyde ou en sulfone, et on convertit éventuellement en son sel le composé de formule I' obtenu.

12. Produit contenant un composé selon l'une des revendications 1 à 10 ou un sel physiologiquement acceptable de celui-ci.

13. Composé selon l'une des revendications 1 à 10, pour l'utilisation en tant que médicament.

14. Composé selon l'une des revendications 1 à 10, pour l'utilisation en tant que médicament dans le traitement de l'hypertension artérielle.

**Revendications** pour l'Etat contractant: AT

1. Composé de formule I'

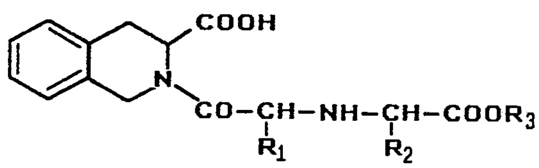

(I)

dans laquelle $R_1$ et $R_2$ sont identiques ou différents, et représentent

a) des groupes alkyle ou alcényle ayant jusqu'à 6 atomes de carbone, qui sont éventuellement substitués chacun par un ou plusieurs substituants identiques ou différents, choisis parmi

1. un groupe cycloalkyle en $C_5$-$C_7$,
2. un groupe cycloalcényle en $C_5$-$C_7$,
3. un atome de fluor,
4. le groupe mercapto,
5. le groupe hydroxy,
6. un groupe alcoxy en $C_1$-$C_2$ qui est éventuellement substitué sur le fragment alkyle par un radical
   6.1. méthoxy,
   6.2. éthoxy,
   6.3. carboxy,
   6.4. carbamoyle,
   6.5. amino ou
   6.6. alkyl($C_1$-$C_6$)-amino,
7. un groupe aryloxy qui est éventuellement substitué par les radicaux 6.1. - 6.6. mentionnés en a), ou par
   7.1. un atome d'halogène et/ou
   7.2. le groupe nitro.
8. un groupe aralkyloxy ayant 1 ou 2 atomes de carbone dans le fragment alkyle, qui est éventuellement substitué sur le fragment alkyle comme décrit en a) 6.1. - 6.6., et qui est éventuellement substitué sur le fragment aryle comme indiqué précédemment pour le fragment alkyle, ou comme décrit en a) 7.1. ou 7.2.,
9. le groupe amino,
10. un groupe monoalkylamino ayant jusqu'à 7 atomes de carbone, qui est éventuellement substitué sur le reste alkyle par un radical
    10.1. hydroxy,
    10.2. carboxy,
    10.3. carbamoyle,
    10.4. éthoxycarbonyle,
    10.5. amino,
    10.6. alkyl($C_1$-$C_6$)-amino,
    10.7. dialkyl($C_1$-$C_6$)-amino,
    10.8. pipéridino ou
    10.9. morpholino,
11. un groupe dialkylamino ayant jusqu'à 7 atomes de carbone, qui est éventuellement substitué sur le reste alkyle comme décrit en a) 10.1. - 10.9.,
12. un groupe cycloalkylamino ayant jusqu'à 7 atomes de carbone, qui est éventuellement substitué sur le reste alkyle comme décrit en a) 10.1. - 10.9.,
13. un groupe dicycloalkylamino ayant jusqu'à 7 atomes de carbone, qui est éventuellement substitué sur le reste alkyle comme décrit en a) 10.1. - 10.9.,
14. un groupe alcoxy ($C_1$-$C_6$)-carbonylamino,
15. un groupe aryloxycarbonylamino qui est éventuellement mono- ou disubstitué sur le fragment aryle par un radical
    15.1. alkyle en $C_1$-$C_2$,
    15.2. alcoxy en $C_1$-$C_2$,
    15.3. méthylènedioxy,
    15.4. amino,
    15.5. hydroxy,
    15.6. acétoxy,
    15.7. carboxy,
    15.8. carbamoyle,
    15.9. éthoxycarbonyle,
    15.10. un atome d'halogène ou
    15.11. le groupe nitro,

16. un groupe aralkyloxycarbonyle qui est éventuellement substitué sur le fragment aryle comme décrit en a) 15.1. - 15.11.,
17. un groupe alkyl($C_1$-$C_6$)-uréido,
18. un groupe aryluréido qui est éventuellement mono- ou disubstitué sur le fragment aryle par un ou des radicaux identiques ou différents, tels que décrits en a) 15.1. - 15.11.,
19. un groupe aralkyluréido qui est éventuellement monoou disubstitué sur le fragment aryle comme décrit en a) 15.1 - 15.11,
20. le groupe formyle,
21. un groupe alcanoylamino ayant jusqu'à 10 atomes de carbone,
22. un groupe aroylamino ayant jusqu'à 10 atomes de carbone, qui est éventuellement mono- ou disubstitué sur le fragment aryle comme décrit en a) 15.1 - 15.11,
23. un groupe aralkanoylamino ayant jusqu'à 10 atomes de carbone, qui est éventuellement mono- ou disubstitué sur le fragment aryle comme décrit en a) 15.1. - 15.11.,
24. un groupe arylamino qui est éventuellement mono- ou disubstitué par un-ou des radicaux identiques ou différents tels que décrits en a) 15.1. - 15.11.,
25. un groupe aralkylamino qui est éventuellement substitué sur le fragment aryle comme décrit en a) 15.1 - 15.11.,
26. un groupe alkylmercapto, alkylsulfinyle ou alkylsulfonyle ayant jusqu'à 7 atomes de carbone, qui dans chaque cas est éventuellement substitué sur le fragment alkyle par un radical
    26.1. méthoxy,
    26.2. éthoxy,
    26.3. hydroxy,
    26.4. carboxy,
    26.5. carbamoyle,
    26.6. éthoxycarbonyle,
    26.7. amino ou
    26.8. alkyl($C_1$-$C_6$)-amino,
27. un groupe phénylmercapto, phénylsulfinyle ou phénylsulfonyle qui est éventuellement substitué sur le fragment aryle comme décrit en a) 26.1 - 26.8., ou par
    27.1. un atome d'halogène ou
    27.2. le groupe nitro ou
    27.3. sulfamoyle,
28. un groupe benzylmercapto, benzylsulfinyle ou benzylsulfonyle, qui est éventuellement substitué sur le fragment alkyle comme décrit en a) 26.1 - 26.8 et qui est éventuellement substitué sur le fragment aryle comme le fragment alkyle précédent ou comme décrit en a) 27.1. - 27.3.,
29. le groupe carboxy,
30. le groupe éthoxycarbonyle,
31. le groupe carbamoyle,
32. un groupe alkylaminocarbonyle ayant jusqu'à 6 atomes de carbone,
33. un groupe cycloalkylcarbamoyle ayant jusqu'à 6 atomes de carbone,
34. un groupe cycloalkylaminocarbonyle ayant jusqu'à 6 atomes de carbone,
35. un groupe dialkylcarbamoyle ayant jusqu'à 6 atomes de carbone,
36. un groupe arylcarbamoyle qui est éventuellement substitué sur le fragment aryle par
    36.1. un atome d'halogène,
    36.2. un radical alkyle en $C_1$-$C_6$,
    36.3. un radical alcoxy en $C_1$-$C_6$,
37. un groupe aralkylcarbamoyle,
38. le groupe guanidino,
39. des radicaux phényle, naphtyle, dihydronaphtyle ou tétrahydronaphtyle, qui sont éventuellement mono- ou disubstitués chacun par
    39.1. un ou des atomes d'halogène, ou un ou des groupes
    39.2. hydroxy,
    39.3. acétoxy,
    39.4. carboxy,
    39.5. carbamoyle,
    39.6. sulfamoyle,
    39.7. nitro,
    39.8. méthyle,
    39.9. éthyle,
    39.10. méthoxy et/ou
    39.11. éthoxy,
40. un radical hétérocyclique monocyclique à 5 - 7 chaînons ou bicyclique à 9 - 10 chaînons, contenant éventuellement un ou deux atomes de S ou O et/ou jusqu'à 4 atomes d'azote par cycle, qui est éventuellement substitué par
    40.1. un atome d'halogène,

40.2. un atome d'oxygène,
40.3. un groupe hydroxy,
40.4. carboxy,
40.5. carbamoyle,
40.6. sulfamoyle,
40.7. nitro,
40.8. alkyle ayant jusqu'à 9 atomes de carbone,
40.9. aralkyle ayant jusqu'à 9 atomes de carbone,
40.10. méthoxy et/ou
40.11. éthoxy;

b) un groupe cycloalkyle ou cycloalcényle ayant de 5 à 7 atomes de carbone;
c) un groupe aryle ou aryle partiellement hydrogéné; ou
d) un radical hétérocyclique mono- ou bicyclique à 5 - 7 ou respectivement 8 - 10 chaînons, dont 1 ou 2 représente(nt) un ou des atome(s) de S ou O et/ou jusqu'à 4 des atomes d'azote, qui est éventuellement substitué comme décrit en a) 40.1. - 40.11.;
et
$R_3$ représente un atome d'hydrogène ou un groupe alkyle ayant de 1 à 6 atomes de carbone, alcényle ayant de 2 à 6 atomes de carbone, aralkyle ayant de 7 à 14 atomes de carbone, y compris le groupe p-nitrobenzyle;

à moins d'indication contraire, le radical aryle comportant de 6 à 10 atomes de carbone, et étant éventuellement substitué par un atome d'halogène ou par un groupe alkyle en $C_1$-$C_6$ ou alcoxy en $C_1$-$C_6$, et
"aralkyle" signifiant le radical benzyle ou phénéthyle et étant éventuellement substitué sur le noyau phényle par un atome d'halogène ou par un groupe alkyle en $C_1$-$C_6$ ou alcoxy en $C_1$-$C_6$;
ou sels physiologiquement acceptables,
caractérisé en ce que

a) on fait réagir un composé de formule II' avec un composé de formule III

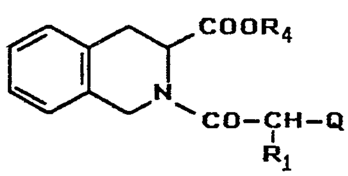

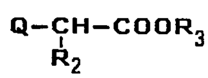

(II') (III)

dans lesquelles formules $R_1$, $R_2$ et $R_3$ sont tels que définis plus haut, Q est un groupe nucléofuge et l'autre radical Q représente -$NH_2$, et $R_4$ représente H ou le groupe méthyle, éthyle, benzyle ou tert-butyle, et éventuellement on convertit un ester obtenu en l'acide carboxylique ($R_2$ et/ou $R_4$ = hydrogène), ou

b) on fait réagir en présence d'un agent de condensation un composé de formule IV

POOC – CH – NH – CH – COOP (IV)
$R_1$ (sur le premier CH), $R_2$ (sur le second CH)

dans laquelle $R_1$ et $R_2$ sont tels que définis plus haut, P représente H, l'un des deux P pouvant toutefois avoir également les autres significations de $R_3$, y compris celle du groupe tert-butyle, avec un composé de formule V'

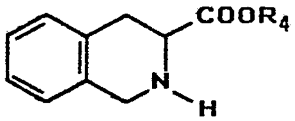

(V')

dans laquelle $R_4$ est tel que défini plus haut, et éventuellement on convertit un ou les deux groupe(s) ester en l'acide carboxylique, ou

c) on fait réagir un composé de formule VI' avec un composé de formule VII

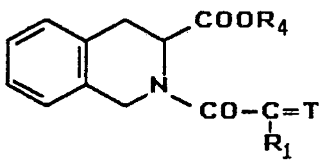
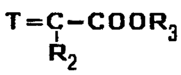

(V') (VII)

dans lesquelles $R_1$, $R_2$, $R_3$ et $R_4$ sont tels que définis plus haut, un radical T représente un atome d'hydrogène et le groupe $NH_2$, et l'autre T représente un atome d'oxygène, puis on réduit la base de Schiff obtenue,

éventuellement on oxyde, dans le composé obtenu selon a) à c), les fonctions soufre en sulfoxyde ou en sulfone, et on convertit éventuellement en le sel le composé de formule I' obtenu.

2. Procédé selon la revendication 1, caractérisé en ce que l'on prépare un composé dans lequel $R_1$ est le radical méthyle et $R_2$ un radical phénéthyle ou phénéthyle substitué par un atome d'halogène ou par un groupe méthyle ou méthoxy.

3. Procédé selon la revendication 1 ou 2, pour la préparation d'un composé de formule I' dans lequel $R_3$ représente un atome d'hydrogène ou un groupe éthyle, butyle, tertbutyle, benzyle, p-nitrobenzyle.

4. Procédé selon la revendication 1 ou 3, pour la préparation d'un composé de formule I' dans lequel $R_1$ représente la chaîne latérale d'un L-aminoacide existant dans la nature, ou de son éther, ester ou amide.

5. Procédé selon la revendication 4, pour la préparation d'un composé de formule I' dans lequel $R_1$ représente le groupe méthyle, isobutyle, méthylthioéthyle, carboxyméthyle, carboxyéthyle, amino-n-butyle, guanidino-n-propyle, imidazole-4-éthyle, benzyle, 4-hydroxybenzyle ou 3-indoleméthyle.

6. Procédé selon la revendication 1 ou 3, pour la préparation d'un composé de formule I' dans lequel $R_1$ représente le groupe méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

7. Procédé selon la revendication 1 ou 3, pour la préparation d'un composé de formule I' dans lequel $R_2$ représente le groupe méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

8. Procédé selon l'une des revendications t à 7, pour la préparation de l'acide 1-[N-(1-carboxy-3-phényl-propyl)(S)-alanyl]-1,2,3,4-tétrahydroisoquinoléine-3-carboxylique ou d'un sel physiologiquement acceptable de celui-ci.

9. Procédé selon l'une des revendications 1 à 7, pour la préparation de l'acide 1-[N-(1-éthoxycarbonyl-3-phénylpropyl)-(S)-alanyl]-1,2,3,4-tétrahydroisoquinoléine-3carboxylique ou d'un sel physiologiquement acceptable de celui-ci.

10. Procédé selon l'une des revendications 1 à 9, pour la préparation d'un composé de formule I' dans lequel les trois centres chiraux au niveau des atomes de carbone a se trouvent dans la configuration L.

11. Procédé pour la préparation d'un produit contenant un composé de formule I' selon l'une des revendications 1 à 10, ou d'un sel physiologiquement acceptable de celui-ci, caractérisé en ce que l'on met celui-ci sous une forme pharmaceutique appropriée.

12. Procédé selon l'une des revendications 1 à 10, pour la préparation d'un composé de formule I', pour l'utilisation en tant que médicament.

13. Procédé selon l'une des revendications 1 à 10, pour la préparation d'un composé de formule I', pour l'utilisation en tant que médicament dans le traitement de l'hypertension artérielle.